(19) Europäisches Patentamt
European Patent Office
Office européen des brevets

(11) Numéro de publication: **0 176 387**
**B1**

(12) **FASCICULE DE BREVET EUROPEEN**

(45) Date de publication du fascicule du brevet: **22.11.90**

(51) Int. Cl.$^5$: **C 07 D 207/32,**
C 07 D 207/34, A 01 N 53/00,
A 23 K 1/16

(21) Numéro de dépôt: **85401627.6**

(22) Date de dépôt: **09.08.85**

(54) **Nouveaux dérivés du pyrrole, leur procédé de préparation et leur application comme pesticides.**

(30) Priorité: **14.08.84 FR 8412791**
**23.04.85 FR 8506134**

(43) Date de publication de la demande:
**02.04.86 Bulletin 86/14**

(45) Mention de la délivrance du brevet:
**22.11.90 Bulletin 90/47**

(84) Etats contractants désignés:
**AT BE CH DE FR GB IT LI LU NL SE**

(56) Documents cités:
**EP-A-0 050 534**
**US-A-3 850 977**
**US-A-4 229 352**

**PATENTS ABSTRACTS OF JAPAN, vol. 6, no. 223 (C-133) 1101r, 9 novembre 1982**

**Le dossier contient des informations techniques présentées postérieurement au dépôt de la demande et ne figurant pas dans le présent fascicule.**

(73) Titulaire: **ROUSSEL-UCLAF**
**35, boulevard des Invalides**
**F-75007 Paris (FR)**

(72) Inventeur: **Tessier, Jean**
**30, rue Jean Moulin**
**F-94300 Vincennes (FR)**
Inventeur: **Demoute, Jean-Pierre**
**249bis, rue de Rosny**
**F-93100 Montreuil Sous Bois (FR)**
Inventeur: **Taliani,Laurent**
**107, Allée Danielle Casanova**
**F-93320 Les Pavillons Sous Bois (FR)**

(74) Mandataire: **Fritel, Hubert et al**
**Département des Brevets ROUSSEL UCLAF B.P. no 9**
**F-93230 Romainville (FR)**

(56) Documents cités:
**PATENTS ABSTRACTS OF JAPAN, vol. 7, no. 232 (C-190) 1377r, 14 octobre 1983**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**EP 0 176 387 B1**

**Description**

La présente invention concerne de nouveaux dérivés du pyrrole, leur procédé et leurs intermédiaires de préparation, leur application comme pesticides et les compositions les renfermant.

On connaissait déjà des esters d'acides cyclopropane carboxylique apparentés à l'acide pyréthrique (cf EP 050 534), on connaissait également des esters d'acide 2,2-diméthyl cyclopropane carboxylique et d'alcool renfermant un noyau pyrrolique (cf Patent Abstracts of Japan, vol no 223 (c-133), 9 Novembre 1982). On vient de découvrir de nouveaux dérivés d'esters pyréthinoïdes et d'alcools renfermant un noyau pyrrolique présentant des propriétés pesticides.

L'invention a pour objet les composés de formule (I):

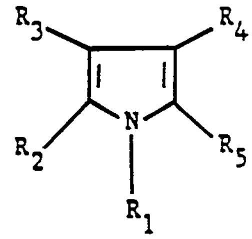

(I)

dans laquelle:

l'un des radicaux $R_2$ ou $R_3$ représente un radical:

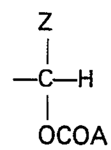

dans lequel

*ou bien A représenté un radical*

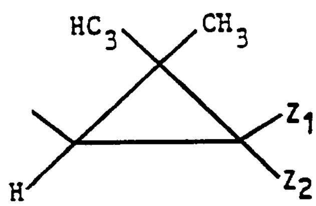

dans lequel

$Z_1$ et $Z_2$ représentent chacun un radical méthyle,

ou $Z_1$ représente un atome d'hydrogène et

*soit* $Z_2$ représente un radical

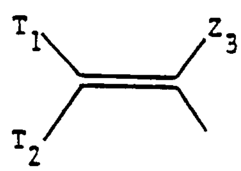

dans lequel $Z_3$ représente un atome d'hydrogène ou d'halogène et $T_1$ et $T_2$, identiques ou différents, représentent un atome d'hydrogène, un atome d'halogène, un radical alcoxyle ou alcoyle renfermant de 1 à 8 atomes de carbone, un radical $CF_3$ ou CN ou un noyau phényle éventuellement substitué par un halogène ou $T_1$ et $T_2$ forment ensemble un radical cycloalcoyle renfermant de 3 à 6 atomes de carbone ou un radical:

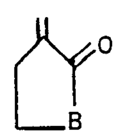

dans lequel B représente un atome d'oxygène ou de soufre;

*soit* $Z_2$ représente un radical:

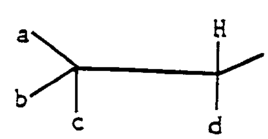

dans lequel a, b, c et d, identiques ou différents répresentent chacun un atome d'halogène,

*soit* $Z_2$ représente un radical

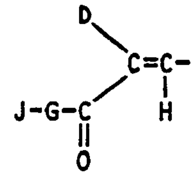

dans lequel D représente un atome d'hydrogène ou d'halogène, un radical alcoxyle renfermant de 1 à 8 atomes de carbone, G représente un atome d'oxygène ou de soufre et J représente un radical alcoyle linéaire [ou] ramifié saturé ou insaturé, renfermant de 1 à 8 atomes de carbone,

*ou bien A représente un radical:*

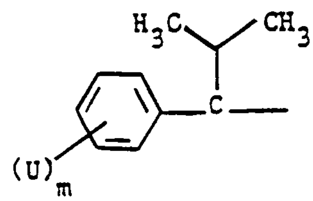

dans lequel U, en position quelconque sur le noyau benzénique, représente un atome d'halogène, un radical alcoyle renfermant de 1 à 8 atomes de carbone ou un radical alcoxy renfermant de 1 à 8 atomes de carbone, m représentant le nombre 0, 1 ou 2 et quand n est 2, les substituants peuvent être identiques différentes, et Z représente un atome d'hydrogène, un groupement C≡N, C≡CH, $CF_3$, ou un radical alkyle renfermant de 1 à 3 atomes de carbone et

celui des radicaux $R_2$ ou $R_3$ qui ne représente pas un radical:

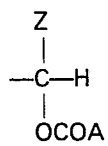

ainsi que les radicaux $R_4$ et $R_5$, identiques ou différents les uns des autres, représentent un atome d'hydrogène, un atome d'halogène, un radical alkyle renfermant jusqu'à 18 atomes de carbone, un radical aryle un radical phénylalkyle renfermant jusqu'à 18 atomes de carbone, un radical cyano, un radical $CF_3$, un radical $CO_2$ alkyle renfermant jusqu'à 8 atomes de carbone, un radical $NO_2$, un radical alkoxyle renfermant jusqu'à 8 atomes de carbone, un radical:

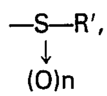 ou 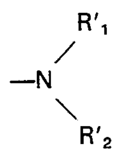

n étant égal à 0, 1 ou 2 et les radicaux R', $R'_1$ et $R'_2$ représentant un radical alkyle renfermant de 1 à 8 atomes de carbone, les radicaux $R_4$ et $R_5$ pouvant former un homocycle carboné saturé ou insaturé renfermant jusqu'à 8 atomes de carbone et

$R_1$ représente:

soit un radical 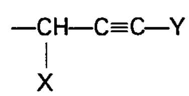

dans lequel X et Y, identiques ou différents l'un de l'autre, représentent un atome d'hydrogène, un atome d'halogène, un radical alkyle renfermant de 1 à 8 atomes de carbone ou un radical phényle

soit un radical 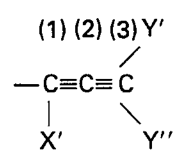

dans lequel X', Y' et Y'', identiques ou différents les uns des autres, représentent l'une des valeurs indiquées ci-dessus pour X et Y, les traits pointillés représentent une double liaison éventuelle entre les carbones 1 et 2;

soit un radical 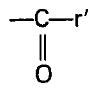

dans lequel r' peut avoir les valeurs indiquées précédemment pour $R_4$ et $R_5$, à l'exception d'halogène, cyano, $NO_2$,

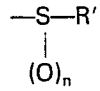

dans lequel n est égal à 1 ou 2 et

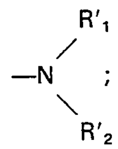 ; soit un radical 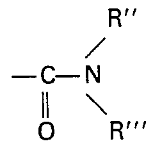

dans lequel R'' et R''', identiques ou différents l'un de l'autre, représentent un atome d'hydrogène, un radical alkyle renfermant de 1 à 18 atomes de carbone, un radical phenyle, un radical [phénylealkyle] renfermant jusqu'à 18 atomes de carbone, un radical $CF_3$, un radical $CO_2$-alkyle renfermant jusqu'à 8 atomes de carbone ou un radical alkoxy renfermant jusqu'à 8 atomes de carbone.

Lorsque $T_1$, $T_2$ ou $Z_3$ représentent un atome d'halogène, il s'agit de préférence d'un atome de fluor, de chlore ou de brome.

Lorsque $T_1$ ou $T_2$ représente un radical alcoyle ou alcoxyle, il s'agit de préférence du radical méthyle, éthyle, n-propyle, méthoxy, éthoxy ou n-propoxy.

a, b, c et d représentent de préférence un atome de chlore ou de brome.

Lorsque D représente un atome d'halogène, il s'agit de préférence d'un atome de fluor, de chlore ou de brome.

Lorsque J représente un radical alcoyle on entend de préférence par alcoyle un radical renfermant de 1 à 8 atomes de carbone comme, par exemple, le radical méthyle, éthyle, propyle, isopropyle, n-butyle, isobutyle ou terbutyle.

Lorsque Z représente un radical alkyle, il s'agit de préférence du radical méthyle.

Lorsque l'un des substituants mentionnés dans la définition de la formule générale I représente un radical alkyle, il s'agit de préférence du radical méthyle, éthyle, n-propyle, isopropyle, butyle, isobutyle ou n-pentyle.

Lorsque l'un des substituants mentionnés dans la définition de la formule générale I représente un atome d'halogène, il s'agit de préférence d'un atome de fluor, de brome ou de chlore.

Lorsque l'un des substituants mentionnés dans la définition de la formule générale I représente un radical [phénylalkyle,] ils s'agit de préférence d'un radical benzyle.

Lorsque l'un des substituants mentionnés dans la définition de la formule générale I représente un radical $CO_2$ alkyle ou un radical alkoxyle, on entend de préférence par "alkyle" un radical méthyle, éthyle, propyle ou isopropyle et par "alkoxyle" un radical méthoxy, éthoxy, propoxy ou isopropoxy.

Lorsque $R_4$ et $R_5$ forment un homocyle carboné, il s'agit de préférence d'un cycle à 4 ou 5 atomes de carbone.

L'invention a tout particulièrement pour objet les composés de formule I dans lesquels $R_1$ représente un radical $-CH_2-C \equiv CH$.

Parmi les composés préférés de l'invention, on peut citer les composés dans lesquels le substituant Z du radical:

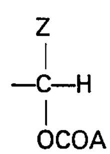

représente un atome d'hydrogène ou un radical cyano.

On peut citer aussi comme composés préférés de l'invention ceux dans lesquels c'est le substituants $R_3$ qui représente le radical:

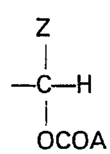

L'invention a tout particulièrement pour objet les composés de formule I dans lesquels les substituants

$R_2$, $R_4$ et $R_5$ représentent chacun un atome d'hydrogène ainsi que ceux dans lesquels l'un des radicaux $R_2$, $R_4$ et $R_5$ représente un radical nitro ou cyano ou un radical trifluorométhyle.

Parmi les composés préférés de l'invention, on peut citer les composés dans lesquels A représente un radical:

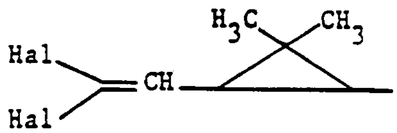

dans lequel Hal représente un atome d'halogène, par exemple un atome de chlore ou de brome.

On peut citer également les composés dans lesquels A représente un radical:

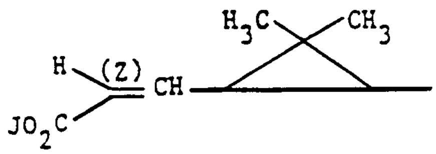

dans lequel J représente un radical alcoyle renfermant de 1 à 8 atomes de carbone, linéaire, ramifié ou cyclisé; la double liaison ayant la géométrie Z. Dans ces composés J représente de préférence le radical méthyle, éthyle, n-propyle, isopropyle ou terbutyle.

On peut citer aussi les composés de formule I dans lesquels A représente un radical:

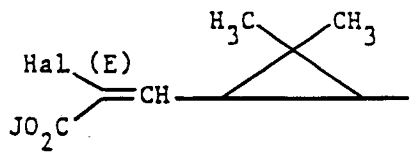

dans lequel Hal représente un atome d'halogène et J représente un radical alcoyle renfermant de 1 à 8 atomes de carbone, la double liaison ayant la géométrie (E).

Dans ces composés J représente de préférence le radical méthyle, éthyle, n-propyle, isopropyle ou terbutyle.

L'invention a tout spécialement pour objet les composés dans lesquels A représente le radical:

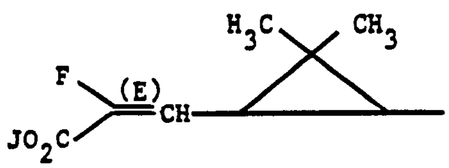

Parmi les composés préférés de l'invention, on peut citer les composés de formule I dont les noms suivent:

le 1R, cis (ΔE) 2,2-diméthyl 3-(3-éthoxy-3-oxo 2-fluoro-1-propényl)-cyclopropane carboxylate de [1-([2-propynyl) 1H-pyrrol-3-yl] méthyle;

le 1R, cis (ΔE) 2,2-diméthyl 3-(3-méthoxy-3-oxo 1-propényl)-cyclopropane carboxylate de [1-([2-propynyl) 1H-pyrrol-3-yl] méthyle;

le 1R, cis (ΔE) 2,2-diméthyl 3-(3-éthoxy-3-oxo 2-fluoro-1-propényl) cyclopropane carboxylate de [1-([2-propynyl) 2-trifluorométhyl 1H-pyrrol-3-yl] méthyle;

le 1R, cis (ΔE) 2,2-diméthyl 3-(3-terbutoxy 3-oxo 2-fluoro 1-propényl) cyclopropane carboxylate de [1-([2-propynyl) 2-trifluorométhyl (1H-pyrrol-3-yl] méthyle.

L'invention a également pour objet un procédé de préparation des composés de formule I caractérisé en ce que l'on soumet un alcool de formule:

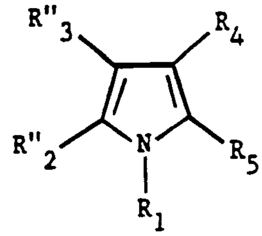

(II)

dans laquelle l'un des radicaux $R''_2$ et $R''_3$ représente un groupement

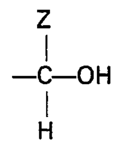

Z, conservant la même signification que précédemment, l'autre radical $R''_2$ ou $R'''_3$ ainsi que les radicaux $R_4$ et $R_5$ conservent la même signification que précédemment en $R_1$ conserve la même signification que précédemment, à l'action d'un acide de formule (III):

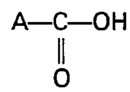

(III)

ou d'un dérivé fonctionnel de cet acide, A conservant la même signification que précédemment.

Le dérivé fonctionnel d'acide utilisé est de préférence un chlorure d'acide.

Lorsque l'on fait réagir l'acide de formule III sur l'alcool, on opère de préférence en présence de dicyclohexylcarbodiimide.

Les composés de formule (I) dans laquelle $R_1$ comporte une double liaison carbone-carbone peuvent aussi être préparés par un procédé caractérisé en ce que l'on traite le dérivé correspondant comportant une triple liaison, par une mole d'hydrogène en présence d'un catalyseur, par exemple le palladium sur sulfate de baryum et de quinoléine.

Les produits de formule II:

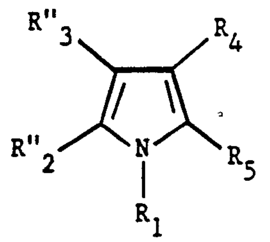

(II)

dans laquelle $R_1$, $R''_2$, $R''_3$, $R_4$ et $R_5$ conservent la même signification que précédemment, sont des produits nouveaux; l'invention a donc pour objet ces produits à titre de produits industriels nouveaux utiles notamment comme produits intermédiaires de préparation des produits de formule I. Les produits de formule II peuvent être obtenus en introduisant un ou plusieurs groupements sur le noyau pyrrole, ou par synthèse totale de cet hétérocycle suivie éventuellement de l'introduction d'un ou plusieurs groupements fonctionnels sur le noyau pyrrole ainsi formé.

C'est ainsi, par exemple, que l'on peut obtenir les différents produits de formule II décrits en exemple d'après les schèmes de synthèse suivants:

Schéma 1 :

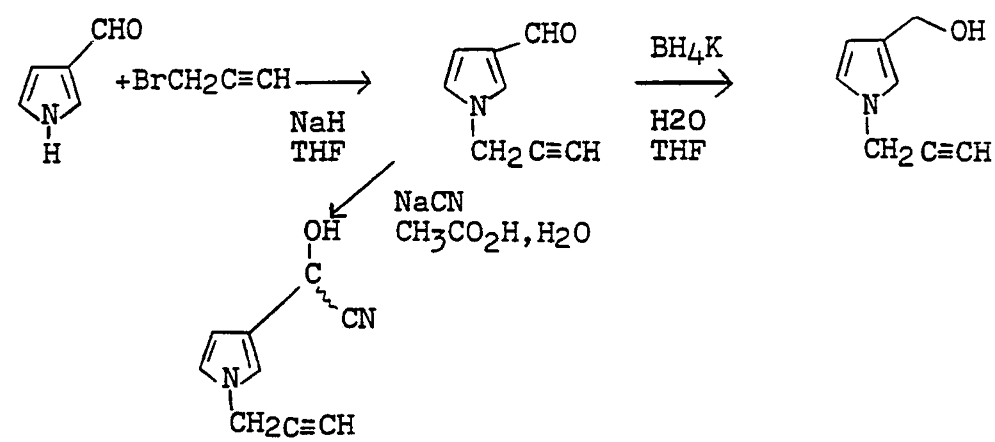

Schéma 2 :

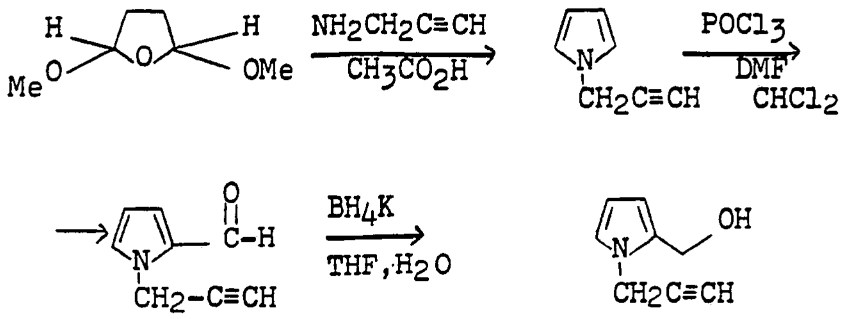


Schéma 3 :

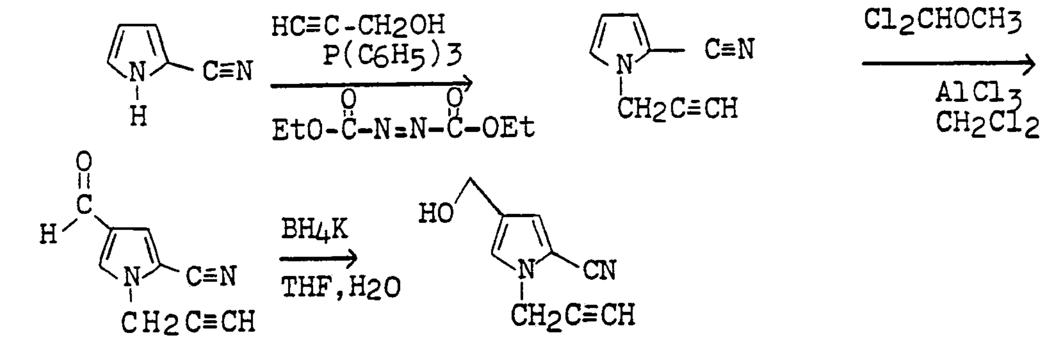


SCHEMA 4 :

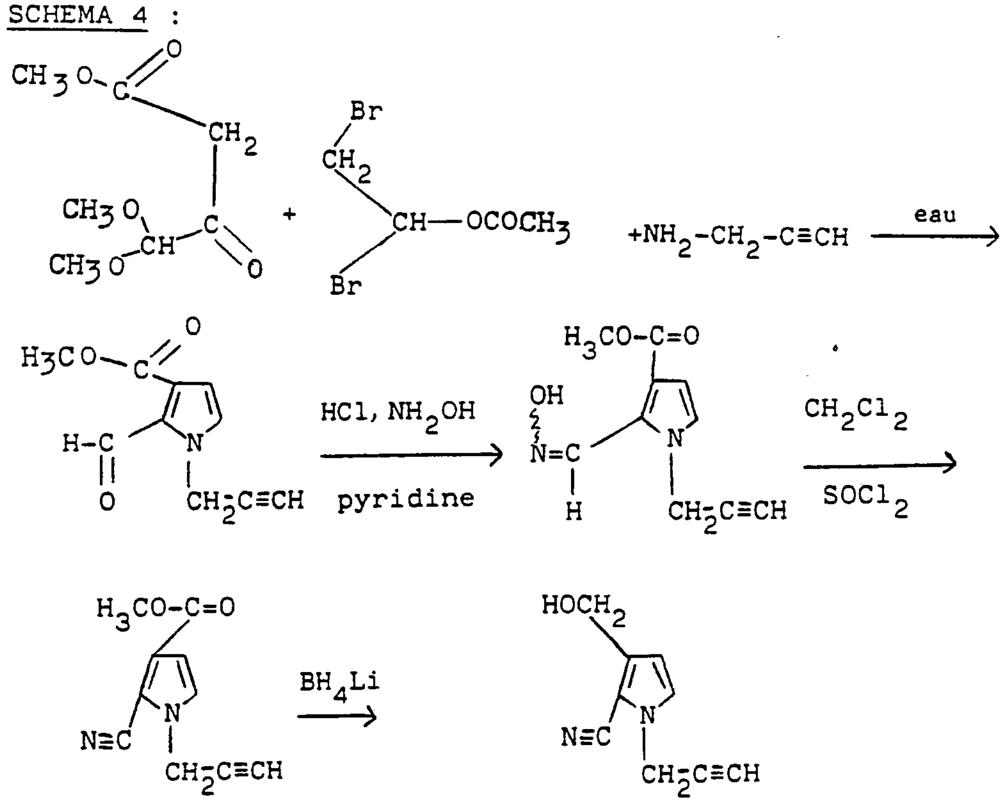

SCHEMA 5 :

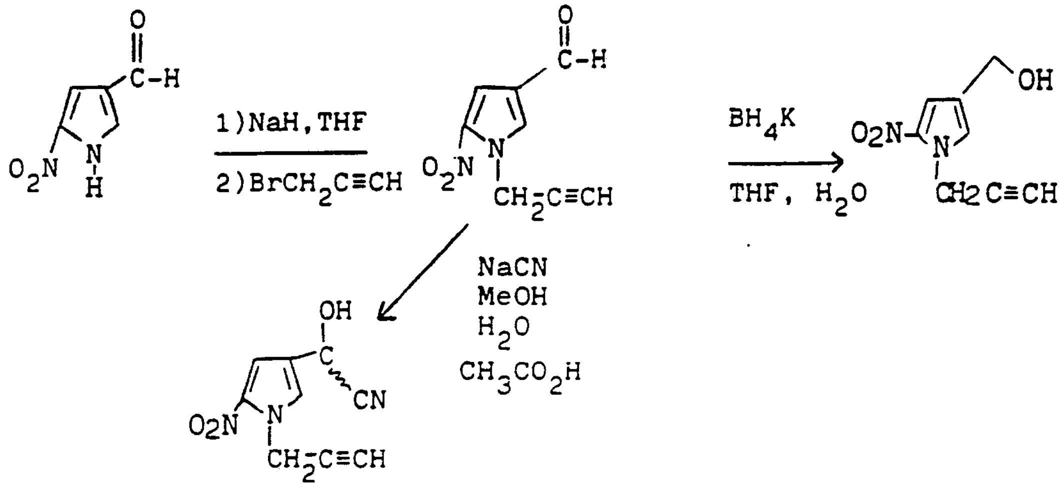


SCHEMA 6 :

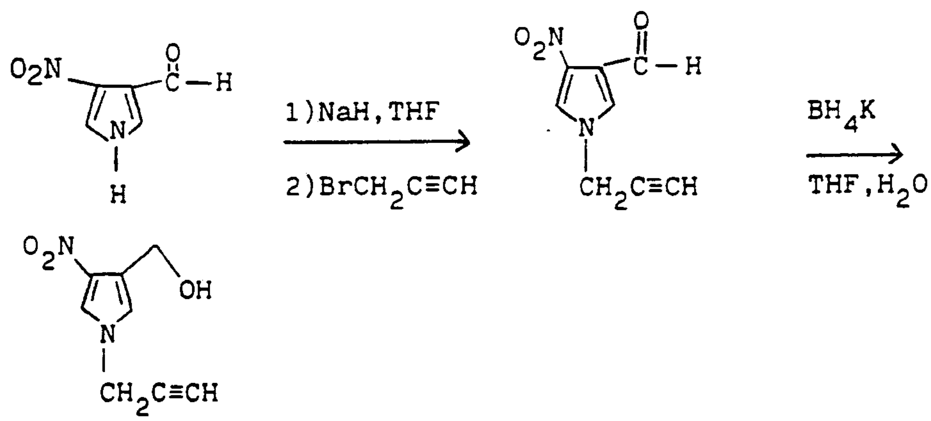


SCHEMA 7 :

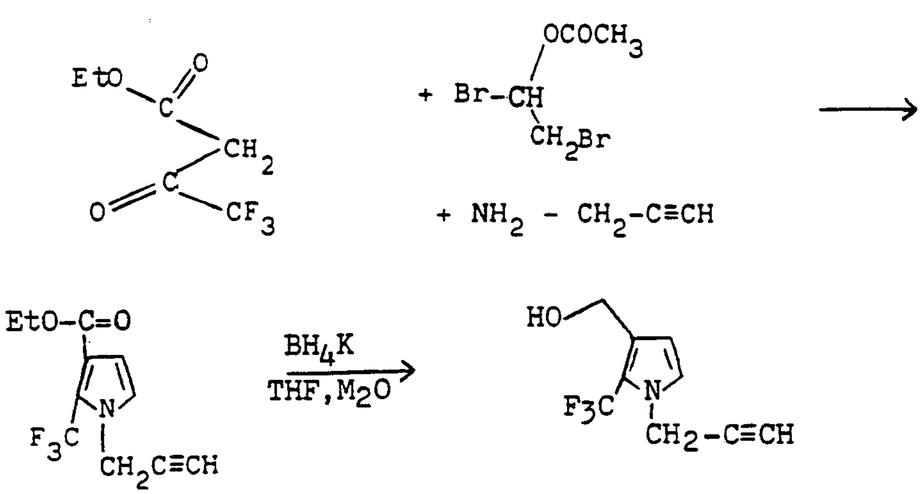

**Schéma 8 :**

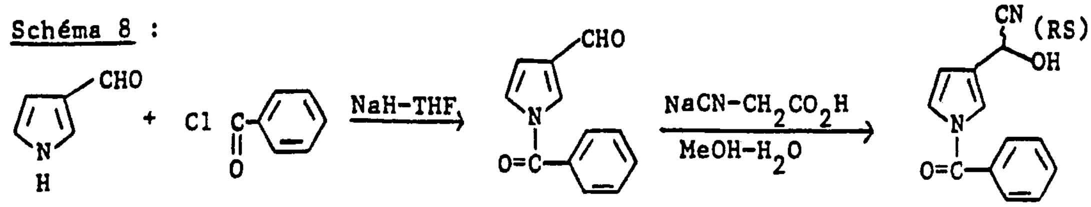


**Schéma 9 :**

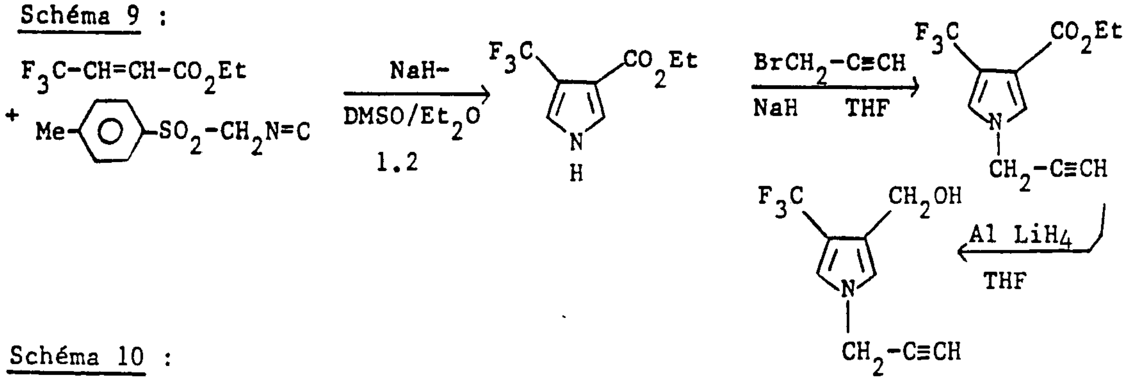


**Schéma 10 :**

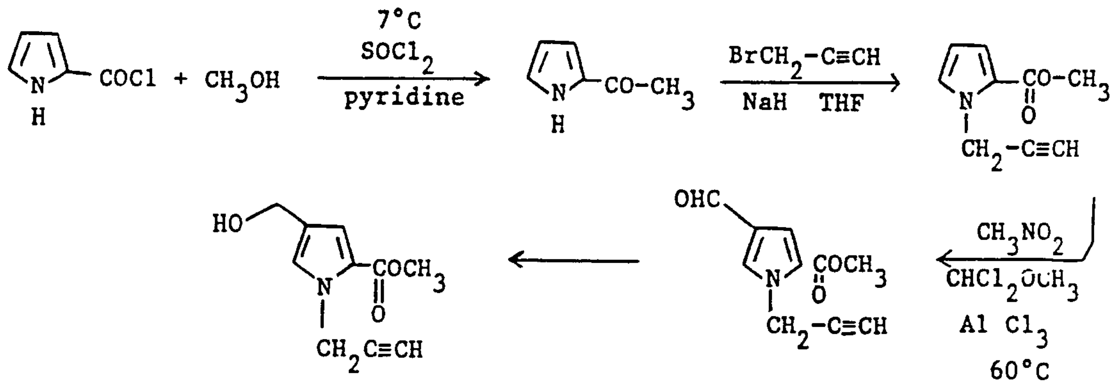


**Schéma 11 :**

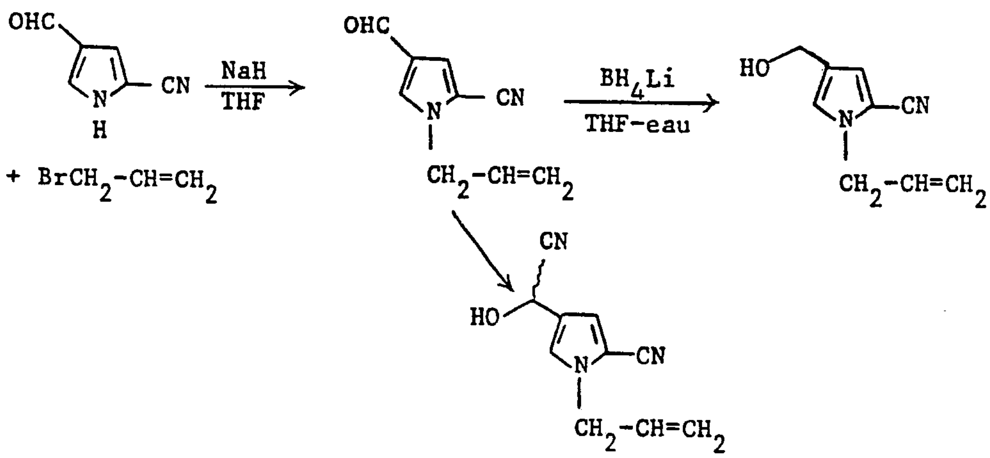


**Schéma 12 :**

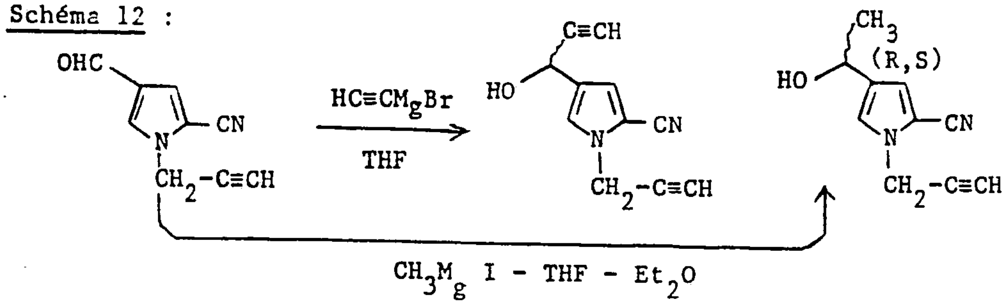

Schéma 13 :

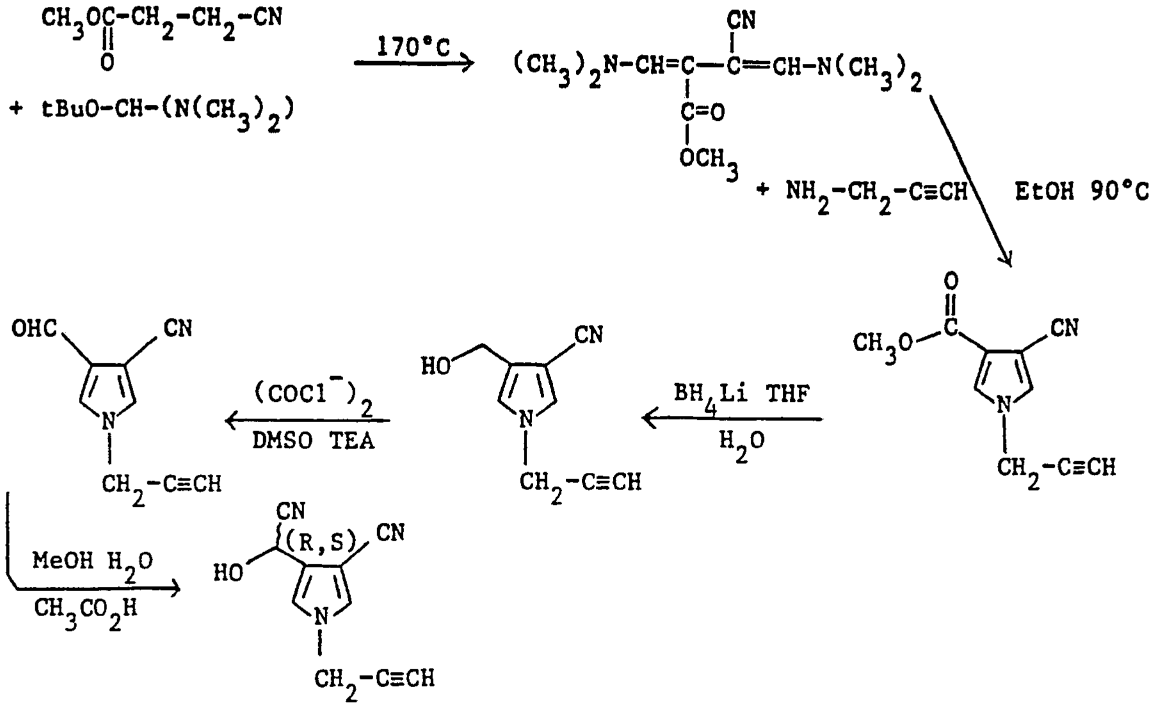


Schéma 14 :

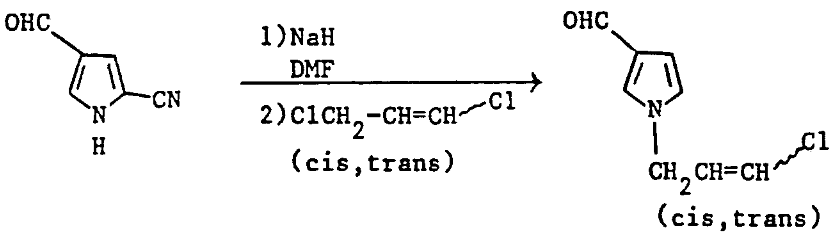


Schéma 15 :

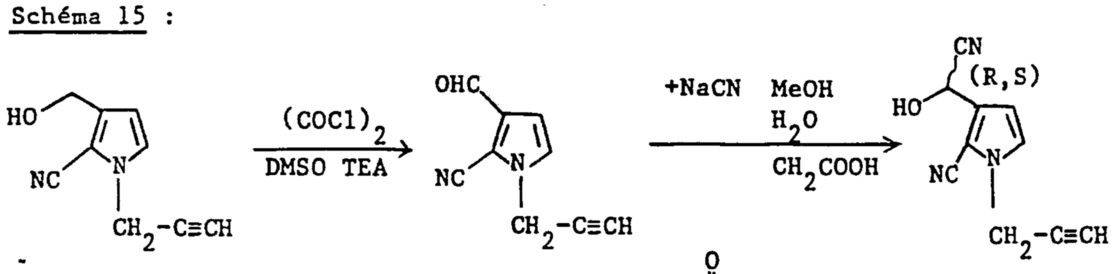


Schéma 16 :

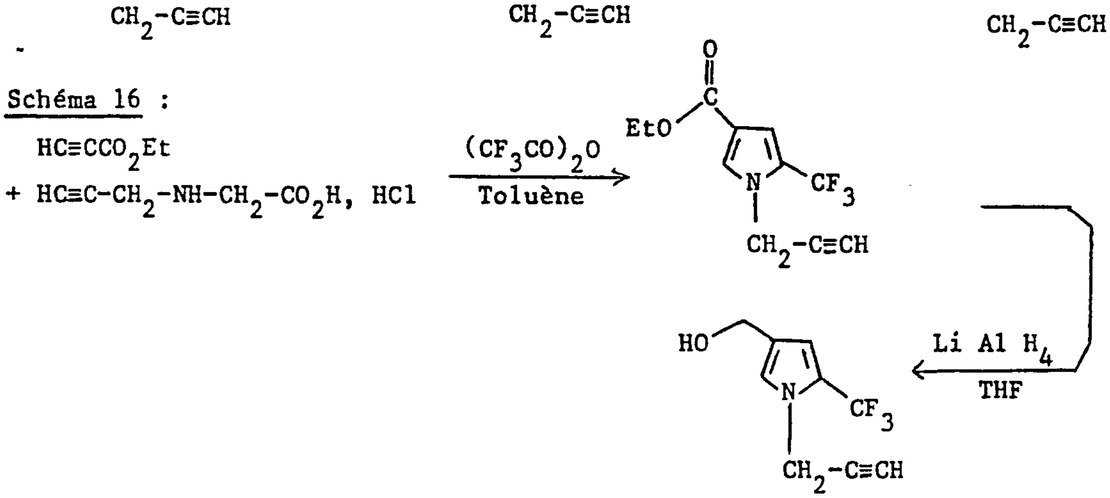

L'ensemble des produits de formule II peut être fait par les procédés schématisés ci-dessus ou par des procédés analogues à ceux-ci, évidents pour l'homme de métier.

Il va de soi que les produits de formule II préférés sont les produits décrits en exemple.

Lorsque l'on veut préparer des composés de formule (I) dans lesquels Z représente un radical-CN, on peut également utiliser une variante du procédé précédent appelé procédé par transfert de phase décrit par exemple dans le brevet belge 851 900, qui consiste à faire réagir un aldéhyde correspondant à l'alcool de formule II et un acide de formule III, en présence d'eau, d'un cyanure alcalin dilué dans l'eau, d'un solvant aprotique sensiblement non miscible à l'eau et d'un catalyseur de transfert de phase.

Lorsque l'on veut préparer un alcool de formule II dans laquelle trois des substituants $R''_2$ ou $R''_3$, $R_4$ et $R_5$ représentent un atome d'hydrogène, on utilise, de préférence, le procédé correspondant au schéma 1 ou 2 selon que c'est $R''_3$ ou $R''_2$ qui représente un radical:

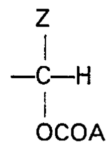

Lorsque l'on veut préparer un alcool de formule II dans lequel le noyau pyrrole renferme un groupement-CN en 2, on utilise de préférence le procédé correspondant au schéma 3 ou au schéma 4 selon les positions respectives des groupements-CN et hydroxyméthyle.

Lorsque l'on veut préparer un alcool de formule II dans lequel le noyau pyrrole renferme un groupement $NO_2$ en 2, on utilise de préférence le schéma 5.

Lorsque l'on veut préparer un alcool de formule II dans lequel le noyau pyrrole renferme un groupement $NO_2$ en 3, on utilise de préférence le schéma 6.

Lorsque l'on veut préparer un alcool de formule II dans lequel le noyau pyrrole renferme un radical $CF_3$ en 2, on utilisé de préférence un procédé correspondant au schéma 7.

Les composés de formule (I) présentent d'intéressantes propriétés qui permettent leur utilisation dans la lutte contre les parasites; il peut s'agir par exemple de la lutte contre les parasites des végétaux, les parasites des locaux et les parasites des animaux à sang chaud. C'est ainsi que l'on peut utiliser les produits de l'invention pour lutter contre les insectes, les nématodes et les acariens parasites des végétaux et des animaux.

L'invention a donc pour objet l'application des composés de formule (I) à la lutte contre les parasites des végétaux, les parasites des locaux et les parasites de animaux à sang chaud.

Les produits de formule (I) peuvent donc être utilisés notamment pour lutter contre les insectes dans la domaine agricole, pour lutter par exemple contre les pucerons, les larves de lépidoptères et les coléoptères. Ils sont utilisés à des doses comprises entre 10 g et 300 g de matière active à l'hectare.

Les produits de formule (I) peuvent également être utilisés pour lutter contre les insectes dans les locaux, pour lutter notamment contre les mouches, les moustiques et les blattes.

Les composés des exemples 1, 2, 15 et 40 notamment, présentent des propriétés insecticides tout à fait remarquables, en particulier un très bon pouvoir de knock down.

Les produits de formule (I) peuvent aussi être utilisés pour lutter contre les acariens parasites de végétaux.

Le composé de l'exemple 40 notamment présente de remarquables propriétés acaricides comme le montrent les résultats de tests biologiques ci-après.

Les composés de formule (I) peuvent aussi être utilisés pour lutter contre les nématodes parasites de végétaux.

Les composés de formule (I) peuvent encore être utilisés pour lutter contre les acariens parasites des animaux, pour lutter par exemple contre les tiques et notamment les tiques de l'espèce Boophilus, ceux de l'espèce Hyalomnia, ceux de l'espèce Amblyomnia et ceux de l'espèce Rhipicephalus, ou pour lutter contre toutes sortes de gales et notamment la gale sarcoptique, la gale psoroptique et la gale chorioptique.

L'invention a donc également pour objet les compositions destinées à la lutte contre les parasites des animaux à sang chaud, les parasites des locaux et des végétaux, caractérisées en ce qu'elles renferment au moins l'un des produits définis ci-dessus.

L'invention a notamment pour objet les compositions insecticides renfermant comme principe actif au moins l'un des produits définis ci-dessus.

Les compositions selon l'invention sont préparées selon les procédés usuels de l'industrie agrochimique ou de l'industrie vétérinaire ou de l'industrie des produits destinés à la nutrition animale.

Ces compositions peuvent se présenter sous forme de poudres, granules, suspensions, émulsions, solutions, solutions pour aérosols, bandes combustibles, appâts ou autres préparations employés classiquement pour utilisation de ce genre de composés.

Outre le principe actif, ces compositions contiennent, en général, un véhicule et/ou un agent tensioactif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005% à 10% en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un électromosquito destroyer.

Dans le cas où l'on utilise un serpentin insecticide, le support inerte peut être, par exemple, composé de marc de pyrèthre, poudre de Tabu (ou poudre de feuilles de Machilus Thumbergii), poudre de tige de pyrèthre, poudre de feuille de cèdre, poudre de bois (telle que la sciure de pin) amidon et poudre de coque de noix de coco. La dose de matière active peut alors être, par exemple, de 0,03 à 1% en poids.

Dans le cas où l'on utilise un support fibreux incombustible, la dose de matière active peut alors être, par exemple, de 0,03 à 95% en poids.

Les compositions selon l'invention pour un usage dans le locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95% en poids.

L'invention a également pour objet, les compositions acaricides renfermant comme principe actif au moins un des produits de formule (I) définie ci-dessus.

Parmi les produits de formule (I) tout particulièrement préférés pour l'usage acaricide, on peut citer les produits des exemples 38 et 40.

L'invention a également pour objet les compositions nématicides renfermant comme principe actif au moins un des produits de formule (I) ci-dessus.

Les compositions insecticides selon l'invention, comme les compositions acaricides et nématicides peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire, contenant de 1 à 80% ou des liquides pour pulvérisation foliaire contenant de 1 à 500 g/l de principe actif. On peut également employer des poudres pour poudrage foliaires contenant de 0,05 à 3% de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

L'invention a également pour objet les compositions acaricides destinées à la lutte contre les acariens parasites des animaux à sang chaud, notamment contre les tiques et les gales, caractérisées en ce qu'elles renferment comme principe actif, au moins l'un des produits de formule (I) définie ci-dessus.

Pour exalter l'activité biologique des produits de l'invention, on peut les additionner à des synergistes classiques utilisé en pareil cas tel le 1-(2,5,8--trioxadodécyl) 2-propyl 4,5-méthylènedioxy benzène (ou butoxyde de pipéronyle) ou la N-(2-éthyl heptyl) bicyclo[2,2-1]5-heptène-2,3-dicarboximide, ou le pipéronyl-bis-2-(2'-n-butoxy éthoxy) éthylacétal (ou tropital).

Lorsqu'il s'agit de lutter contre les acariens parasites des animaux, on incorpore très souvent les produits de l'invention dans des compositions alimentaires en association avec un mélange nutritif adapté à l'alimentation animale. Le mélange nutritiel peut varier selon l'espèce animale, il peut renfermer des céréales, des sucres et des grains, des tourteaux de soja, d'arachide et de tournesol, des farines d'origine animale, par exemple des farines de poissons, des acides aminés de synthèse, des sels minéraux, des vitamines et des anti-oxydants.

L'invention a donc également pour objet les compositions destinées à l'alimentation animale, renfermant comme principe actif au moins l'un des produits de formule (I) telle que définie précédemment.

On peut indiquer également que les produits de l'invention peuvent être utilisés comme biocides ou comme régulateurs de croissance.

L'invention a également pour objet les associations douées d'activité insecticide, acaricide ou nématicide, caractérisées en ce qu'elles contiennent comme matière active, d'une part un au moins des composés de formule générale (I), et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools α-cyano 3-phénoxy benzyliques des acides 2,2-diméthyl 3-(2,2-dichlorovinyl) cyclopropane-1-carboxyliques, par les esters d'alcool-α-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones, d'alcool 3,4,5,6-tétrahydrophthalimido méthylique, d'alcool 5-benzyl 3-furyl méthylique, d'alcool 3-phénoxy benzylique et

d'alcools α-cyano 3-phénoxy benzyliques des acides 2,2-diméthyl 3-(1,2,2,2-tétrahalo éthyl)cyclopropane-1-carboxyliques, dans lesquels "halo" représente un atome defluor, de chlore ou de brome, étant entendu que les composés (I) peuvent exister sous toutes leurs formes stéréoisomères possibles de même que les copules acides et alcools des esters pyréthrinoïdes ci-dessus.

Les associations selon l'invention présente notamment l'intérêt soit de permettre de combattre, par la polyvalence de leur action, une gamme de parasites, plus étendue, soit de manifester, dans certains cas, un effet de synergie.

L'invention a pour objet les compositions pesticides définies précédemment, caractérisées en ce qu'elles renferment en outre un synergiste des pyréthrinoïdes.

Comme synergistes classiques utilisés en pareil cas, on peut citer le 1-(2,5,8-trioxadodécyl) 2-propyl 4,5-méthylène dioxy benzène (ou butoxyde de pipéronyle) ou le N-(2-éthyl heptyl) bicyclo [2,2-1] 5-heptène 2,3-dicarboximide, ou le pipéronyl-bis-2-(2'-n-butoxy éthoxy) éthyl acétal (ou tropital).

Les exemples suivants illustrent l'invention sans toutefois la limiter.

Exemple 1

1R,cis(ΔZ)-3-(3-méthoxy-3-oxo 1-propenyl)-2,2-diméthyl-cyclopropane carboxylate de [1-(2-propynyl)-1H-pyrrol-3-yl] méthyle

On dissout 542 mg de 3-(1-(2-propynyl)pyrrolyl) méthanol, 10 $cm^3$ de chlorure de méthylène, ajoute en refroidissant à 0°/5°c 795 mg d'acide (1 Rcis) 2,2-diméthyl 3-(3-oxo, 3-methoxy, 1(ΔZ) propényle)cyclopropane carboxylique et agite pendant 10 minutes. On ajoute goutte à goutte à 7°c une solution renfermant 8 $cm^3$ de chlorure de méthylène, 835 mg de dicyclohexylcarbodiimide et 5 mg de diméthylaminopyridine. Lorsque l'addition est terminée, on laisse revenir à la température ambiante le mélange réactionnel et le maintient sous agitation pendant 16 heures. On filtre, concentre à sec le filtrat. On reprend à l'éther l'extrait sec. On filtre pour éliminer l'insoluble. On concentre le filtrat et obient 1,112 g de produit brut que l'on chromatographie sur silice en éluant par le mélange hexane-éther isopropylique, (55—45) avec 2% de triéthylamine. On obtient un produit que l'on purifie à nouveau dans l'éther isopropylique. On obtient ainsi 700 mg de produit recherché.

$\alpha_D = +23° \pm 2°$ (c = 0,3% toluène)

En opérant, comme à l'exemple 1, en suivant les schémas indiqués ci-après, on a obtenu les produits suivants:

Exemple 2

1R,cis (ΔE)-2,2-diméthyl-3-(3-éthoxy-3-oxo-2-fluoro-1-propényl)-cyclopropane carboxylate de [1-(2-propynyl)-1 H-pyrrol-3-yl] méthyle.

Exemple 3

1R,cis (ΔZ)-2,2-diméthyl 3-(3-terbutyloxy-3-oxo-1-propényl)-cyclopropane carboxylate de [1-(2-propynyl)-1 H-pyrrol-3-yl] méthyle.

Exemple 4

1R,cis(ΔE)-2,2-diméthyl 3-(3 méthoxy-3 oxo-2 fluoro-1-propényle) cyclopropane carboxylate de [1-(2-propynyl)-1 H-pyrrol-3-yl] méthyle.

Exemple 5

1R,cis(ΔE)-2,2-diméthyl 3-(3-terbutoxy-3-oxo-2-fluoro-1-propényl) cyclopropane carboxylate de [1-(2-propynyl 1 H-pyrrol-3-yl] méthyle.

Exemple 6

1R,cis 2,2-diméthyl 3-(dibromoéthényl) cyclopropane carboxylate de [1-(2-propynyl)-1 H pyrrol-3-yl] méthyle.

Exemple 7

1R,cis (ΔZ)-2,2-diméthyl 3-(3-éthoxy-3-oxo-1-propényl) cyclopropane carboxylate de [1-(2-propynyl)-1 H pyrrol-3-yl] méthyle.

Exemple 8

1R,trans 2,2-diméthyl 3-(2,2-difluoroéthényl) cyclopropane carboxylate de 1-[1-(2-propynyl)-1 H-pyrrol-3-yl]méthyle.

Exemple 9

1R,cis 2,2-diméthyl 3-(2,2-difluoroéthényl) cyclopropane carboxylate de 1-[1-(2 propynyl)-1 H-pyrrol-3-yl] méthyle.

Exemple 10

2,2-diméthyl-3,3-diméthyl-cyclopropane carboxylate de [1-(2-propynyl) 1 H pyrrole-3 yl] méthyle.

Exemple 11

[2S]-2-(4-difluorométhoxy phényl) 3-méthyl butyrate de [1-[2-propynyle) 1 H-pyrrol-3 yl] méthyle.

Exemple 12

1R,trans (ΔZ) et (ΔE) 2,2-diméthyl-3-(3,3,3-trifluoro-2-chloro-1-propényl) cyclopropane carboxylate de [1-(2-propynyl) 1 H-pyrrol-3-yl] méthyle.

Exemple 2 :

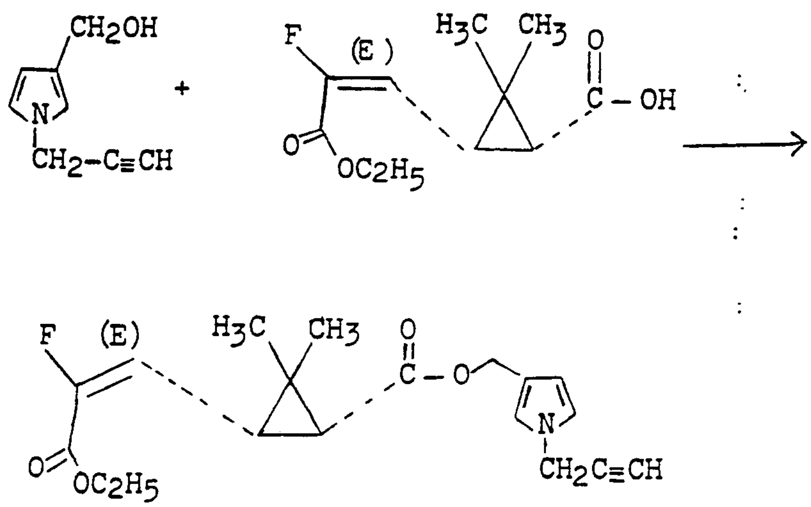

$\alpha_D$ = + 5° ± 2° (c=0,3% toluène)

Exemple 3 :

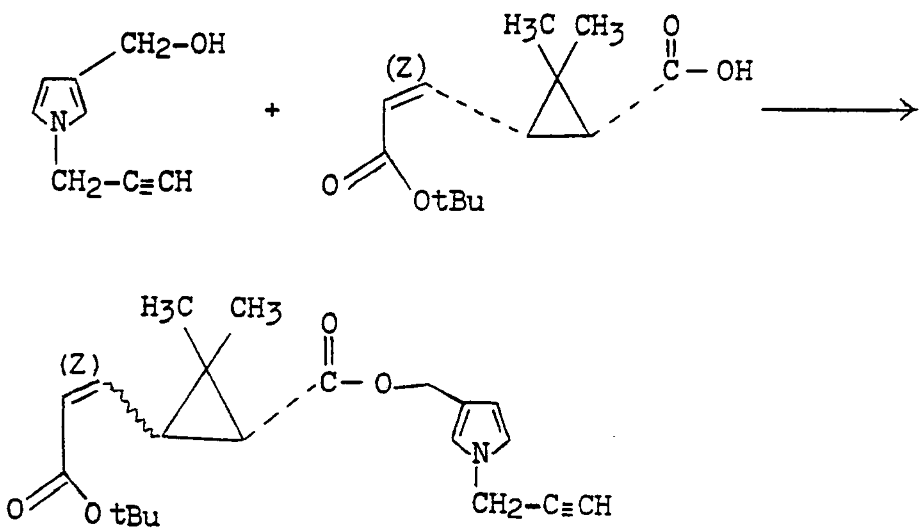

F=104°c $\alpha_D$= +33°±1,5°(c=1% toluène)

Exemple 4 :

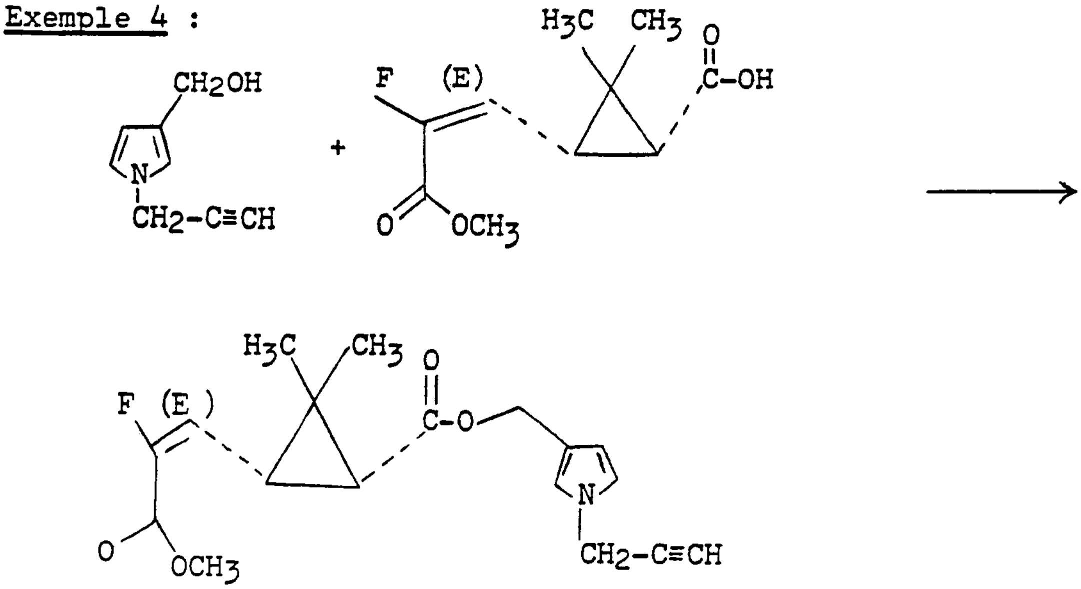

α D = +15°±3° (c=0,3% toluène)

Exemple 5 :

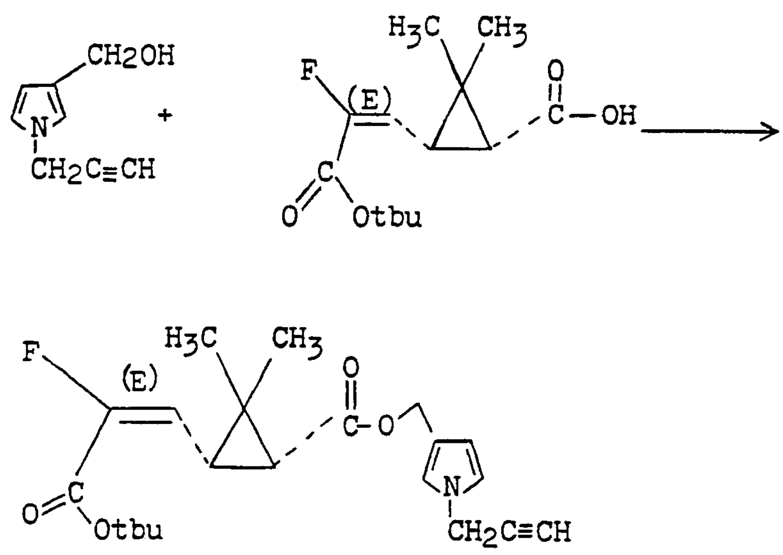

F = 63°c

α D = +19,5° ± 1,5° (c=1% toluène)

Exemple 6 :

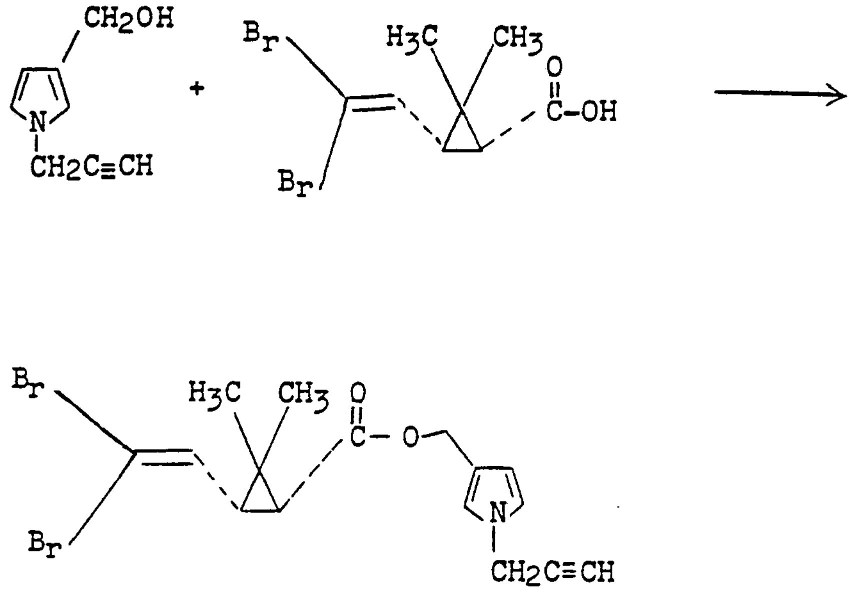

$\alpha_D$ = -15,5° (c=0,5 % toluène)

Exemple 7 :

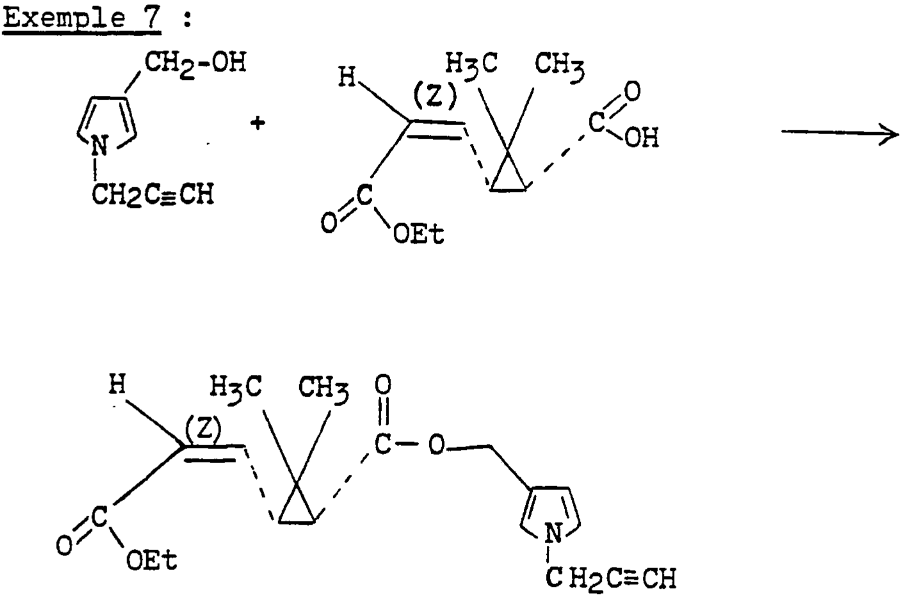

F = 50°c

$\alpha_D$ = +23° ± 1° (c=1% toluène)

Exemple 8 :

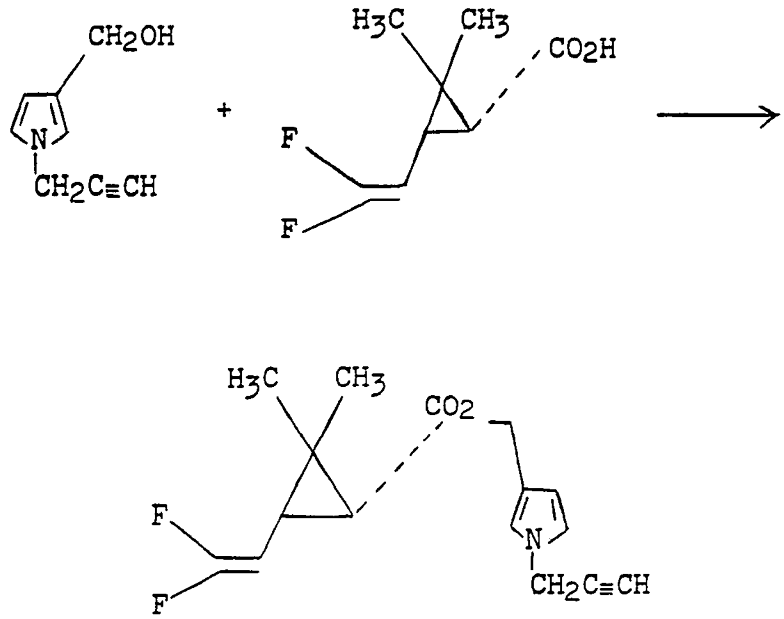

$\alpha_D$ = -34° ± 1° (c = 1,5 % toluène)

Exemple 9 :

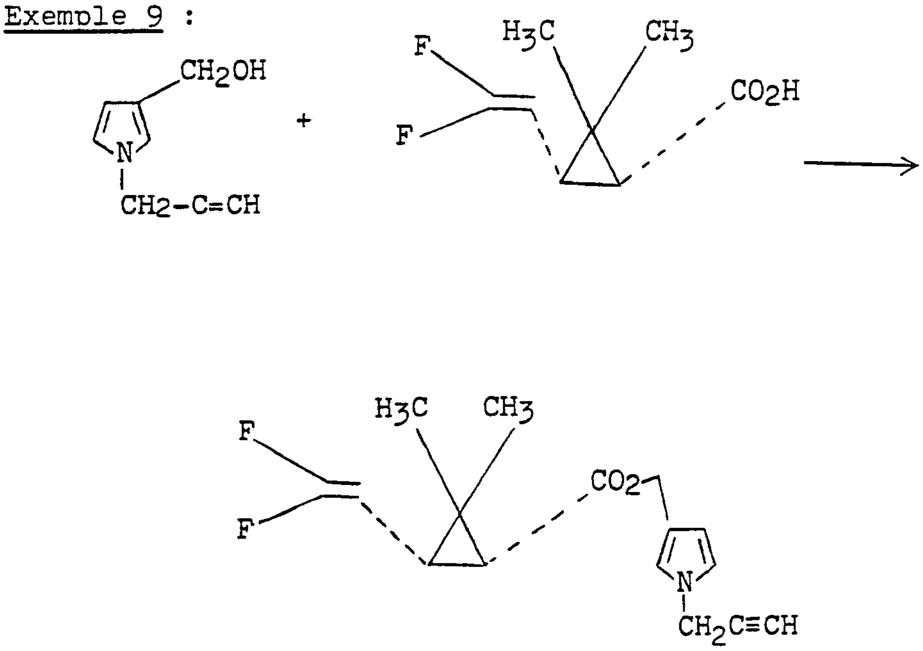

$\alpha_D$ = -27° ± 2° (c = 0,5 % toluène)

Exemple 10 :

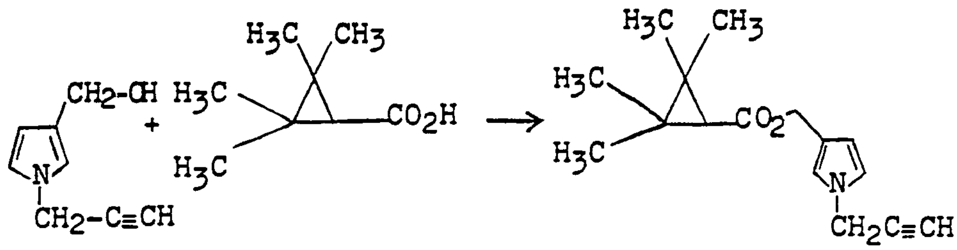

Exemple 11 :

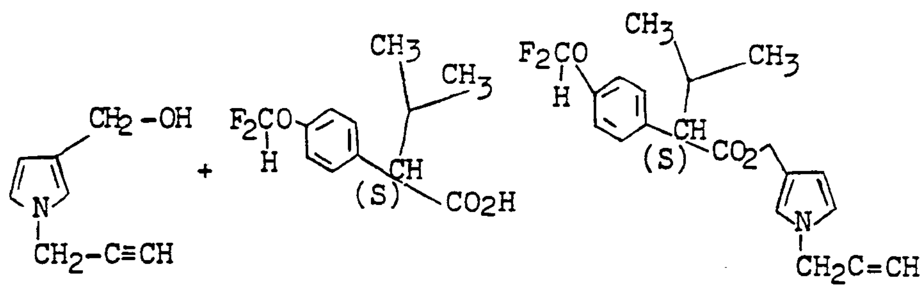

$\alpha_D$ = +5° ±4° ( c = 0,3 % toluène)

Exemple 12 :

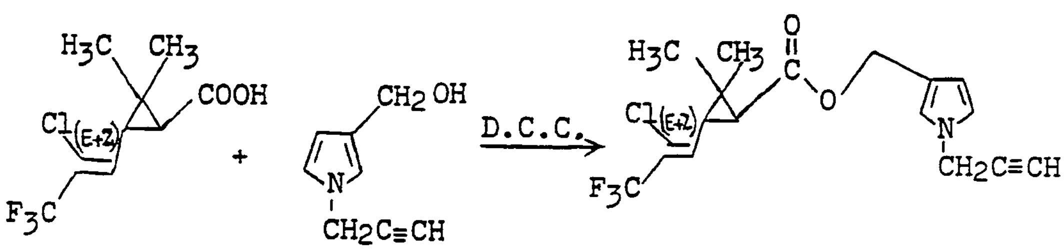


$\alpha_D$ = +6,5° ±4° (c = 0,3 % toluène)

Preparation 1

Le 1-(2-propynyl) 1 H-pyrrole-3-méthanol utilisé comme produit du départ des produits 1 à 11 est préparé de la façon suivante:

*STADE A:* 1-(2-propynyl)-1 H-pyrrole 3-carboxaldehyde

On dissout 2,924 g de pyrrole-3-carboxaldehyde (J. ORG. CHEM. 1981, *46*, 839 dans 45 $cm^3$ de tétrahydrofurane. On ajoute 1,488 g d'hydrure de sodium à 50% dans l'huile. On agite 10 minutes à froid puis 40 minutes à la température ambiante. On refroidit et ajoute 2,5 $cm^3$ de bromure, de propargyle. On agite à 5°C pendant 1 H 30. On ajoute 2 $cm^3$ de bromure de propargyle et agite à 5°C pendant 1 heure. On verse dans l'eau et extrait au chlorure de méthylène. On distille le solvant à la pression atmosphérique. On obtient 2,868 g de produit que l'on purifié par chromatographiesur silice en éluant par le mélange hexane-acétate d'éthyle (65—35).

On obtient ainsi 2,868 g du produit recherché.

*STADE B:* 1-(2-propynyl) 1 H pyrrole 3-méthanol

On dissout 3,1 g de produit préparé au stade A dans une solution de 90 $cm^3$ de tétrahydrofuranne et 19 $cm^3$ d'eau. On agite à la température ambiante pendant 15 minutes et ajoute 2,517 g d'hydrure de bore et de potassium. On agite à la température ambiante pendant 5 H 30. On verse le mélange réactionnel sur une solution acqueuse saturée en chlorure de sodium. On extrait au chlorure de méthyle. On sèche et concentre à sec à 20°C. On obtient 3,4 g de produit brut que l'on utilise tel.

En opérant comme à l'exemple 1, en suivant les schèmas indiqués, on a obtenu les produits suivants:

Exemple 13

1R,cis-2,2-diméthyl-3-(2,2-dibromoéthényl) cyclopropane carboxylate de (R,S)-cyano [1-(2-propynyl) 1 H pyrrol-3-yl] méthyle, que l'on sépare en ses deux isomères A et B.

Exemple 14

1R,cis (ΔZ) 2,2-diméthyl 3-(3-méthoxy-3-oxo-1-propényl) cyclopropane carboxylate de (R,S)-cyano [1-(2-propynyl) 1 H pyrrol-3-yl] méthyle.

Exemple 15

1R,cis (ΔE) 2,2-diméthyl 3-(3-éthoxy-3-oxo-2-fluoro-1-propényl)-2,2-diméthyl cyclopropane carboxylate de cyano [1-(2-propynyl) 1 H pyrrol-3-yl] méthyle (isomères A et B).

Exemple 16

isopropyl-4 chloro-phényl acétate de (R,S) cyano [1-(2-propynyl) 1 H pyrrol-3-yl] méthyle.

Exemple 13 :

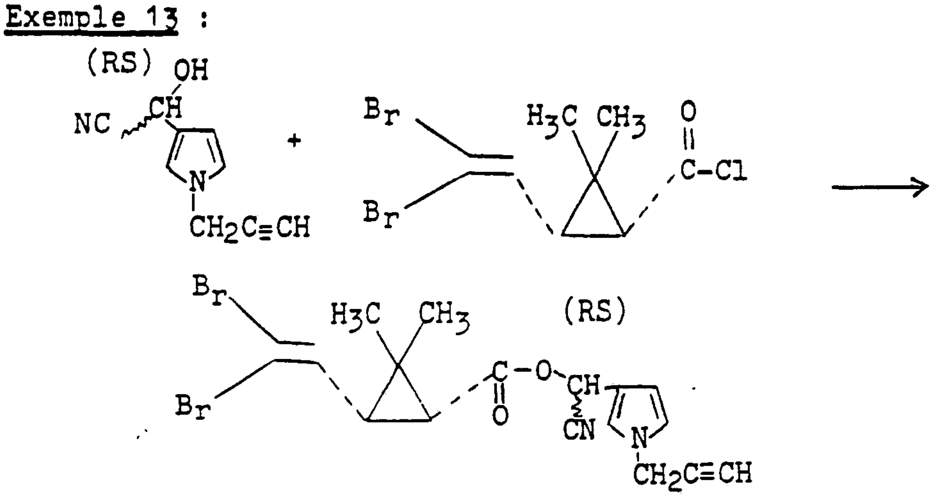

$\alpha_D = -2° \pm 2°$ (c=0,2 % toluène)

Exemple 14 :

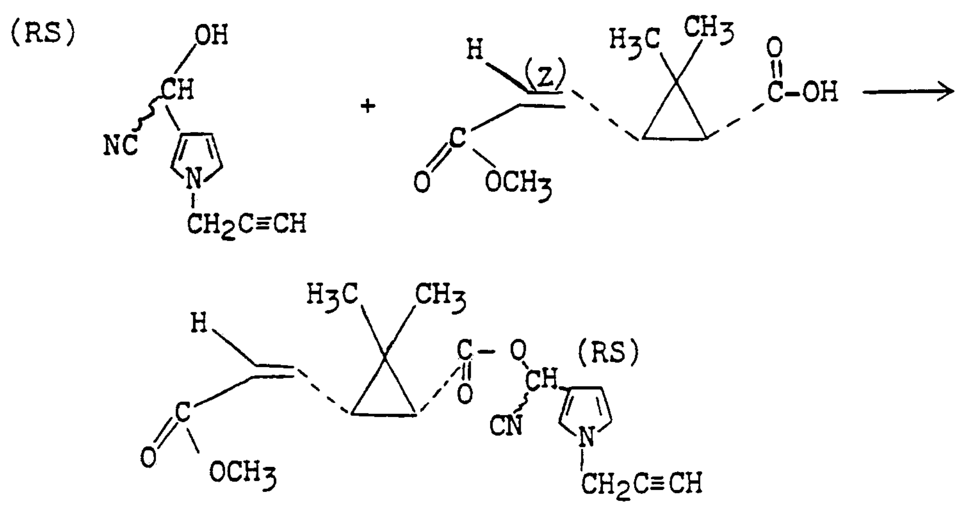

$\alpha_D = +24° \pm 2°$ c (c=0,5 % toluène)

Exemple 15 :

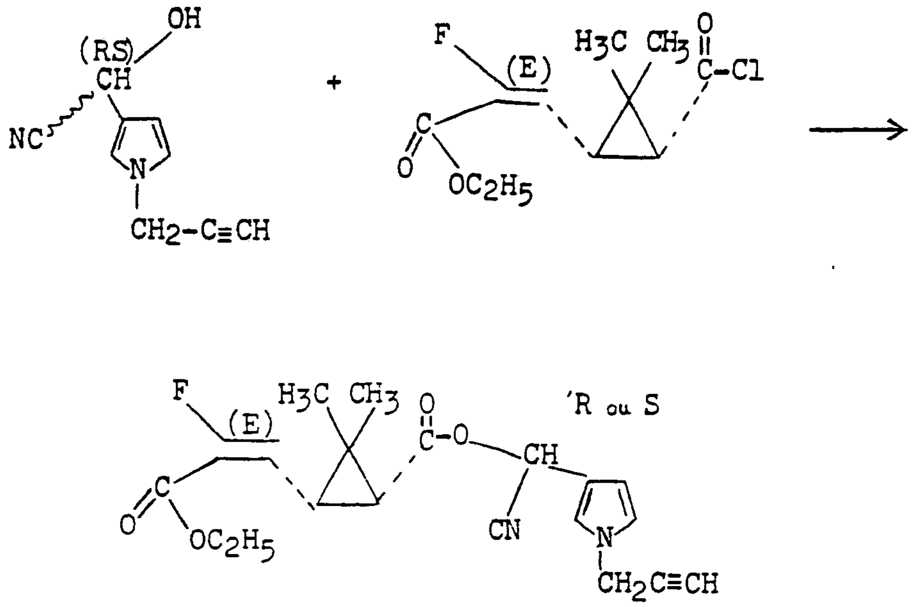

$\alpha_D = -4,5° \pm 2°$ (c = 0,3% toluène)

ou $+42° \pm$ (c = 0,5% toluène)

Exemple 16 :

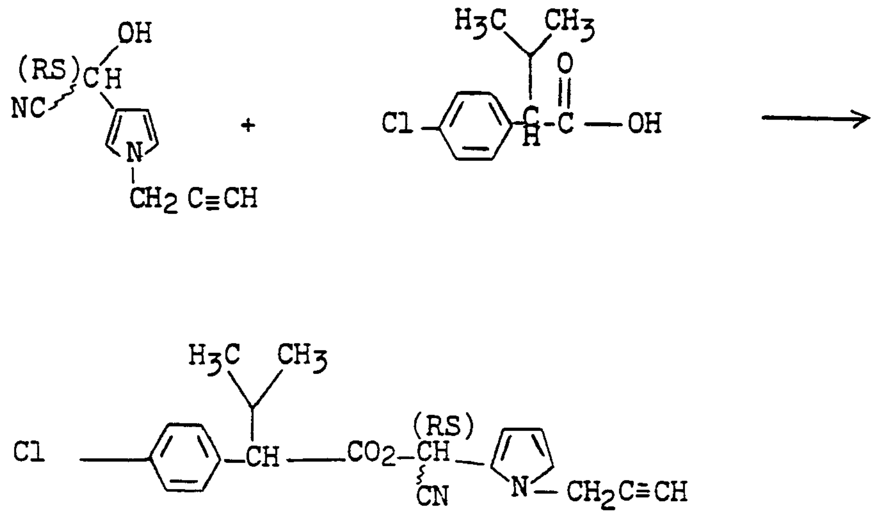

$\alpha_D = -20,5° \pm 2°$ (C=0,5% toluène)

Préparation 2

L' α-hydroxy-1(2-propynyl)-1 H pyrrole 3-acétonitrile utilisé comme produit de départ des exemples 13 à 15 est préparé de la façon suivante:

On ajoute 0,44 $cm^3$ d'acide acétique dans une solution renfermant 510 mg de produit préparé au stade B de la préparation 1,7 $cm^3$ de méthanol et 2 $cm^3$ d'eau. On ajoute 0.44 $cm^3$ d'acide acétique et refroidit à 20°C. On ajoute 2 82 mg de cyanure de sodium. Le mélange est agité pendant 1 H 30 à 20°C, puis refroidi à +10°C. On ajoute à nouveau 1,4 g de cyanure de sodium et 2,2 $cm^3$ d'acide acétique. On agite pendant 4 heures, verse le mélange réactionnel dans l'eau et extrait à l'acétate d'éthyle. On lave la phase organique à l'eau et la sèche. On concentre à sec. On obtient 590 mg de produit recherché.

Exemple 17

En opérant comme à l'exemple 1, en suivant le schéma indiqué, on obtient le (1 R,cis) 2,2-diméthyl-3-(2,2-dibromoéthényl) cyclopropane carboxylate de [1-(2-propynyl) 1 H pyrrol-2 yl]méthyle.

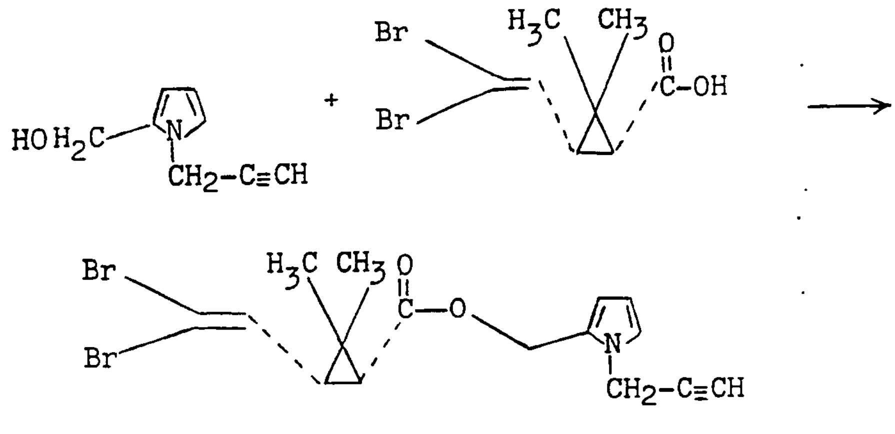

$\alpha_D = -12° \pm 1°$ (c = 1 % toluène)

Préparation 3

2-[1-(2-propynyl)-pyrrolyl]méthanol utilisé comme produit de départ de l'exemple 16 est préparé comme suit:

*STADE A:* 1,2-propynyl pyrrole

On mélange 10,32 g de propargylamine et 24,76 g de 2,5-diméthoxyhydrofuranne dans 38 $cm^3$ d'acide acétique. On amène le bain de chauffage à 110°C et agite pendant trois quart d'heure. On verse dans 250 $cm^3$ d'eau et ajoute 345 $cm^3$ de soude 2 N. On extrait à l'éther, sèche et concentre à sec. On obtient 15 g d'un produit que l'on chromatographie sur silice en éluant par le mélange hexane-acétate d'éthyle (8—2). On concentre à 30°C sous pression réduite. On obtient 10 g de produit recherché.

*STADE B:* 2-[1-(2-propynyl) pyrrolyl] carboxaldéhyde

On ajoute 16 g d'oxychlorure de phosphore dans 7,64 g de diméthyl formamide. On ajoute 50 $cm^3$ de chlorure de méthylène et agite à 0°C pendant une demi-heure. On ajoute à 0°C une solution renfermant 11 g de 1-(2-propynyl)pyrrole carboxaldéhyde dans 40 $cm^3$ de chlorure de méthylène. On porte le mélange réactionnel au reflux pendant une demi heure. On refroidit à 20°C et ajoute une solution aqueuse d'acétate de sodium (58,6 g dans 117 $cm^3$). On porte à nouveau au reflux pendant 1/4 d'heure sous bonne agitation. On extrait au chlorure de méthylène, l'ave à l'eau, sèche et concentre. On chromatographie le produit obtenu sur silice en éluant par le mélange hexane-acétate d'éthyle (6—4). On obtient ainsi 9,3 g de produit recherché.

*STADE C:* 2-[1-(2-propynyl) pyrrolyl] méthanol

On ajoute 0,7 g de borohydure de potassium dans un solution renfermant 1,75 g de produit préparé au stade B, 50 $cm^3$ de tétrahydrofuran et 10 $cm^3$ d'eau. On agite pendant 1 heure à 20°C, ajoute 0,35 g de borohydrure de potassium et poursuit l'agitation pendant 1/2 heure à 20°C. On sature de chlorure de sodium et extrait à l'acétate d'éthyle. On réunit les phases organiques et les sèche. On évapore le solvant à 40°c sous pression réduite. On isole ainsi 1,78 g de produit recherché.

En opérant comme à l'exemple 1, en suivant les schèmes indiqués ci-après, on a obtenu les produits suivants:

Exemple 18

1R,cis (ΔE) 2,2-diméthyl 3-(3-éthoxy 3-oxo 2-fluoro 1-propényl) cyclopropane carboxylate de [2-cyano-[(2-propynyl) 1 H-pyrrol-4-yl] méthyle.

Exemple 19

1R,cis 2,2-diméthyl 3-(2,2-dibromoéthyl) cyclopropane carboxylate de 2-cyano[1-(2-propynyl)-1 H pyrrol-4-yl] méthyle.

Exemple 20

1R,cis 2,2 diméthyl 3-(3-méthoxy-3-oxo-1-propényl)-2,2-diméthyl cyclopropane carboxylate de 2-cyano[1-(2-propynyl)-1 H-pyrrol-4-yl] méthyle.

Exemple 21

1R,cis (ΔE) 2,2-diméthyl 3-(3-terbutoxy-3-oxo-2-fluoro-1-propényl) cyclopropane carboxylate de 2-cyano[1-(2-propynyl)-1 H pyrrol-4-yl] méthyle.

Exemple 18 :

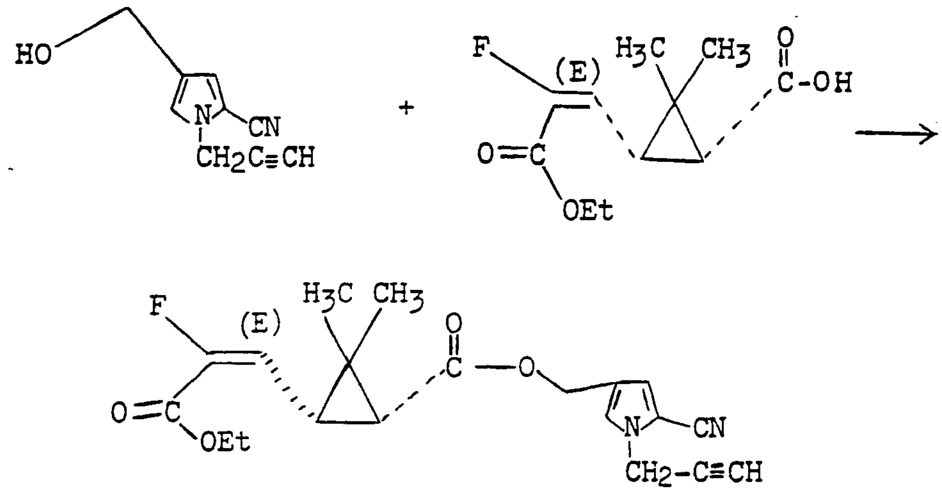

α D= 30° ± 2° (c=0,5 % toluène)

Exemple 19 :

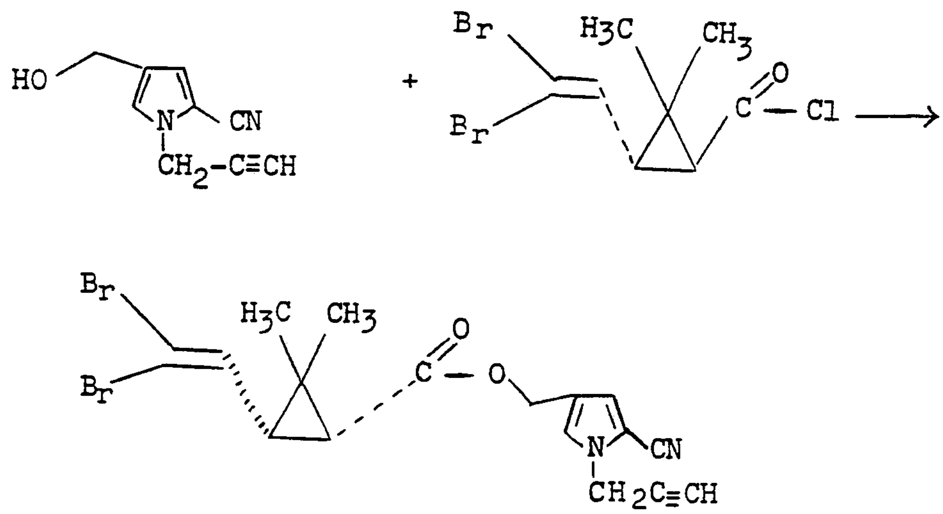

α D=-3° ± 2° (c=0,5% toluène)

Exemple 20 :

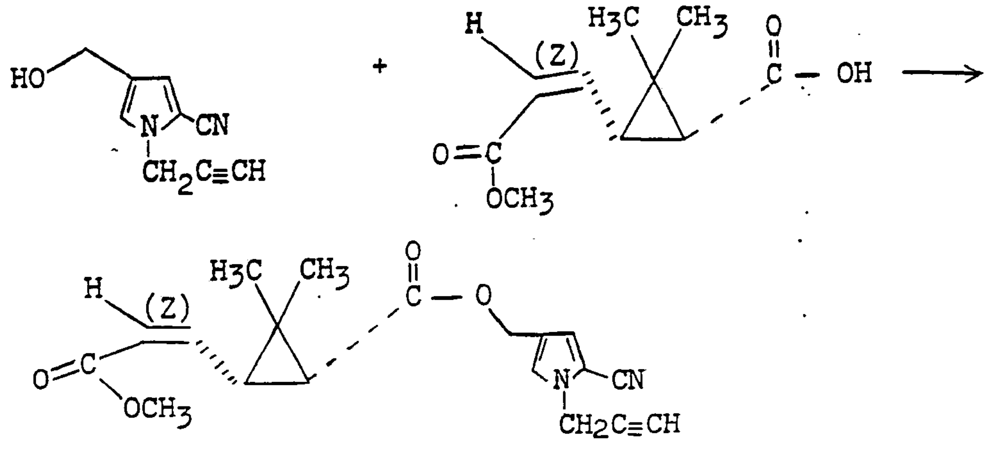

$\alpha_D$ = +55,5° ± 2° (c = 0,5% toluène)

Exemple 21 :

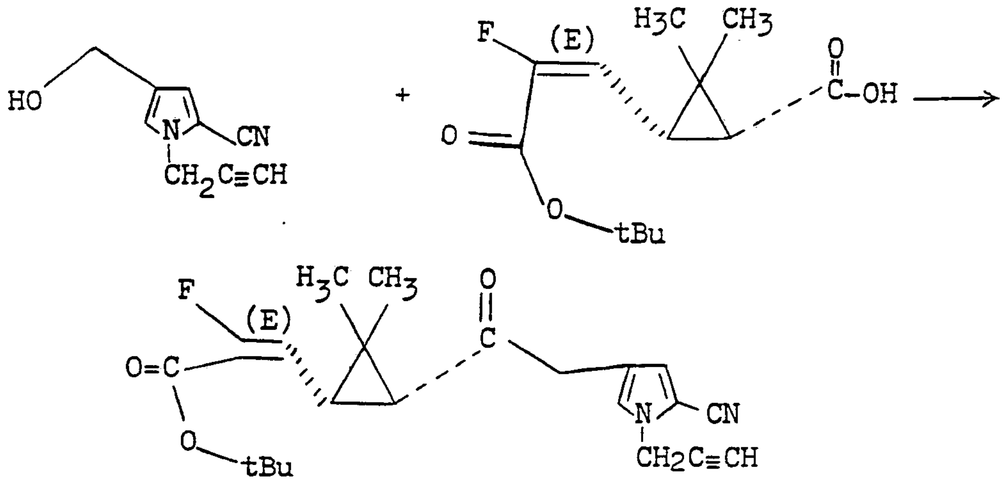

$\alpha_D$ = +35° ± 2° (c = 0,5% toluène)

Préparation 4

Le 4-hydroxyméthyle 1-(2 propynyl)-1 H-pyrrole 2-carbonitrile utilisé comme produit de départ, est préparé de la façon suivante:

*STADE A:* 1-(2-propynyl) 1 H-pyrrole 2-carbonitrile

On mélange 51,57 g de 2-cyanopropyl préparé selon procédé indiqué dans Can. J. Chem *59,* 2763 (1981) 146,9 g de triphénylphosphine, 43,95 g d'alcool propargylique, ajoute 42 $cm^3$ de tétrahydrofuranne, refroidit à +5°C et introduit en ½ heure à 0/5°C 97,5 g d'azodicarboxylate d'éthyle. On laisse revenir à la température ambiante et maintient sous agitation pendant 18 heures. On évapore le solvant sous pression réduite. On reprend dans l'éther éthylique le résidu cristallisé obtenu. On filtre. On concentre le filtrat. On obtient une huile que l'on chromatographie sur silice en éluant avec le mélange hexane-acétate d'étyle (7—3). On obtient 34 g de produit recherché.

*STADE B:* 4-formyl /(2-propynyl) 1 H-pyrrole 2-carbonitrile

On refroidit à −78°C un mélange renfermant 2,36 g de chlorure d'aluminium dans 6 $cm^3$, de chlorure de méthylène anhydre. On ajoute ensuite 1,3 g de produit préparé au stade A en solution dans 8 $cm^3$, de chlorure de méthylène et 0,5 $cm^3$ de nitrométhan. On ajoute ensuite à −55°C 1,49 g de dichlorométhyléther en solution dans 15 $cm^3$, de chlorure de méthyléne. On maintient la mélange réactionnel pendant 1 heure à −60°C et laisse revenir à 20°C. On maintient le mélange réactionnel à 20°C pendant 18 Heures. On verse dans l'eau, agite, neutralise à pH 7 à l'aide de 33 $cm^3$ de soude 2 N. On extrait au chlorure de méthylène. On lave avec une solution de bicarbonate de potassium 1 M, sèche et concentre à sec. On obtient 1,5 g de produit, qu'on lave avec dupentane et sèche. On obtient 1,1 g de produit recherché fondant à 97°C.

*STADE C:* 4-(hydroxyméthyl)-1-(2-propynyl)-1-H pyrrol 2-carbonitrile

On ajoute 372 mg de borohydrure de potassium dans une solution renfermant 546 mg de produite préparé au stade B 40 $cm^3$ de tétrahydrofuranne et 5,5 $cm^3$ d'eau. On maintient sous agitation à la température ambiante pendant un quart d'heure. On ajoute 50 $cm^3$ d'une solution aqueuse saturée de chlorure de sodium. On extrait à l'acétate d'éthyle, sèche et concentre à sec. On obtient 600 mg de produit recherché.

Exemple 22

1 R,cis (2,2-diméthyl 3-(2,2-dibromoéthényl) cyclopropane carboxylate de (R,S)-cyano /2-cyano 1-(2-propynyl) 1 H-pyrrol-4-yl/ méthyl.

On verse 447 mg de 4-formyl 1(2-propynyl) 1 H-pyrrol 2-carbonitrile dans 13 $cm^3$ de toluène. On ajoute ensuite 173 mg de cyanure de sodium, 38 mg de bromure de tétrabutylammonium, 894 mg de chlore de l'acide 1 R,cis 2,2-diméthyl 3-(2,2-dibromoéthényl)- cyclopropane-carboxylique. On ajoute 0,13 $cm^3$ d'eau. On agite à 20°C pendant 18 heures. On ajoute 100 $cm^3$ de benzène, sèche et concentre à sec. On obtient 1017 mg de produit recherché.

$\alpha_D$ = +9° ± 2° (c = 0,3% toluéne)

En opérant comme à l'exemple 1, en suivant les schémas indiqués ci-après, on a obtenu les produits suivants:

Exemple 23

1 R,cis (△Z) 2,2-diméthyl 3-(3-méthoxy 3-oxo 1-propényl) cyclopropane carboxylate de cyano /2-cyano 1-(2-propynyl) 1 H-pyrrol-4-yl/ méthyle.

Exemple 24

1 R,cis (△E) 2,2-diméthyl 3-(3-éthoxy 3-oxo 2-fluoro 1-propényl) cyclopropane carboxylate de cyano /2-cyano 1-(2-propynyl) 1 H-pyrrol-4-yl/ méthyle.

Exemple 25

1 R,cis (△E) 2,2-diméthyl 3-(3-terbutoxy 3-oxo 2-fluoro 1-propényl) cyclopropane carboxylate de cyano /2-cyano 1-(2 propynyl) 1 H-pyrrol-4-yl/ méthyle.

Exemple 23 :

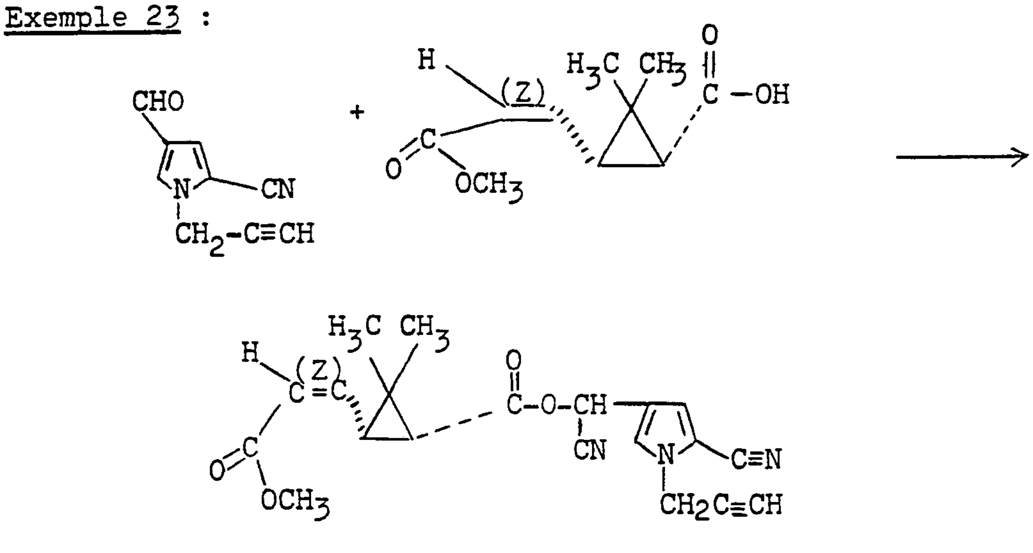

$\alpha_D$ = +53,5° ±2° (c=0,5% toluène)

Exemple 24 :

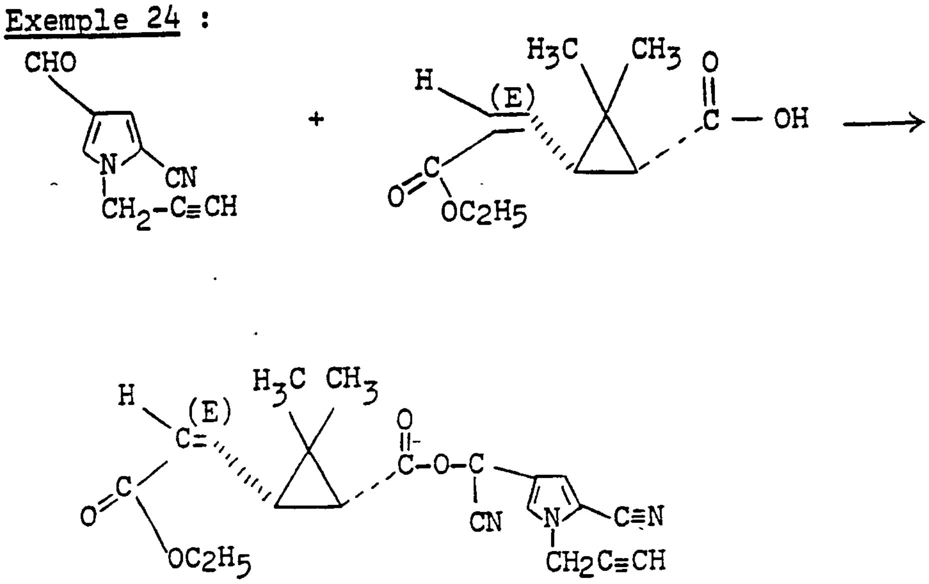

α D= +40° ± 1° (c=1% toluène)

Exemple 25 :

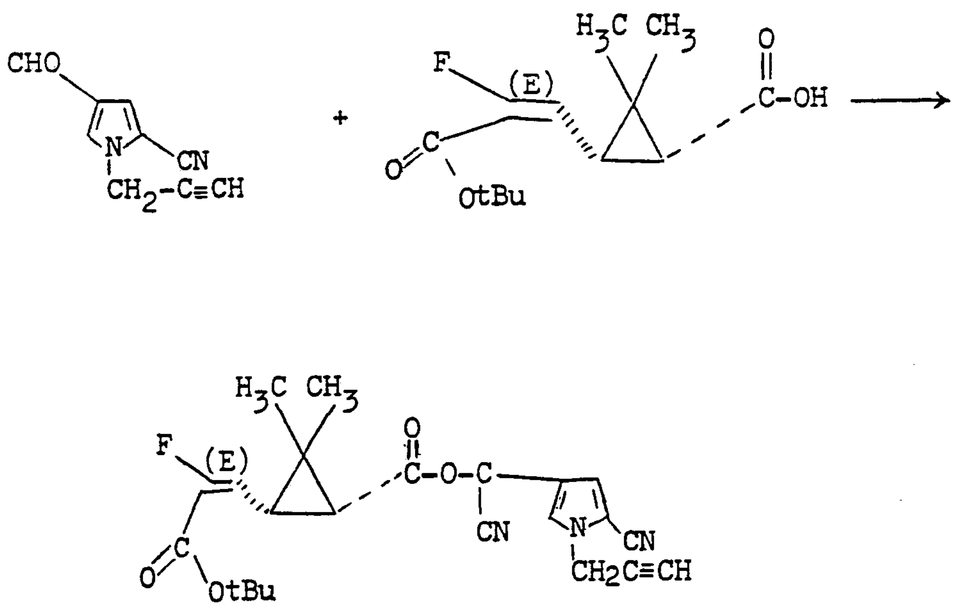

α D= +39° ±1° (c=1% toluène)

En opérant comme à l'exemple 1, en suivant les schémas indiqués ci-après, on a obtenu le produit suivant:

Exemple 26

1R,cis 2,2-diméthyl 3-(2,2-dibromo éthényl) cyclopropane carboxylate de /1-(2-propynyl) 2-cyano-1H-pyrrol-3-yl/ méthyle.

Exemple 26 :

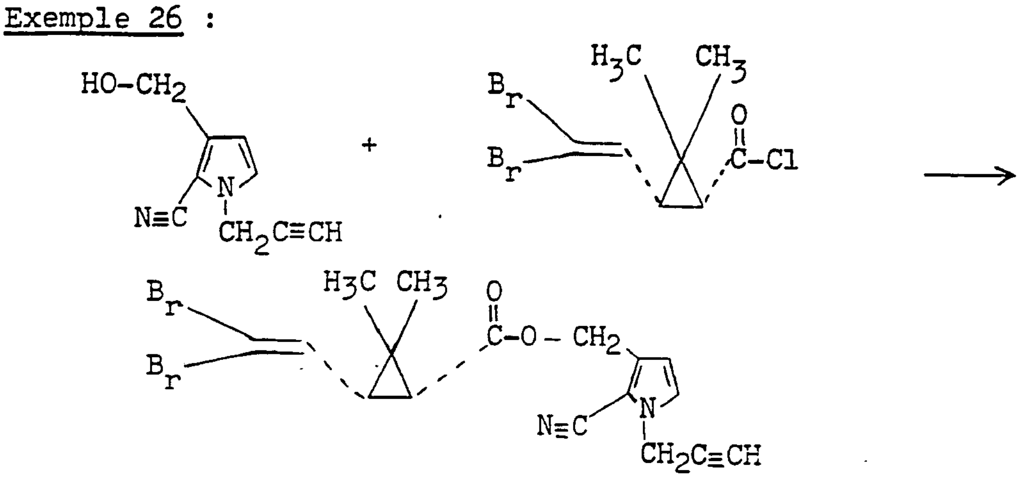

$\alpha_D = -5{,}5° \pm 1°$ (c = 1 % toluène)

Préparation 5

Le 3-(hydroxyméthyl) 1-(2-propynyl) 1 H-pyrrole 2-carbonitrile utilisé comme produit de départ des exemples 25 à 28 est préparé de la façon suivante:

*STADE A:* 2-formyl-1-(2-propynyl) 1 H-pyrrole 3-carboxylate de méthyle

On refroidit à 20°C une solution de 7,05 g de 1,1-diméthoxy 2-oxo butanoate de méthyle et 4,4 g de propargylamine dans 20 $cm^3$ d'eau. On agite 10 minutes, ajoute goutte à goutte 9,9 g d'acétate de 1,2 dibromoéthyle. On maintient sous agitation à 20°C pendant 24 heures. On verse dans l'eau. On extrait à l'acétate d'éthyle, lave avec une solution de bicarbonate de soude, sèche et concentre à sec. On obtient 10 g d'un produit que l'on chromatographie sur silice en éluant à l'aide du mélange cyclohexane-acétate d'éthyle (75—25). On obtient 1,9 g de produit recherché fondant à 76°C.

*STADE B:* 1-(2 propynyl) 2- oximino méthyl 3-pyrrole carboxylate de méthyle

On maintient sous agitation pendant 3 heures à la température ambiante 2 g du produit préparé au stade A, 725 mg de chlorhydrate d'hydroxylamine et 11 $cm^3$ de pyridine anhydre. On concente la pyridine sous pression réduite. On reprend à l'acétate d'éthyle et lave à l'eau. On sèche et concentre. On obtient 2,3 g de produit recherché fondant à 88—90°C.

*STADE C:* 2-cyano 1-(2-propynyl) 1 H pyrrole 3-carboxylate de méthyle

On refroidit à 20°C une solution obtenue en mélangeant 3,54 g de chlorure de thionyle en solution dans 2 $cm^3$ de chlorure de méthylène, 2,2 g de produit obtenu au stade précédent dans 15 $cm^3$ de chlorure de méthylène. On maintient le mélange réactionnel sous agitations à la température ambiante pendant 24 heures, concentre et obtient 1,8 g de produit que l'on chromatographie sur silice en éluant avec le mélange cyclohexane-acétate d'éthyle (7—3). On obtient 1,430 g de produit recherché fondant à 112°C.

*STADE D:* 3 -(hydroxyméthyl) 1-(2-propynyl) 1 H-pyrrole 2-carbonitrile

On agite pendant 24 heures à 20°C un mélange renfermant 830 mg de produit préparé au stade précédent, 8 $cm^3$ de tétrahydrofuranne et 2 $cm^3$ d'eau. On ajoute ensuite 280 mg de borohydrure de lithium. On ajoute 30 $cm^3$ d'acétate d'éthyle, sèche et concentre à sec. On obtient 750 mg de produit que l'on chromatographie sur silice en éluant avec le mélange cyclohexane acétate d'éthyle (6—4). On obtient 138 mg de produit recherché fondant à 65°C.

En opérant comme à l'exemple 1, en utilisant les schémas indiqués ci-après, on obtient les produits suivants:

Exemple 27

1R,cis (△E)-2,2 diméthyl 3-(3-éthoxy 3-oxo 2-fluoro 1-propényl) cyclopropane carboxylate de /1-(2-propynyl) 1 H-pyrrol 3-yl/ méthyle.

Exemple 28

1R,cis 2,2-diméthyl 3-(2,2 dibromoéthényl) cyclopropane carboxylate de /1-(2 propynyl) 2-nitro-1 H pyrrol-4 yl/ méthyle.

Exemple 29

1R,cis (△Z)2,2-diméthyl 3-(3-méthoxy 3-oxo 1-propényl) cyclopropane carboxylate de /2-nitro 1-(2 propynyl) 1 H-pyrrol 4-yl/ méthyle.

Exemple 30

1R,cis (△E)2,2-diméthyl 3-(3-terbutoxy 3-oxo 2-fluoro 1-propényl) cyclopropane carboxylate de /2-nitro 1-(2 propynyl) 1 H-pyrrol-4-yl/ méthyle.

Exemple 27 :

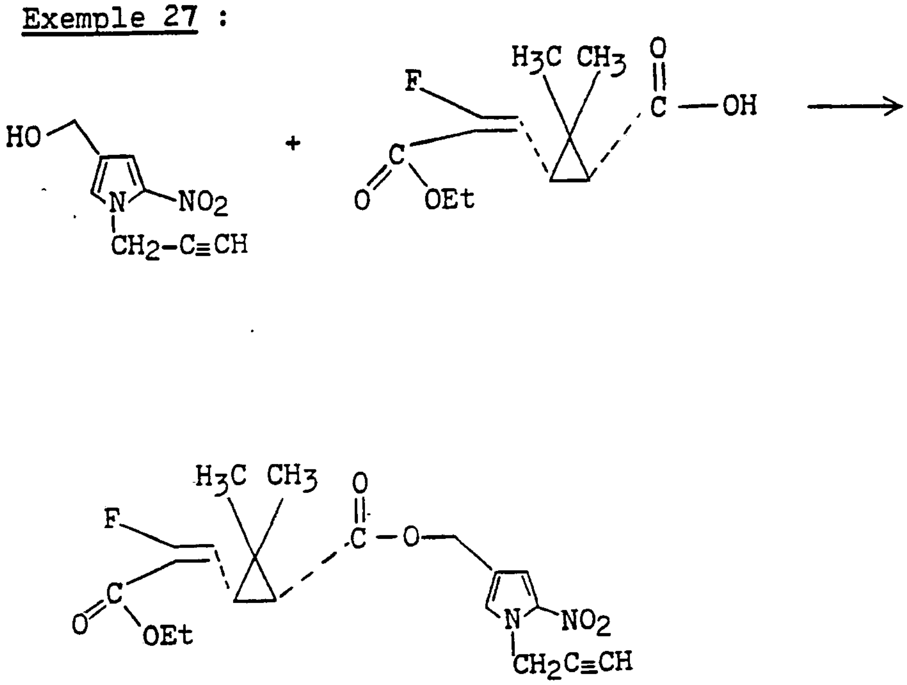

$\alpha_D$ = +30° ±2° (c=0,5 % toluène)

Exemple 28 :

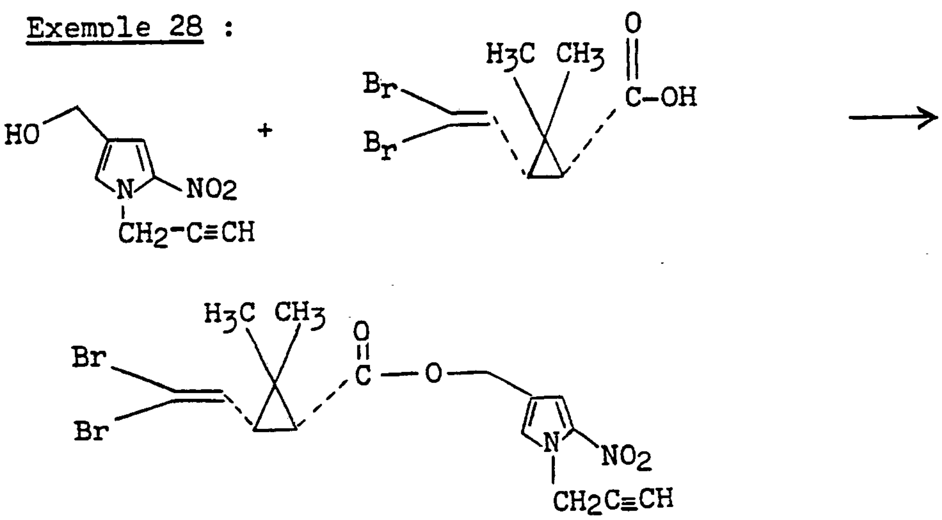

$\alpha_D$ =-1,5° ±1° (c=1% toluène)

Exemple 29 :

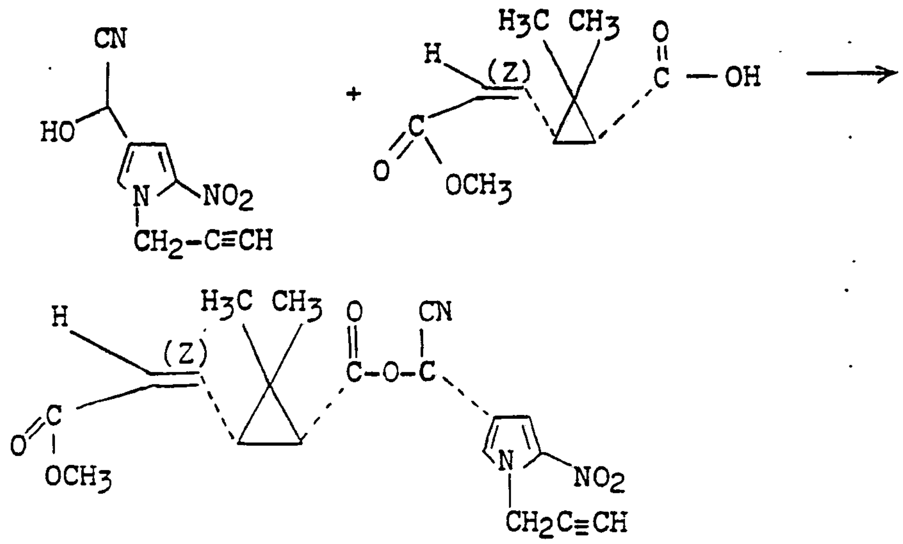

$\alpha_D$ = +57° ±2° (c=0,5 % toluène)

Exemple 30 :

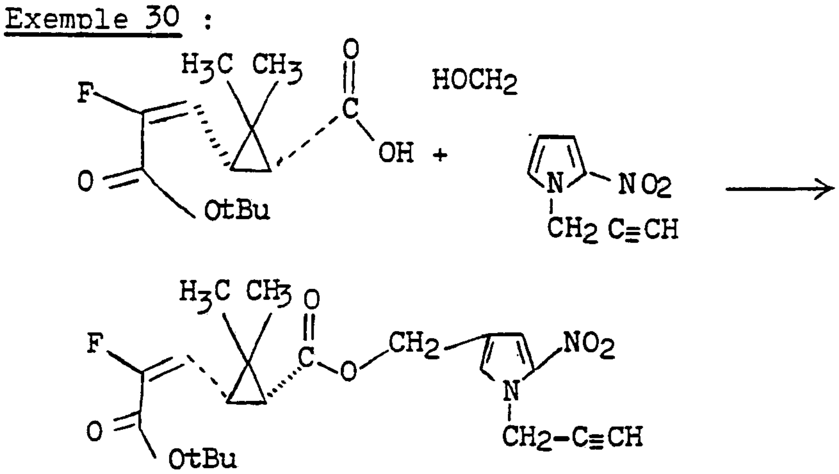

$\alpha_D$ = 42° ±2° (c= 0,5 % toluène)

Préparation 6

Le 2 nitro 1(2 propynyl) 1 H pyrrole 4-méthanol utilisé comme produit de départ des exemples 29 à 32 est préparé de la façon suivante:

*STADE A:* 2-nitro 1-(2-propynyl) 1H-pyrrol 4-carboxaldéhyde

On ajoute à +5°C 310 mg d'hydrure de sodium à 50% dans l'huile dans une solution renfermant 0,9 g de 2-nitro 1 H-pyrrol 4-carboxaldéhyde (prépare selon procédé décrit dans Bull Soc Chim Fran p 283 à 291/1972) et 15 $cm^3$ de tétrahydrofuranne. On laisse revenir à la température ambiante et ajoute 0,53 $cm^3$ de bromure de propargyle. On agite le mélange réactionnel sous agitation pendant 6 heures à 60°C, puis pendant 18 heures à 20°C. On filtre et concentre à sec. On obtient 1 g d'un produit que l'on chromatographie sur silice en éluant par le mélange heptane-acétate d'éthyl (1—1). On obtient ainsi 450 mg de produit recherché fondant à 128°C.

*STADE B:* 2-nitro 1-(2 propynyl) 1 H pyrrol 4-méthanol

On ajoute 270 mg de borohydrure de potassium dans une solution renfermant 450 mg du produit préparé au stade A, 30 $cm^3$ de tétrahydrofuranne et 4 $cm^3$ d'eau. On maintient sous agitation pendant 15 minutes, verse sur une solution saturée de chlorure de sodium, extrait à l'acétate d'éthyle, sèche et concentre. On obtient 455 mg deproduit recherché.

En opérant comme à l'exemple 1, en suivant les schémas indiqués ci-après, on a obtenu les produits suivants:

Exemple 31

1R,cis 2,2-diméthyl 3-(2,2-dibromo éthényl) cyclopropane carboxylate de (R,S) cyano /2-nitro 1-(2-propynyl) 1-H-pyrrol 4-yl/ méthyle.

Exemple 32

1R,cis (△E) 2,2-diméthyl 3-(3-éthoxy-3-oxo-2-fluoro 1-propényl) cyclopropane carboxylate de (R,S) cyano /2 nitro 1-(2-propynyl) 1-H-pyrrol 4-yl/ méthyle.

Exemple 31 :

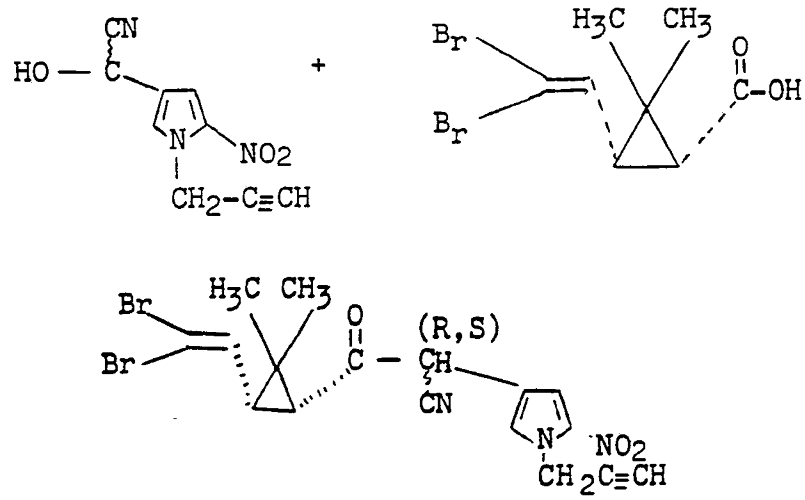

α D = +26,5° ±2° (c= 0,5 % toluène)

Exemple 32 :

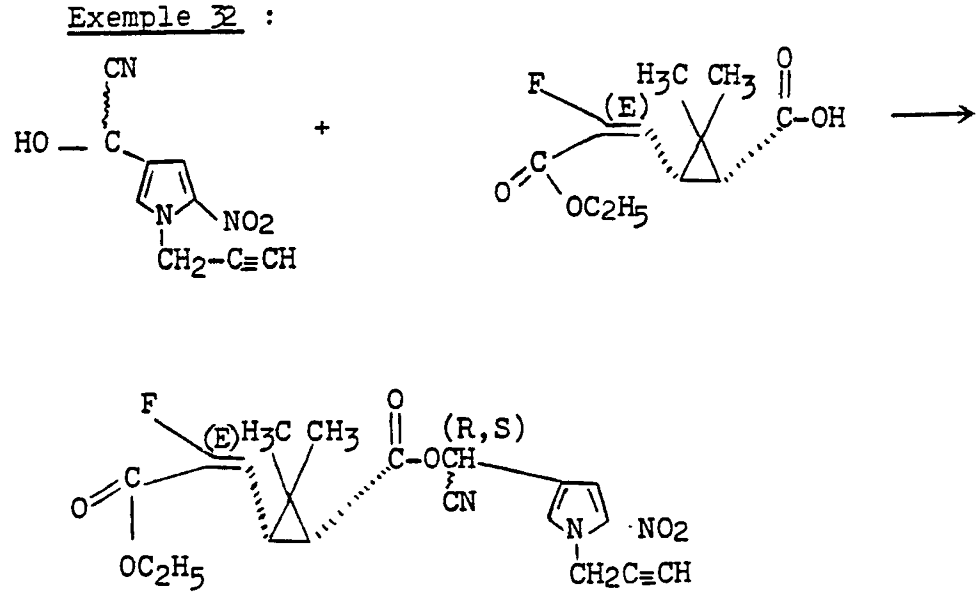

$\alpha_D$ = +42° ±5° (c= 0,2% toluène)

Préparation 7

l' α-hydroxy-1-(2-propynyl)-1 H pyrrole-5-nitro 3-acétonitrile utilisé comme produit de départ des exemples précédentes est préparé comme suit:

On mélange 801 mg de 2-nitro 1(2-propynyl) 1 H pyrrol 4-carboxaldéhyde, 15 $cm^3$ de méthanol et 3 $cm^3$ d'eau. On ajoute 3,6 $cm^3$ d'acide acétique et refroidit à +5°C. On ajoute 2,2 g de cyanure de sodium et laisse la température revenir à 20°C pendant 4 heures. On verse le mélange réactionnel dans l'eau glacée, extrait à l'éther éthylique et lave la phase éthérée à l'eau. On séche et concentre. On obtient 920 mg de produit recherché.

En opérant comme à l'exemple 1, en suivant le schéma indiqué ci-après, on a obtenu le produit suivant:

Exemple 33

1R,cis 2,2-diméthyl 3-(2,2-dibromo éthényl) cyclopropane carboxylate de /3 nitro 1(2-propynyl) 1 H-pyrrol-4 yl/ méthyle.

Exemple 33 :

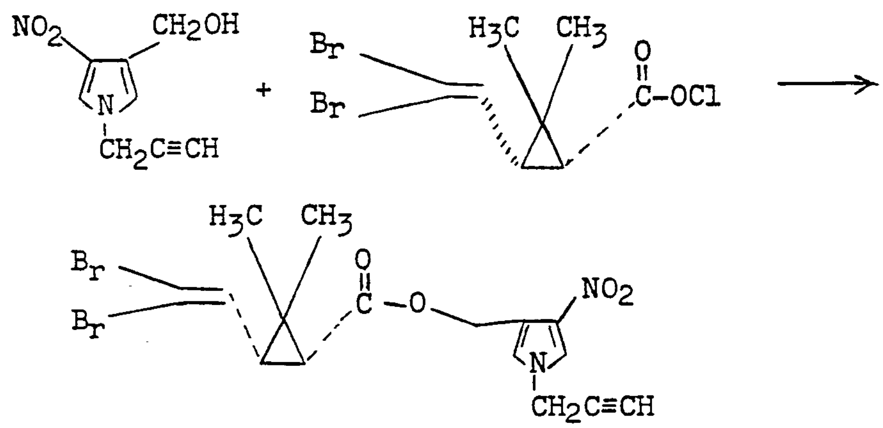

$\alpha_D$ = -26,5° ±2° (c = 0,5 % toluène)

Préparation 8

le 3-nitro 2(2-propynyl)-1 H pyrrole 4-méthanol utilisé comme produit de départ est préparé comme suit:

*STADE A:* 3-nitro 1(2-propynyl)-1 H pyrrole 4-carboxaldéhyde.

On verse 826 mg de 3 nitro 1 H pyrrole 4-carboxaldéhyde préparé selon le procédé dans Bull Soc Chim p 283 à 291 (1972) dans 40 $cm^3$ de tétrahydrofuranne. On ajoute ensuite à +5°C 280 mg d'hydrure de sodium à 50% dans l'huile. On laisse la température à 20°C pendant une heure. On ajoute alors 1,19 g de bromure de propargyle. On agite pendant 18 heures à 20°C. On filtre et concentre. On concentre. On chromatographie sur silice en éluant par le mélange hexane-acétate d'éthyle (1—1). On isole alors 800 mg de produit fondant à 120°C.

*STADE B:* 3-nitro 1(2-propynyl) 1 H-pyrrole 4-méthanol.

On introduit à 20°C, 242 mg de borohydrure de potassium dans une solution renfermant 400 mg de produit préparé au stade A, 30 $cm^3$ de tétrahydrofuranne et 4 $cm^3$ d'eau. On agit 15 minutes à température ambiante, ajoute 10 $cm^3$ d'une solution saturée de chlorure de sodium, et agite 5 minutes à 20°C. On extrait à l'acétate d'éthyle, sèche et concentre. On obtient 410 mg du produit recherché fondant à 60°C.

En opérant comme à l'exemple 1, en suivant le schéma indiqué ci-après, on a obtenu le produit suivant:

Exemple 34

1R,cis 2,2-diméthyl 3-(2,2-dibromo éthényl) cyclopropane carboxylate de (R,S)-cyano /3-nitro 1-(2-propynyl) 1 H-pyrrol 4-yl/ méthyle.

Exemple 34 :

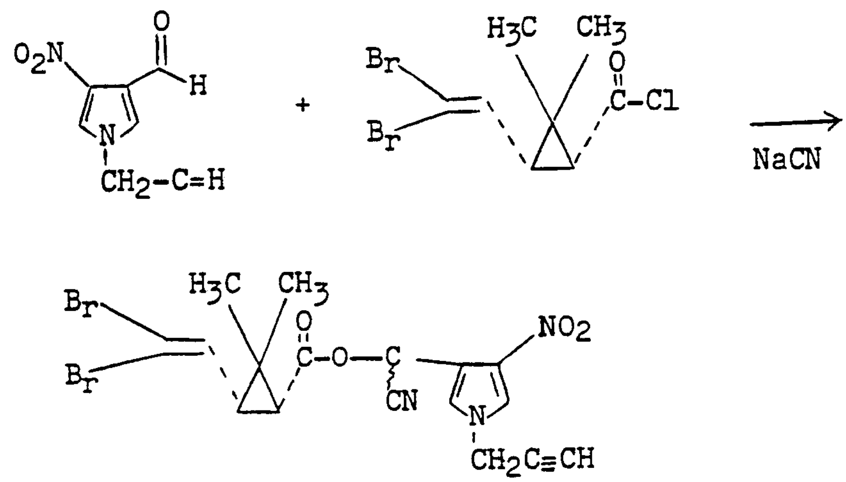


$\alpha_D$ = -28,5° ± 1°(c = 1,3 % toluène)

En opérant comme à l'exemple 1, en suivant le schémas indiqué ci-après, on a obtenu le produit suivants:

Exemple 35

1R,cis (△E) 2,2-diméthyl 3-(3-éthoxy 3-oxo 2-fluoro 1-propényl) cyclopropane carboxylate de /(1-propadien) 1 H pyrrol-3-yl/ méthyle.

Exemple 36

1R,cis 2,2 diméthyl 3,3-dibromo éthényl cyclopropane carboxylate de /(1-propadien) 1 H-pyrrole 3-yl) méthyle.

Exemple 35 :

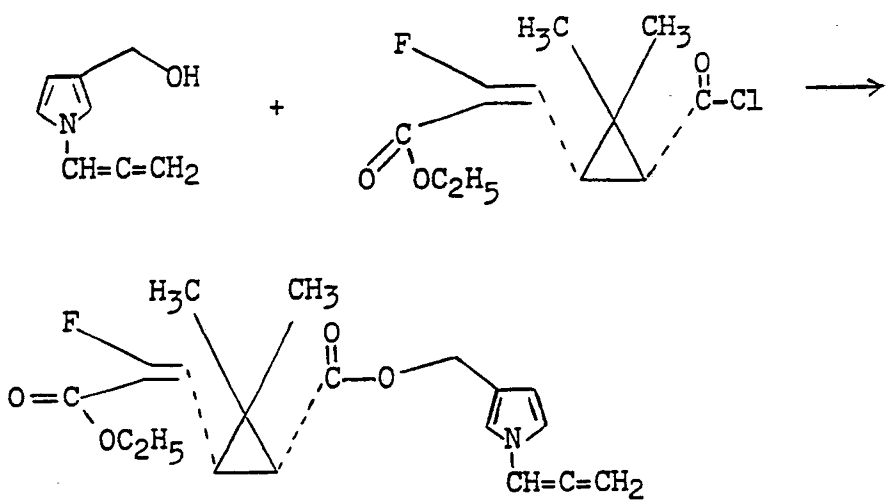

$\alpha_D$ = +17° ±1° (c = 0,8 % toluène)

Exemple 36 :

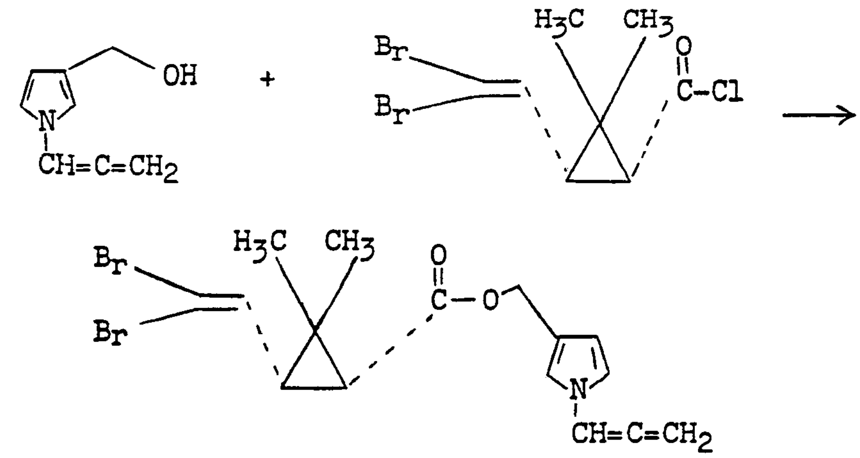

$\alpha_D$ = −13,5° ±1° (c = 1 % toluène)

Préparation 9

le 3-(1-(propadiène) pyrrolyl) méthanol utilisé comme produit de départ est préparé comme suit:

*STADE A:* 1-propadiényl 1 H-pyrrole 3-carboxaldéhyde

On dissout 1 g de pyrrole-3-carboxaldéhyde dans 25 $cm^3$ de tétrahydrofuranne. On agite à la température ambiante pendant 15 Minutes. On refroidit à 0°/5°C et ajoute 528 mg d'hydrure de sodium à 50% dans l'huile. On agite 10 minutes à froid, puis laisse revenir à la température ambiante et agite pendant 40 minutes sous azote. On ajoute 1 $cm^3$ de bromure de propargyle. On agite pendant 2 heures à 40°/50°C. On verse la mélange réactionnel dans 15 $cm^3$ d'eau. On extrait au chlorure de méthylène. On sèche et concentre par distillation du solvant. On chromatographie sur silice en éluant par le mélange hexane-acétate d'éthyle (7—3). On obtient 1,166 g de produit recherché.

*STADE B:* 3-(1-(propadiène) pyrrolyl) méthanol

On dissout 1,009 g de produit préparé au stade A dans une solution renfermant 35 $cm^3$ de tétrahydrofuranne et 6,7 $cm^3$ d'eau. On agite pendant 15 minutes à 20°C. On ajoute 523 mg de borohydrure de potassium. On agite à 20°C pendant 2 heures 15 minutes. On verse sur une solution saturée de chlorure de sodium dans l'eau. On extrait au chlorure de méthylène, sèche et concentre. On obtient 0,899 g de produit que l'on utilise tel quel.

En opérant comme à l'exemple 1, en opérant selon le schéma indiqué ci-après, on a obtenu le produit suivants:

Exemple 37

IR,cis 2,2-diméthyl 3-(2,2-dibromo éthényl)-cyclopropane carboxylate de /1-(2-propynyl) 2-trifluorométhyl 1 H-pyrrol 3-yl/ méthyle.

Exemple 38

1R,cis (ΔE) 2,2-diméthyl 3-/3 éthoxy 3-oxo 2-fluoro 1-propényl/ cyclopropane carboxylate de /1-(2-propynyl) 2-trifluorométhyl 1 H-pyrrol- 3-yl/ méthyle.

Exemple 37 :

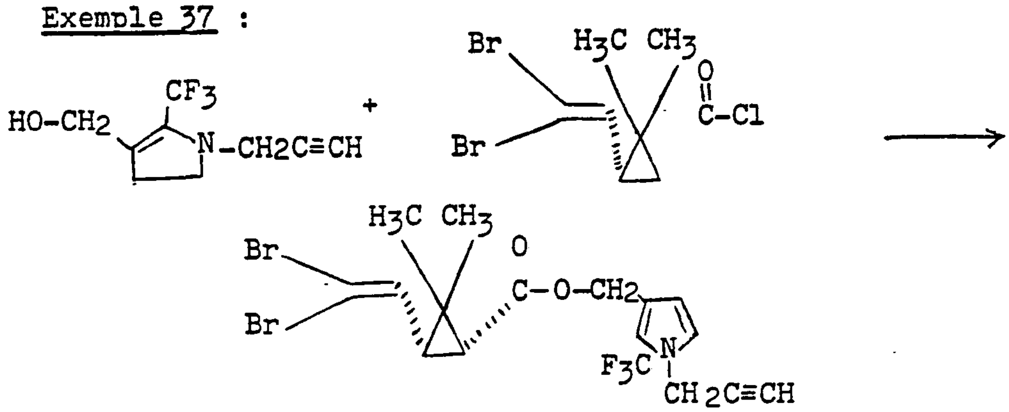

α D = −12° ±2° (c= 0,5 % toluène)

Exemple 38 :

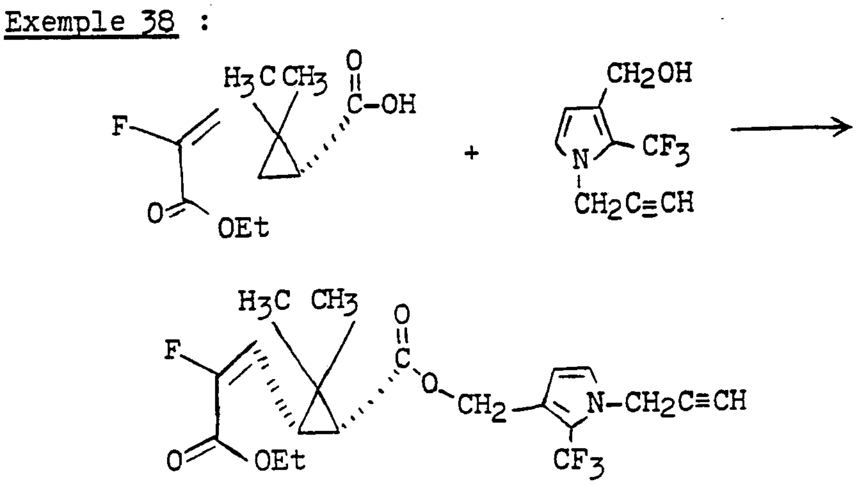

$\alpha D = +1 \pm 1°$ (c= 0,5 % de toluène)

Préparation 10

1-(2-propynyl) 2-trifluorométhyl 3-pyrrol méthanol

*STADE A:* 1-(2-propynyl) 2-trifluoro méthyl 3-pyrrol carboxylate d'éthyle.

On introduit à 10°C 1,8 $cm^3$ de 3-oxo 4,4,4 trifluoro butanoate d'éthyle dans 3,15 $cm^3$ de 2 propynylamine en solution dans 10 $cm^3$ d'eau. On maintient sous agitation pendant 10 minutes et ajoute 1,65 $cm^3$ d'acétate de 1,2 dibromo éthyle.

On porte à 70°C pendant 45 minutes. On extrait à l'acétate d'éthyle. On sèche et concentre. On obtient 6 g d'huile que l'on chromatographie sur silice, on obtient 360 mg de produit recherché.

*STADE B:* 1-(2-propynyl) 2-trifluorométhyl 3-pyrrole-méthanol.

On mélange à 5°C une solution renfermant 1,74 g de produit préparé au stade A, 10 $cm^3$ de tétrahydrofuranne et 270 mg d'hydrure d'aluminium et de lithium. On maintient sous agitation à la température ambiante pendant 3 heures. On refroidit, ajoute 10 $cm^3$ d'acétate d'éthyle et quelques gouttes d'une solution saturée detartrate double de potassium et de sodium. On essore, concentre à froid et obtient 2,4 g d'une huile que l'on chromatographie sur silice en éluant avec le mélange cyclohexane-acétate d'éthyle (8—2). On obtient 40 mg de produit recherché fondant à 63°C.

En opérant comme précédemment, on a pu préparé les produit suivants:

Exemple 39 :

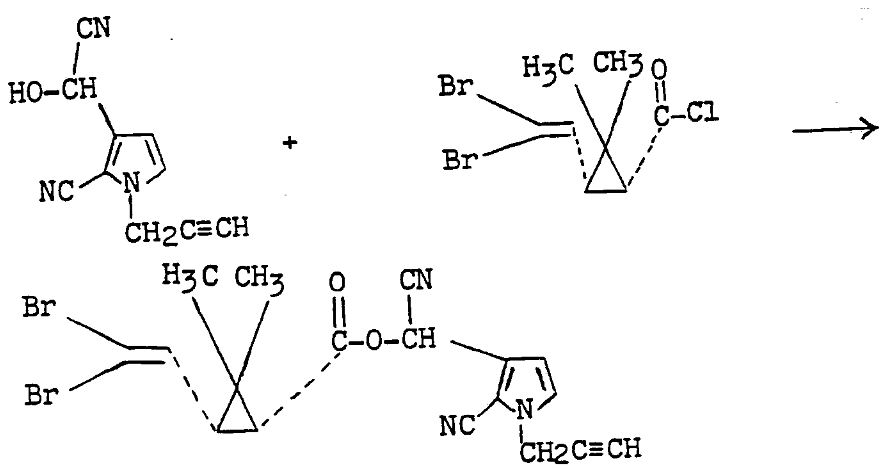

Exemple 40

1R, cis (△E) 2,2-diméthyl 3 (3-terbutyloxy 3-oxo 2-fluoropropényl) cyclopropane carboxylate de /2-trifluorométhyl 1(2-propynyl) (1H) pyrrol-3-yl/ méthyle.

On ajoute, goutte à goutte, une solution renfermant 455 mg de dicyclohexylcarbodiimide, 3 mg de diméthylaminopyridine et 5 $cm^3$ de chlorure de méthylène anhydre dans un mélange refroidi au bain de glace renfermant 450 mg de 1-(2-propynyl) 2-trifluorométhyl 3-pyrrol-méthanol, 570 mg d'acide 1R, cis (△E) 2,2-diméthyl 3-(3-terbutoxy 3-oxo 2 fluoro 1-propényl) cyclopropane carboxylique et 4 $cm^3$ de

chlorure de méthylène anhydre. On laisse le mélange réactionnel revenir à la température ambiante et l'agite pendant 5 heures. On élimine le précipité formé par filtration. On concentre le filtrat que l'on reprend à l'éther isopropylique. On filtre l'insoluble. On concentre le filtrat et chromatographie le résidu sur silice en éluant avec le mélange hexane-acétate d'éthyle 9—1. On obtient ainsi 518 mg du produit recherché.

Exemple 41

1R, cis 2,2-diméthyl 3-éthényl cyclopropane carboxylate de /2-trifluorométhyl 1-(2-propynyl) (1H) pyrrol 3-yl/ méthyle.

En opérant comme à l'exemple 1 à partir de 610 mg d'acide 1R, cis 2,2-diméthyl 3-éthényl cyclopropane carboxylique et de 800 mg de 1-(2-propynyl) 2-trifluorométhyl 3-pyrrol méthanol, on obtient 1,28 g du produit recherché.

$[\alpha]_D = +9°$ (0,2% toluène).

Exemple 42

1R,cis (△Z) 2,2-diméthyl 3-/3-méthoxy (3-oxo 1-propényl)/ cyclopropane carboxylate de /2-trifluorométhyl 1-(2-propynyl) 1H-pyrrol-3-yl/ méthyl.

En opérant comme à l'exemple 1, à partir de 860 mg d'acide 1R,cis (△Z) 2,2-diméthyl 3-(3-méthoxy 3-oxo) 1-propényl cyclopropane carboxylique et de 800 mg de 1-(2-propynyl) 2-trifluorométhyl 3-pyrrole méthanol, on obtient 1,18 g de produit recherche.

$[\alpha]_D = +17,5°$ (0,7% toluène).

Exemple 43

1R,cis (Z) 2,2-diméthyl 3-/(2-chloro 3,3,3-trifluoro) 1-propényl/ cyclopropane carboxylate de /2-cyano 1-(2-propynyl-1H-pyrrol-3-yl/ méthyle.

Dans 12 $cm^3$ de chlorure de méthylène, on introduit 430 mg de /2-cyano 1-(2-propynyl-1H-pyrrol/ 3-méthanol, 600 mg d'acide 1R,cis (Z) 2,2-diméthyl 3-/(2-chloro 3,3,3-trifluoro) 1-propényl/ cyclopropane carboxylique, ajoute à la solution obtenue, à 0°C, goutte à goutte une solution de 509 mg de dicyclohexylcarbodiimide et de 3 mg de diméthyl amino pyridine dans 2 $cm^3$ de chlorure de méthylène, agite pendant 5 heures à 20°C, filtre, concentre le filtrat à sec, ajoute de l'éther isopropylique, élimine par filtration l'insoluble résiduel, concentre le filtrat à sec, ajoute un mélange d'hexane et d'éther isopropylique (1—1), filtre, concentre le filtrat à sec, chromatographie le résidu sur silice en éluant avec un mélange d'hexane et d'éther isopropylique (1—1), ajoute au produit obtenu contenant encore un peu d'urée, 2 $cm^3$ d'éther isopropylique, élimine par filtration l'insoluble formé, concentre à sec par distillation sous pression réduite et obtient 896 mg de produit recherché.

$[\alpha]_D = +7°$ (c = 0,4% toluène).

*Analyse:* $C_{18}H_{16}ClF_3N_2O_2$: 884,789.

| | | | | | |
|---|---|---|---|---|---|
| Calculé: | C% 56,19 | H% 4,19 | Cl% 9,21 | F% 14,8 | N% 7,28 |
| Trouvé: | 56,3 | 4,4 | 9,0 | 14,9 | 7,2. |

Exemple 44

IR,cis (E) 2,2-diméthyl 3-(3-éthoxy 2-fluoro 3-oxo 1-propényl) cyclopropane carboxylate de cyano /1-(2-propynyl) 1H-pyrrol-3-yl/ méthyle.

Dans 30 $cm^3$ de chlorure de méthylène, on dissout 2 g de R,S α-cyano /2-cyano 1-(2-propynyl) 1H-pyrrol-3-yl/ 4-méthanol, 2,48 g d'acide 1R,cis (E) 2,2-diméthyl 3-(3-éthoxy 2-fluoro 3-oxo 1-propényl) cyclopropane carboxylique, ajoute á +10°C, en 30 minutes environ, une solution de 2,22 g de dicyclohexylcarbodiimide et de 13 mg de 4-diméthyl amino pyridine dans 30 $cm^3$ de chlorure de méthylène, agite pendant 18 heures à 20°C, filtre, concentre à sec, ajoute de l'éther éthylique, filtre, concentre à sec, chromatographie le résidu sur silice en éluant par un mélange d'hexane et d'acétate d'éthyle (7—3) et obtient ainsi 3,14 g d'acide recherché.

$[\alpha]_D = +40°$ (c = 1%, toluène).

*Analyse:* $C_{21}H_{20}FN_3O_4$: 397,409.

| | | | | |
|---|---|---|---|---|
| Calculé: | C% 63,47 | H% 5,07 | F% 4,78 | N% 10,57 |
| Trouvé: | 63,4 | 5,1 | 4,8 | 10,3 |

Préparation 11

Le 4-hydroxy 1-(2-propynyl) 1H-pyrrol 5-cyano 3-acétonitrile utilisé au départ de l'exemple 44 peut être préparé comme suit.

On mélange 6 g de 1-(2-propynyl) 1H-pyrrol 2-carbonitrile, 125 $cm^3$ de méthanol et 25 $cm^3$ d'eau, refroidit à +5°C et ajoute 15,2 $cm^3$ d'acide acétique puis en plusieurs fois 9,3 g de cyanure de sodium. On laisse remonter la température à 20°C pendant 4 heures, verse sur un litre d'eau et extrait par de l'éther éthylique. On sèche les phases organiques, concentre à sec et obtient 7 g de produit attendu. F = 60°C.

Exemple 45

1R,cis (E) 2,2-diméthyl 3-/2-fluoro 3-oxo 3-tertbutoxy propynyl/ cyclopropane carboxylate de /1-(2-propynyl) 3-trifluorométhyl) 1H-pyrrol-4-yl/ méthyle.

Dans 25 $cm^3$ de chlorure de méthylène, on dissout 1 g de 1-(2-propynyl) 3-trifluorométhyl 1H-pyrrol-4-méthanol, 1,5 g d'acide 1R,cis (E) 2,2-diméthyl 3-/2-fluoro 3-oxo 3-tertbutoxy propényl/ cyclopropane

carboxylique, introduit à 0°C progressivement un mélange de 1,3 g de dicyclohexylcarbodiimide, 150 mg de 4-diméthyl amino pyridine dans du chlorure de méthylène, agite pendant 15 minutes à 0°C, puis pendant 2 heures à 20°C, filtre, concentre le filtrat à sec, ajoute de l'éther isopropylique, amène à 0°C, filtre, concentre à sec par distillation sous pression réduite, chromatographie à deux reprises le résidu sur silice en éluant par un mélange d'hexane et d'acétate d'éthyle (85—15) et obtient 1,7 g de produit recherché.

$[\alpha]_D$ = 40,5° (c = 0,7%, chloroforme).

*Analyse:* $C_{22}H_{25}F_4NO_4$: 443,443.

| | | | | |
|---|---|---|---|---|
| Calcule: | C% 59,6 | H% 5,68 | F% 17,14 | N% 3,16 |
| Trouvé: | 59,2 | 5,7 | 17,1 | 3,2 |

Préparation 12

Le 4-(2-propynyl) 3-trifluorométhyl 1H-pyrrol 4-méthanol, utilisé au départ de l'exemple 45 peut être préparé de la façon suivante.

*STADE A:* 3-trifluorométhyl 1H-pyrrol 4-carboxylate d'éthyle.

Dans 200 cm³ de mélange de diméthylsulfoxyde et d'éther éthylique (1—2), on introduit 7,4 g de 4,4,4-trifluoro (E) butènoate d'éthyle /E.T. Mc. BEE, J. Am. Soc. Vol. 76, p. 3724 (1954)/ et 8,6 g d'isocyanure de tosylméthyle, ajoute par petites fractions 2,9 g d'hydrure de sodium à 50% dans l'huile de vaseline, observe un dégagement d'hydrogène, agite 30 minutes à 20°C, ajoute quelques gouttes d'acide acétique, verse dans un mélange d'eau et de glace, extrait à l'éther, concentre à sec par distillation sous pression réduite, chromatographie le résidu sur silice en éluant par un mélange d'hexane et d'acétate d'éthyle (7—3) et obtient 6,6 g de produit recherché. F = 165°C.

*Analyse:* $C_8H_8F_3NO_2$: 207,154.

| | | | | |
|---|---|---|---|---|
| Calculé: | C% 46,39 | H% 3,85 | F% 27,51 | N% 6,76 |
| Trouvé: | 46,5 | 3,9 | 27,1 | 6,8 |

*STADE B:* 1-(2-propynyl) 3-trifluorométhyl 1H-pyrrol 4-carboxylate d'éthyle.

Dans 10 cm³ de tétrahydrofuranne, on introduit 1 g de 3-trifluorométhyl 1H-pyrrol 4-carboxylate d'éthyle, obtenu au stade A puis à 0°C, par petites fractions, 240 mg d'hydrure de sodium à 50% dans l'huile de vaseline, agite pendant 30 minutes à 20°C, refroidit à 0°C, introduit une solution de 0,45 cm³ de bromure de propargyle dans 1 cm³ de tétrahydrofuranne, agite pendant 30 minutes à 0°C puis pendant 1 heure à 20°C, refroidit à 0°C, ajoute un peu d'hydrure de sodium, puis 0,45 cm³ de bromure de propargyle en solution dans 1 cm³ de tétrahydrofuranne, agite à 0°C, verse dans l'eau, extrait au chlorure de méthylène, concentre à sec par distillation sous pression réduite, chromatographie le résidu sur silice en éluant par un mélange d'hexane et d'acétate d'éthyle (8—2) et obtient 0,950 mg de produit recherché. F = 58°C.

*STADE C:* 1-(2-propynyl) 3-trifluorométhyl 1H-pyrrol-4-méthanol.

Dans une solution de 12,6 g de 1-(2-propynyl) 3-trifluorométhyl 1H-pyrrol 4-carboxylate d'éthyle obtenu au stade B, dans 65 cm³ de tétrahydrofuranne, on introduit à 0°C, par petites fractions, 1,95 g d'hydrure de lithium aluminium, agite pendant 2 heures à 20°C, ajoute 10 cm³ d'acétate d'éthyle pour éliminer l'excès éventuel d'hydrure, verse le mélange réactionnel dans une solution saturée de tartrate double de sodium et de potassium, extrait à l'acétate d'éthyle, concentre à sec par distillation sous pression réduite, chromatographie le résidu sur silice en éluant par un mélange d'hexane et d'acétate d'éthyle (7—3) et obtient 5,7 g de produit recherché. F = 45°C.

En opérant comme à l'exemple 43 ou selon le procédé (II) ou (III) en suivant les schémas indiqués ci-après, on a obtenu les produits suivants:

Exemple 46

IR,cis 2,2-diméthyl 3-(2,2-dibromoéthényl) cyclopropane carboxylate de RS cyano /(1-benzoyl) 1H-pyrrol-3-yl/ méthyle.

Exemple 47

IR,cis 2,2-diméthyl 3-(2,2-dibromoéthényl) cyclopropane carboxylate de /1-(2-propynyl 2-méthoxy 2-oxo 1H-pyrrol-3-yl/ méthyle.

Exemple 48

IR,cis 2,2-diméthyl 3-(2,2-dibromoéthényl) cyclopropane carboxylate de R ou S cyano /1-(2-propynyl 2-méthoxycarbonyl 1H-pyrrol-3-yl/ méthyle.

Exemple 49

1R,trans (E,1/3) (Z,2/3) 2,2-diméthyl 3-(2-chloro 2-trifluorométhyléthényl) cyclopropane carboxylate de /1-(2-propynyl 2-cyano 1H-pyrrol-3-yl/ méthyle.

Exemple 50

IR,cis 2,2-diméthyl 3-(2,2-dibromoéthényl) cyclopropane carboxylate de RS cyano /1-(2-propényl) 1H-pyrrol-3-yl/ méthyle.

Exemple 51

IR,cis 2,2-diméthyl 3-(3-méthoxy 3-oxo 1-propényl) cyclopropane carboxylate de /1-(2-propynyl) 2-cyano 1H-pyrrol-3-yl/ méthyle.

Exemple 52

IR,cis 2,2-diméthyl 3-(2-fluoro 2-éthoxy 2-oxo 1-propényl) cyclopropane carboxylate de /1-(2-propynyl) 2-cyano 1H-pyrrol-3-yl/ méthyle.

Exemple 46:

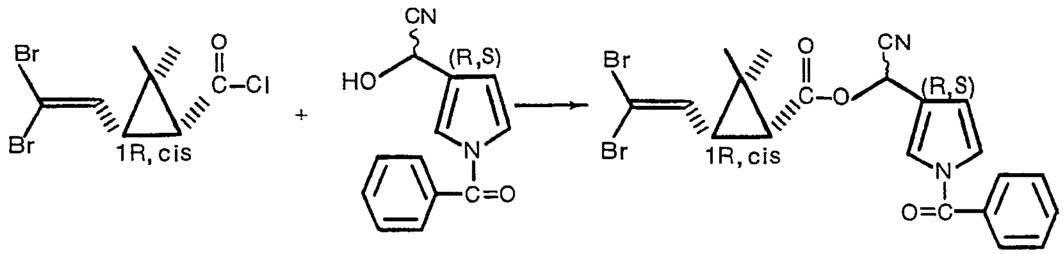

CCM=Rf=0,5 (hexane—acetate d'ethyle 7/3)

Exemple 47:

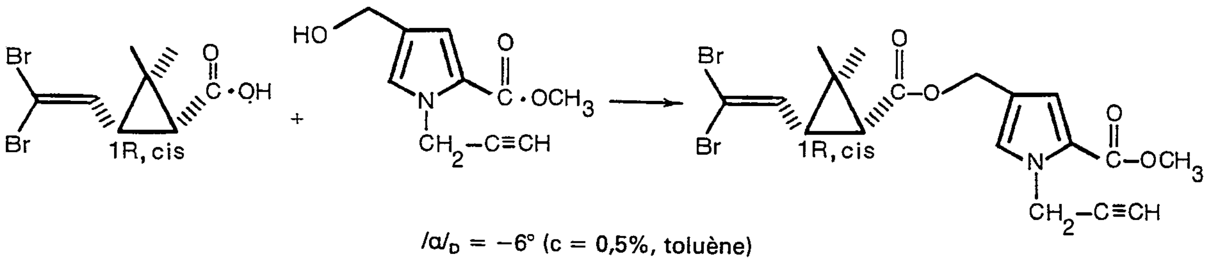

/α/$_D$ = −6° (c = 0,5%, toluène)

Préparation 13

L'alcool α-cyano 3-/(1-benzoyl) 1-pyrrol/ méthylique utilisé au départ de l'exemple 46 peut être préparé comme suit.

*STADE A:* 1-benzoyl 3-formyl pyrrole.

On dissout 500 mg de 3-formyl pyrrole dans 10 cm$^3$ de tétrahydrofuranne, ajoute en une seule fois à +20°C, 227 mg d'hydrure de sodium à 55% en dispersion dans l'huile de vaseline, note un dégagement gazeux, agite pendant 15 minutes, introduit goutte à goutte à +5°C, une solution de 0,58 cm$^3$ de chlorure de benzoyle dans 2 cm$^3$ de tétrahydrofuranne, agite pendant 2 heures à 20°C, dilue à l'eau, extrait à l'éther, concentre à sec par distillation sous pression réduite, chromatographie le résidu sur silice en éluant dans un mélange d'hexane et d'acétate d'éthyle (7—3) et obtient 558 mg de produit recherché.

Spectre de RMN (deutérochloroforme)
pics de 6,8 à 6,9 ppm atteibué à l'hydrogène en position 4 du pyrrole
pic à 7,4 ppm attribué à l'hydrogène en position 5 du pyrrole
pics de 7,5 à 8,0 ppm attribué aux hydrogènes du phényle
pic à 7,93 ppm attribué à l'hydrogène en position 2 du pyrrole
pic à 10,0 ppm attribué à l'hydrogène du formule.

*STADE B:* Alcool α-cyano 3-/(1-benzoyl) 1-pyrrol/ méthylique.

On mélange 0,9 g de 1-benzoyl 3-formyl pyrrole, 12 cm$^3$ de méthanol, 4 cm$^3$ d'eau, 1 cm$^3$ d'acide acétique et 0,47 g de cyanure de sodium, agite à +20°C pendant 4 heures, verse le mélange réactionnel dans l'eau salée glacée, extrait à l'acétate d'éthyle, concentre à sec par distillation sous pression réduite, chromatographie le résidu sur silice en éluant par un mélange d'hexane et d'acétate d'éthyle (1—1) et obtient 0,57 g de produit recherché. F = 85°C.

Spectre de RMN (deutérochloroforme)
pic à 5,5 ppm attribué à l'hydrogène de

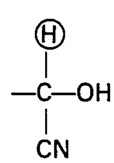

pic à 6,5 ppm attribué à l'hydrogène en 4 du pyrrole
pics de 7,3 à 7,9 ppm attribué aux hydrogènes du phényle.

Exemple 48:

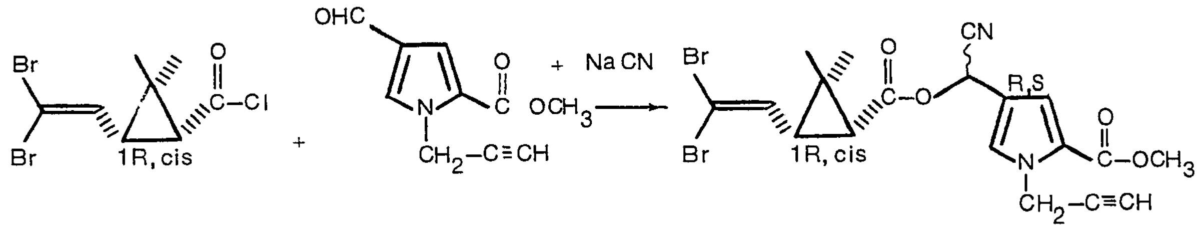

$/\alpha/_D = -36,5°$ (c = 0,4%, toluène)

Préparation 14

Le 4-(hydroxyméthyl) 1-(2-propényl) 1H-pyrrol 2-carboxylate de méthyle utilisé au départ de l'exemple 47 peut être préparé comme suit.

*STADE A:* 1H-pyrrol 2-carboxylate de méthyle.

Dans un mélange de 4,3 $cm^3$ de pyridine et 3,5 $cm^3$ de méthanol, on ajoute, goutte à goutte, à froid, 40 $cm^3$ d'une solution chlorométhylénique à 45 mmoles/1 de 2-chlorocarbonyl 1H-pyrrole. On lave à l'eau le mélange réactionnel puis avec 6 $cm^3$ de soude N, extrait au chlorure de méthylène, sèche les phases organiques, concentre à sec puis chromatographie le résidu sur silice, élue avec un mélange hexane-acétate d'éthyle (8—2), on obtient 4,014 g de produit attendu. F ≃ 74°C.

*STADE B:* 1-(2-propynyl) 1H-pyrrol 2-carboxylate de méthyle.

On dissout 3,302 g de 1H-pyrrol 2-carboxylate de méthyle dans 40 $cm^3$ de tétrahydrofuranne, ajoute à 0°C 1,422 g d'hydrure de sodium en suspension à 50% dans l'huile de vaseline, agite pendant 1 heure à 20°C, introduit goutte à goutte une solution de 2 $cm^3$ de bromure de propargyle dans 10 $cm^3$ de tétrahydrofuranne, agite pendant 11 heures à 50°C, après avoir ajouté 5 $cm^3$ de bromure de propargyle, verse le mélange réactionnel dans l'eau, extrait au chlorure de méthylène, concentre à sec par distillation sous pression réduite, chromatographie le résidu sur silice en éluant par un mélange d'hexane et d'acétate d'éthyle (9—1) et obtient 2,993 g de produit recherché.

Spectre de RMN (deutérochloroforme)

pics à 2,37—2,42—2,46 ppm attribués à l'hydrogène de $—H—C\equiv CH$

pic à 3,8 ppm attribué aux hydrogènes de $—OCH_3$

pics à 5,16—5,20 ppm attribués aux hydrogènes de $—CH_2—C\equiv CH$

pics de 6,1 à 6,2 ppm attribués à l'hydrogène en 4 du pyrrole

pics de 6,9 à 7,0 ppm et de 7,1 à 7,2 ppm attribués aux hydrogènes en position 3 et 5 du pyrrole.

*STADE C:* 4-formyl 1-(2-propynyl) 1H-pyrrol 2-carboxylate de méthyle.

On mélange 2,981 g de 1-(2-propynyl) 1H-pyrrol 2-carboxylate de méthyle dans 11 $cm^3$ de chlorure de méthylène et 1 $cm^3$ de nitrométhane dans 4 $cm^3$ de chlorure de méthylène pour obtenir la solution A. On met en suspension 4,312 g de chlorure d'aluminium dans 11 $cm^3$ de chlorure de méthylène, refroidit à −62°C et ajoute, goutte à goutte, la solution A puis une solution contenant 2,3 $cm^3$ de dichlorométhyl méthyl éther et 19 $cm^3$ de chlorure de méthylène. On agite 2 heures à −60°C puis 1 heure à température ambiante. On ajoute 200 $cm^3$ de chlorure de méthylène et 300 $cm^3$ d'eau, neutralise avec 11 $cm^3$ de soude 10N, agite, décante, lave la phase organique avec une solution de bicarbonate de potassium, sèche et concentre à sec. On chromatographie le résidu sur silice, élue par le mélange hexane-acétate d'éthyle (7—3) et obtient 2,734 g de produit attendu.

*STADE D:* 4-(hydroxyméthyl) 1-(2-propynyl) 1H-pyrrol 2-carboxylate de méthyle.

On dissout 747 mg de 4-formyl 1-(2-propynyl) 1H-pyrrol 2-carboxylate de méthyle dans 12 $cm^3$ de tétrahydrofuranne et 3,3 $cm^3$ d'eau, ajoute 426 mg d'hydrure de bore et de potassium, agite pendant 5 heures à 20°C, verse le mélange réactionnel dans une solution aqueuse saturée de chlorure de sodium, extrait au chlorure de méthylène, concentre à sec par distillation sous pression réduite et obtient 758 mg de produit recherché. F = 77°C.

Spectre IR (chloroforme)

absorption à 3609 $cm^{-1}$ attribué à —OH;

absorption à 3307 $cm^{-1}$ attribué à $—C\equiv CH$;

absorption à 2110 $cm^{-1}$ attribué à $—C\equiv C$;

absorption à 1702 $cm^{-1}$ attribué à —C=O;

absorption à 1445 $cm^{-1}$ attribué à $—CH_3$;

absorption à 1564, 1476 $cm^{-1}$ attribués à l'hétérocycle.

Exemple 49:

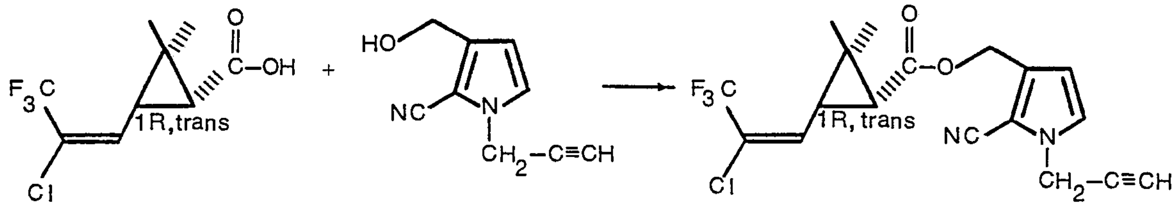

⅓(E) ⅔(Z)

/α/$_D$ = −5° (c = 0,3%, toluène)

Exemple 50:

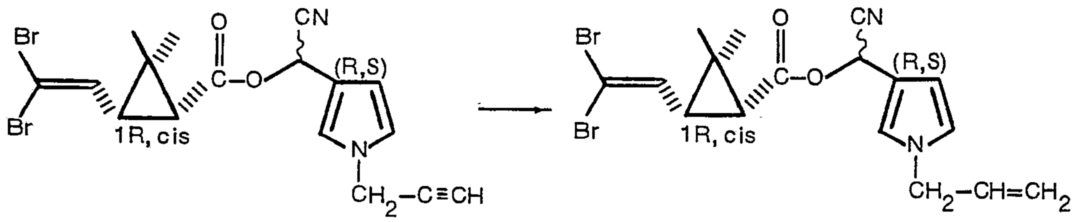

/α/$_D$ = −54° (c = 0,6%, toluène)

Exemple 50

1R,cis 2,2-diméthyl 3-(2,2-dibromoéthényl) cyclopropane carboxylate de R,S cyano /1-(2-propényl) 1H-pyrrol-3-yl/ méthyle.

Dans un appareil à hydrogèner, on mélange 682 mg de 1R,cis 2,2-diméthyl 3-(2,2-dibromoéthényl) cyclopropane carboxylate de R,S cyano /1-(2-propynyl) 1H-pyrrol-3-yl/ méthyle, 25 $cm^3$ de dioxanne, ajoute 147 mg de palladium sur sulfate de baryum à 5%, 1/10 $cm^3$ de quinoléine, purge, agite sous hydrogène, absorbe 35 $cm^3$ d'hydrogène et arrête l'hydrogénation. Le mélange réactionnel est filtré, concentré à sec le filtrat par distillation sous pression réduite, chromatographie le résidu en éluant par le mélange hexane-acétate d'éthyle (85—15) à 2% de triéthylamine et obtient 535 mg de produit recherche. F = 60°C.

[α]$_D$ = −54° (c = 0,6%, toluène).

Exemple 51:

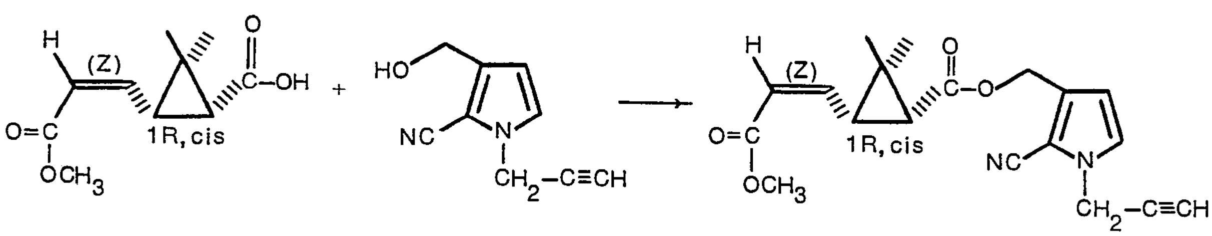

/α/$_D$ = +39° (c = 0,6%, toluène).

Exemple 52:

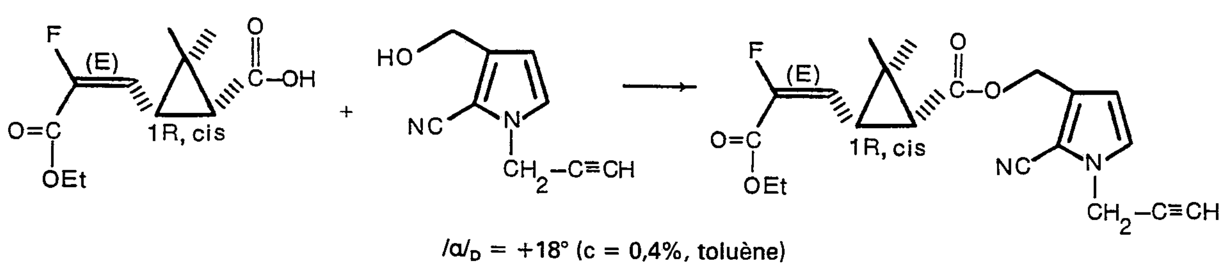

/α/$_D$ = +18° (c = 0,4%, toluène)

En opérant comme à l'exemple 43 ou selon le procédé (II) ou (III) ou (IV), en suivant les schémas indiqués, on a obtenu les produits suivants:

Exemple 53

1R,cis (E) 2,2-diméthyl 3-/2-fluoro 2-éthoxy 2-oxo 1-propényl/ cyclopropane carboxylate de /1-(2-propényl) 2-cyano 1H-pyrrol-4-yl/ méthyle.

Exemple 54

1R,cis 2,2-diméthyl 3-(2,2-dibromoéthényl) cyclopropane carboxylate de /1-(2-propényl) 2-cyano 1H-pyrrol-4-yl/ méthyle.

Exemple 55

1R,cis 2,2-diméthyl 3-(2,2-dibromoéthényl) cyclopropane carboxylate de R,S cyano /1-(2-propényl) 2-cyano 1H-pyrrol-4-yl/ méthyle.

Exemple 56

1R,cis (E) 2,2-diméthyl 3-/2-fluoro 3-éthoxy 3-oxo 1-propényl/ cyclopropane carboxylate de /1(2-propényl) 1H-pyrrol-3-yl/ méthyle.

Exemple 57

1R,cis (E) 2,2-diméthyl 3-/2-fluoro 3-éthoxy 3-oxo 1-propényl/ cyclopropane carboxylate de /1-(2-propynyl) 2-cyano 1H-pyrrol-4-yl/ propyn-2-yle.

Exemple 58

1R,cis (E) 2,2-diméthyl 3-/2-fluoro 3-éthoxy 3-oxo 1-propényl/ cyclopropane carboxylate de R,S cyano /1-(2-propényl) 2-cyano 1H-pyrrol-4-yl/ méthyle.

Exemple 59

1R,cis 2,2-diméthyl 3-(2,2-dibromoéthényl) cyclopropane carboxylate de /1-(2-propynyl) 2-cyano 1H-pyrrol-4-yl/ 1-éthyle.

Exemple 53:

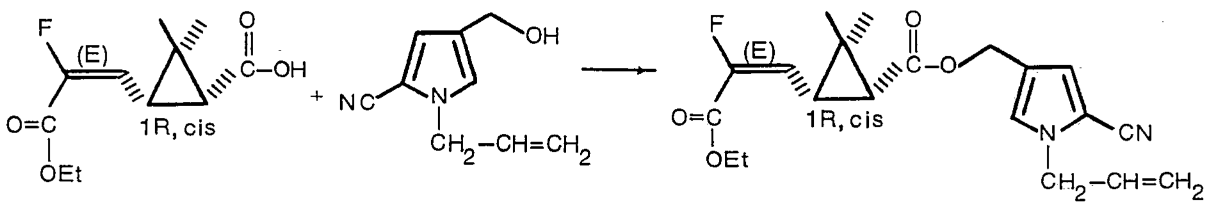

$/\alpha/_D = +25°$ (c = 0,7%, toluène)

Préparation 15

Le /2-cyano -1-(2-propényl) 1H-pyrrol-4-méthanol/ utilisé au départ de l'exemple 53 peut être préparé comme suit.

*STADE A:* 2-cyano 1-(2-propényl) 1H-pyrrol 4-carboxaldéhyde.

Dans un mélange de 20 g de 2-cyano 4-formyl 1H-pyrrole et de 250 $cm^3$ de tétrahydrofuranne, on ajoute par petites fractions 8,01 g de suspension d'hydrure de sodium à 50% dans l'huile de vaseline, ajoute à 20°C, goutte à goutte, le mélange de 14 $cm^3$ de bromure d'allyle et de 20 $cm^3$ de tétrahydrofuranne, agite pendant 2 heures à 60°C, ajoute 14 $cm^3$ de bromure d'allyle, agite pendant 2 heures à 60°C, verse le mélange réactionnel dans l'eau, extrait au chlorure de méthylène, concentre à sec par distillation sous pression réduite, chromatographie le résidu sur silice en éluant par un mélange d'hexane et d'acétate d'éthyle (6—4) et obtient 24,1 g de produit recherché.

*STADE B:* /2-cyano -1-(2-propényl) 1H-pyrrol-4-méthanol/.

On mélange 14,1 g de 4-formyl 2-cyano 1-(2-propényl) 1H-pyrrole, 160 $cm^3$ de tétrahydrofuranne, 43 $cm^3$ d'eau, ajoute 481 mg de borohydrure de lithium, agite à 20°C pendant 30 minutes, ajoute 493 mg de borohydrure de lithium, agite à 20°C pendant 30 minutes, ajoute de l'acétate d'éthyle, verse dans une solution aqueuse saturée de chlorure de sodium, extrait à l'acétate d'éthyle, concentre à sec par distillation sous pression réduite, chromatographie le résidu sur silice en éluant par un mélange d'hexane et d'acétate d'éthyle (55—45) et obtient 13,882 g de produit recherché.

Spectre de RMN (deutérochloroforme)

pic à 1,97 ppm attribué à l'hydrogène de —OH
pic à 4,5 ppm attribué aux hydrogènes de —$CH_2O$
pics à 4,5—4,7 ppm attribués aux hydrogènes de —N—$CH_2$
pics de 5,0 à 5,4 ppm attribués aux hydrogènes de =$CH_2$
pics de 5,7 à 6,2 ppm attribués à l'hydrogène de —CH=
pics à 6,8—6,9 ppm attribués aux hydrogènes aromatiques.

Exemple 54:

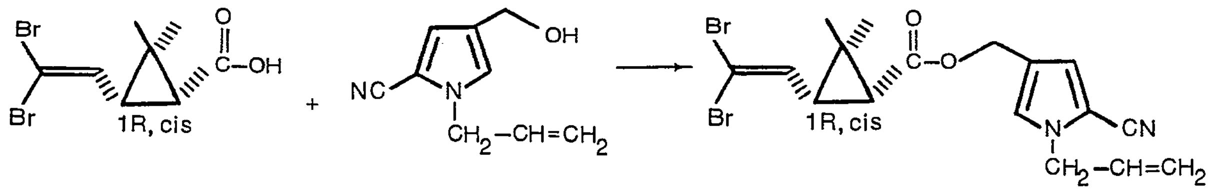

/α/$_D$ = −11,5° (c = 0,5%, toluène)

Exemple 55:

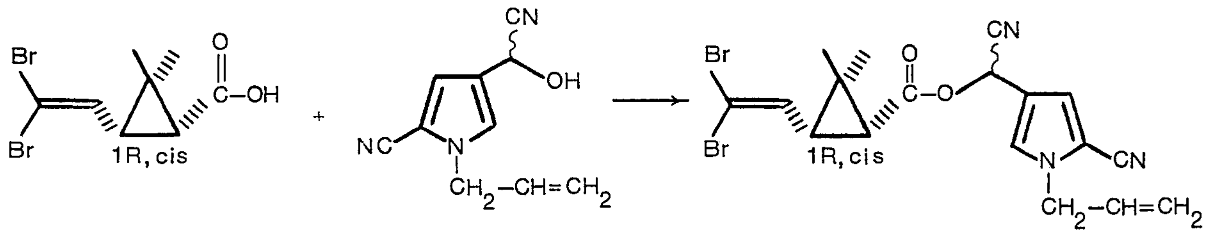

/α/$_D$ = +7° (c = 0,5%, toluène)

Exemple 56:

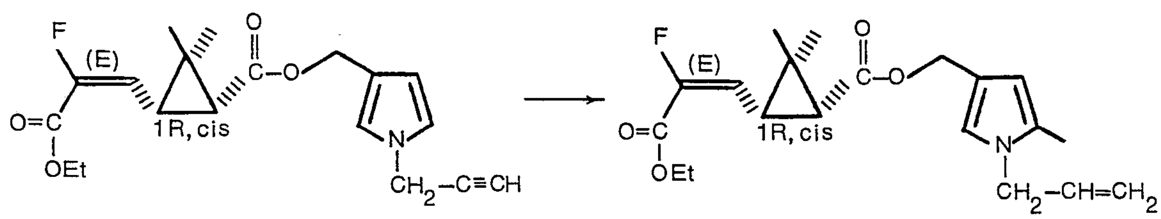

/α/$_D$ = +15,5° (c = 0,4%, toluène)

Préparation 16

L'α-hydroxy /2-cyano 1-(2-propényl)/ 1H-pyrrol 4-acétonitrile utilisé au départ de l'exemple 55 peut être préparé comme suit.

On mélange 1,072 g de 2-cyano 1-(2-propényl) 1H-pyrrol 4-carboxaldéhyde, 15 $cm^3$ de méthanol, 4,5 $cm^3$ d'eau et 2,5 $cm^3$ d'acide acétique, ajoute à 0°C, 2 g de cyanure de sodium, agite à 20°C pendant 4 heures et 30 minutes, ajoute 3 $cm^3$ d'acide acétique, 3 g de cyanure de sodium, agite pendant 2-heures à 20°C, lave à l'eau, extrait au chlorure de méthylène, concentre à sec par distillation sous pression réduite et obtient 1,25 g de produit recherché utilisé tel quel.

Exemple 56

1R,cis (E) 2,2-diméthyl 3-(2-fluoro 3-éthoxycarbonyl 1-propényl) cyclopropane carboxylate de 1-(2-propényl 1H-pyrrol-3-yl) méthyle.

Dans un appareil à hydrogéner, on introduit 700 mg de 1R,cis (E) 2,2-diméthyl 3-(2-fluoro 3-éthoxycarbonyl 1-propényl) cyclopropane carboxylate de 1-(2-propynyl 1H-pyrrol-3-yl) méthyle, 0,14 $cm^3$ de quinoléine, 200 mg de palladium sur sulfate de baryum à 5%, agite sous hydrogène, consomme 45 $cm^3$ d'hydrogène, arrête la réaction, filtre, concentre à sec le filtrat par distillation sous pression réduite, chromatographie le résidu sur silice en éluant avec un mélange d'hexane et d'acétate d'éthyle (7—3) à 2% de triéthylamine et obtient 482 mg de produit recherché.

[α]$_D$ = +15,5° (c = 0,4%, toluène).

Exemple 57:

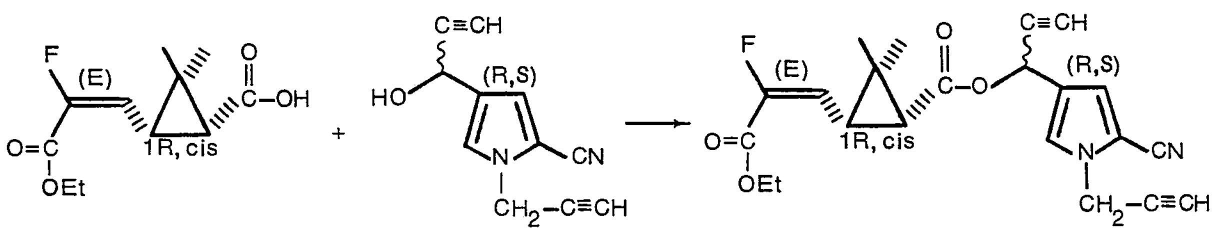

/α/$_D$ = +19° ± 2° (c = 0,5%, toluène)

Préparation 17

Le 1RS hydroxy /2-cyano 1-(2-propynyl) 1H-pyrrol-4-yl/ 2-propynyle utilisé au départ de l'exemple 59 peut être préparé comme suit.

Dans 70 cm³ de solution de bromure d'éthynyl magnésium dans le tétrahydrofuranne titrant 0,82 mmole/l, on introduit à +20°C en 10 minutes environ, 3 g de 1-(2-propynyl) 2-cyano 4-formyl 1H-pyrrole en solution dans 20 cm³ de tétrahydrofuranne, agite pendant 3 heures et 30 minutes, verse le mélange réactionnel dans une solution aqueuse saturée au phosphate monosodique, extrait à l'éther, concentre à sec par distillation sous pression réduite, purifie le résidu par chromatographie sur silice en éluant par un mélange d'hexane et d'acétate d'éthyle (1—1) et obtient 2,897 g. F = 66°C.

Spectre de RMN (deutérochloroforme)

pics à 2,3—2,4 ppm attribués à l'hydrogène de l'hydroxyle

pics à 2,51—2,55—2,59 ppm attribués à l'hydrogène de —C≡CH du propynyle

pics à 2,61—2,65 ppm attribués à l'hydrogène

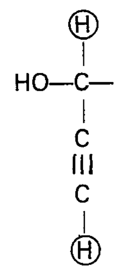

pics à 4,77—4,82 ppm attribués aux hydrogènes du méthylène de $-CH_2-CH{\equiv}CH$

pics à 5,40 ppm attribué à l'hydrogène de

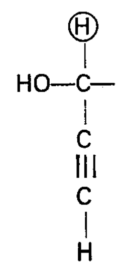

pics à 6,90—6,93 ppm et 7,17—7,20 ppm attribués aux hydrogènes en 3 et 5 du pyrrole.

Exemple 58:

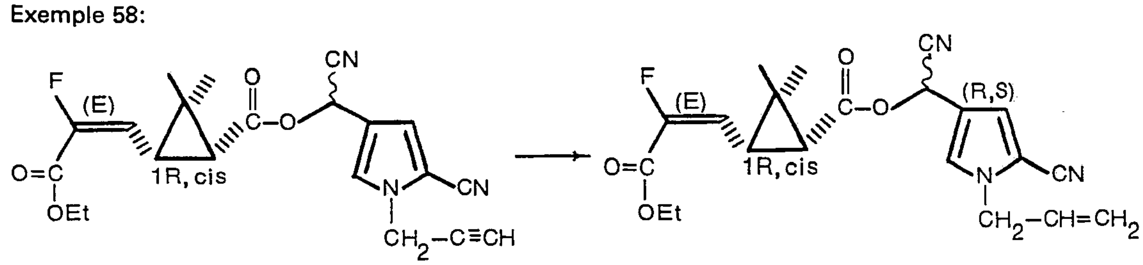

$/\alpha/_D = +39,5°$ (c = 0,4%, toluène)

Exemple 59:

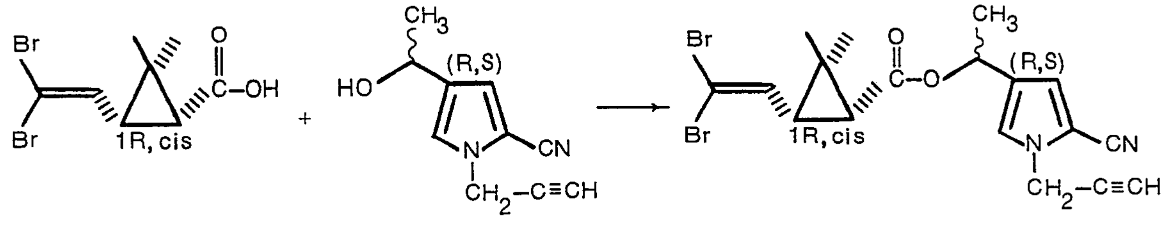

$/\alpha/_D = -6,5° \pm 2$ (c = 6%, toluène)

Préparation 18

Le 2-cyano 4-/1(RS) hydroxyéthyl 1-(2-propynyl/ 1H-pyrrole utilisé au départ de l'exemple 59 peut être préparé comme suit.

Dans 50 $cm^3$ d'une solution d'iodure de méthyl magnésium dans le tétrahydrofuranne titrant 1,7 mmol/l, on introduit lentemment à +10°C une solution de 4 g de 1-(2-propynyl) 2-cyano 4-formyl 1H-pyrrole dans 45 $cm^3$ de tetrahydrofuranne, agite pendant 4 heures à 20°C, verse le mélange réactionnel dans une solution aqueuse saturée de phosphate monosodique, extrait à l'éther, concentre à sec par distillation sous pression réduite, chromatographie le résidu sur silice en éluant par un mélange d'hexane et d'acétate d'éthyle (1—1) et obtient 2,855 g de produit recherché. F < 50°C.

Spectre de RMN (deutérochloroforme)

pics à 1,4—1,5 ppm attribués aux hydrogènes du méthyle

pics à 2,50—2,54—2,58 ppm attribués à l'hydrogène de —C≡C—H

pics à 4,68—4,80 et 4,90—5,03 ppm attribués à l'hydrogène de

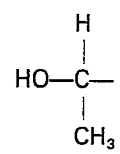

pics à 4,76—4,80 ppm attribués aux hydrogènes du méthylène de $CH_2$—C≡C—H

pics à 6,80—6,83 ppm et 4,91 et 5,03 ppm attribués aux hydrogènes en 3 et en 5 du pyrrole.

En opérant comme à l'exemple 43 ou comme selon le procédé (I), en suivant les schémas indiqués, on a obtenu les produits suivants:

Exemple 60

1R,cis (E) 2,2-diméthyl 3-(2-fluoro 3-éthoxy 3-oxo 1-propényl) cyclopropane carboxylate de /1-(2-propynyl) 2-cyano 1H-pyrrol-4-yl/ éthyle.

Exemple 61

1R,cis 2,2-diméthyl 3-(2,2-dibromoéthényl) cyclopropane carboxylate de 1R,S /1-(2-propynyl) 2-cyano 1H-pyrrol-4-yl/ 2-propynyle.

Exemple 62

1R,cis 2,2-diméthyl 3-(2,2-dibromoéthényl) cyclopropane carboxylate de /1-(2-propynyl) 4-cyano 1H-pyrrol-3-yl/ méthyle.

Exemple 63

1R,cis (E) 2,2-diméthyl 3-(2-fluoro 3-éthoxy 3-oxo 1-propényl) cyclopropane carboxylate de /1-(2-propynyl) 4-cyano 1H-pyrrol-3-yl/ méthyle.

Exemple 64

1R,cis 2,2-diméthyl 3-(2,2-dibromoéthényl) cyclopropane carboxylate de R,S cyano /1-(2-propynyl) 4-cyano 1H-pyrrol-3-yl/ méthyle.

Exemple 65

1R,cis (E) 2,2-diméthyl 3-(2-fluoro 3-éthoxy 3-oxo 1-propényl) cyclopropane carboxylate de /2-cyano /1-(3-chloro 2 (Z) propényl/ 1H-pyrrol-4-yl/ méthyle.

Exemple 66

1R,cis (E) 2,2-diméthyl 3-(2-fluoro 3-éthoxy 3-oxo 1-propényl) cyclopropane carboxylate de /2-cyano /1-(3-chloro 2 (E) propényl/ 1H-pyrrol-4-yl/ méthyle.

Exemple 67

1R,cis (E) 2,2-diméthyl 3-(2-fluoro 3-éthoxy 3-oxo 1-propényl) cyclopropane carboxylate de (R,S) cyano /1-(2-propynyl) 2-cyano 1H-pyrrol-3-yl/ methyle.

Exemple 60:

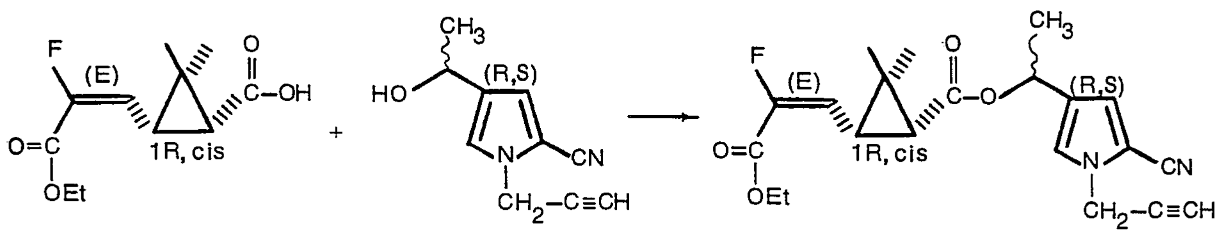

/α/$_D$ = +21° ± 2° (c = 0,6%, toluène)

Exemple 61:

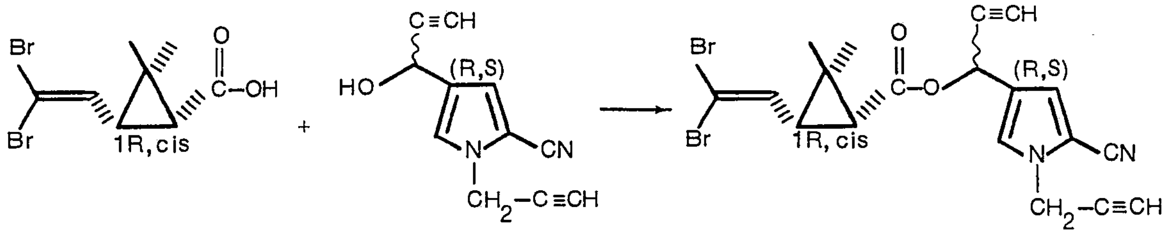

$/\alpha/_D$ = 10° ± 2° (c = 0,5%, toluène)

Exemple 62:

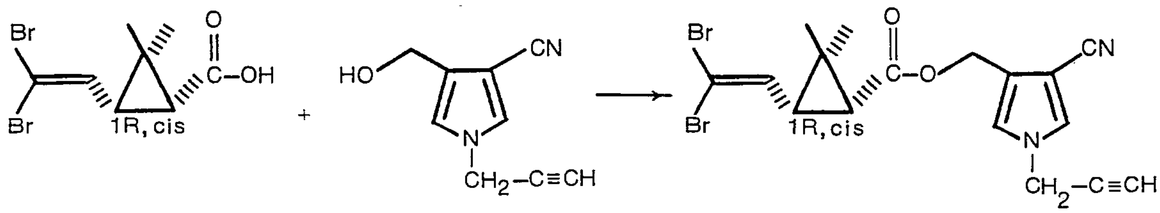

$/\alpha/_D$ = −10,5° (c = 0,5%, toluène)

Exemple 63:

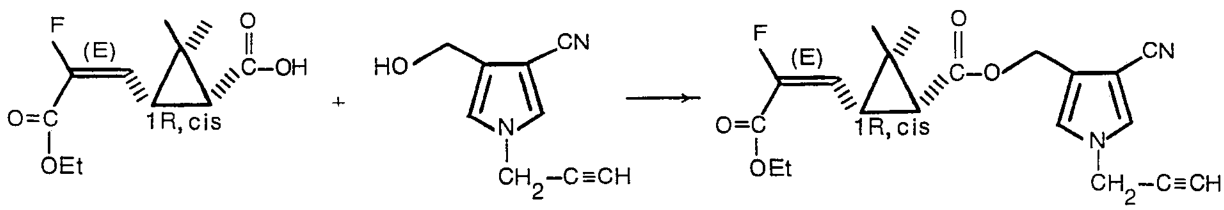

$/\alpha/_D$ = +3,2° (c = 0,5%, toluène)

Exemple 64:

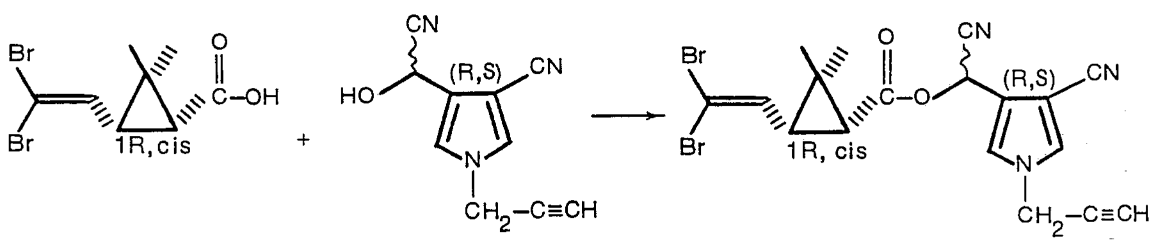

$/\alpha/_D$ = −15,5° (c = 0,5%, toluène)

Préparation 19

Le 4-/(R,S) α-cyano méthanol/ 1-(2-propynyl) 1H-pyrrol 3-carbonitrile utilisé au départ de l'exemple 64 peut être préparé comme suit.

*STADE A:* (1,4-bis diméthylamino) 2-cyano 3-carboxylate de méthyle 1,3- butadiène.

On mélange 23 g de α-cyano propionate de méthyle, 126 cm³ de terbutoxy bis (diméthylamino) méthane, porte le mélange réactionnel à 170°C pendant 5 heures, on distille au début de l'isobutanol, laisse le mélange réactionnel descendu à 50°C, place le mélange réactionnel sous vide (0,1 mn de mercure), porte à 110°C pendant 1 heure, purifie par chromatographie sur silice en éluant par un mélange d'hexane et d'acétate d'éthyle (1—9) et obtient 15,9 g de produit recherché. F = 106°C.

*STADE B:* 3-cyano 1-(2-propynyl) 1H-pyrrol 4-carboxylate de méthyle.

On mélange 14,5 g de 1,4-bis (diméthylamino) 2-cyano 3-carboxylate de méthyle 1,3-butadiène, 400 cm³ d'éthanol, 4,2 cm³ de monopropyargylamine, porte le mélange réactionnel au reflux pendant 5 heures, refroidit, verse sur une solution aqueuse saturée au chlorure de sodium, extrait au chlorure de méthylène, concentre à sec par distillation sous pression réduite, chromatographie le résidu sur silice en éluant par un mélange d'hexane et d'acétate d'éthyle (65—35) et obtient 3,75 g de produit recherché. F = 134°C.

*STADE C:* 4-hydroxyméthyl 1-(2-propynyl) 1H-pyrrol 3-carbonitrile.

On mélange 3,90 g de 3-cyano 1-(2-propynyl) 1H-pyrrol 4-carboxylate de méthyle, 35 $cm^3$ de tétrahydrofuranne, 11 $cm^3$ d'eau, 1,556 g de borohydrure de lithium, agite pendant 3 heures et 30 minutes à 20°C, verse dans une solution aqueuse saturée de chlorure de sodium, agite, extrait à l'acétate d'éthyle, concentre à sec par distillation sous pression réduite, chromatographie le résidu sur silice en éluant avec un mélange d'hexane et d'acétate d'éthyle (4—6) et obtient 1,33 g de produit recherché. F = 95°C.

*STADE D:* 4-formyl 1-(2-propynyl) 1H-pyrrol 3-carbonitrile.

Dans 9,5 $cm^3$ de solution molaire de chlorure d'oxalyle dans le chlorure de méthylène, on introduit goutte à goutte à −60°C, 18 $cm^3$ de solution molaire de diméthylsulfoxyde dans le chlorure de méthylène, agite à −60°C pendant 5 minutes, ajoute goutte à goutte à −60°C une solution de 724 g de 4-hydroxylméthyl 1-(2-propynyl) 1H-pyrrol 3-carbonitrile, agite à −60°C pendant 5 heures, ajoute goutte à goutte à −60°C 25 $cm^3$ de solution molaire de triéthylamine dans le chlorure de méthylène, agite à −60°C pendant 30 minutes, laisse le mélange réactionnel revenir à température ambiante, ajoute de l'eau, agite décante, lave la phase organique par une solution aqueuse saturée de chlorure de sodium, concentre à sec par distillation sous pression réduite, chromatographie le résidu sur silice en éluant par un mélange d'hexane et d'acétate d'éthyle (1—1) et obtient 702 mg de produit recherché. F = 114°C.

*STADE E:* 4-/(R,S) α-cyano méthanol/ 1-(2-propynyl) 1H-pyrrol 3-carbonitrile.

On mélange 495 mg de 4-formyl 1-(2-propynyl) 1H-pyrrol 3-carbonitrile 15 $cm^3$ de méthanol, 2,1 $cm^3$ d'eau, 1,2 $cm^3$ d'acide acétique, 900 mg de cyanure de sodium, laisse revenir à 20°C, agite pendant 15 minutes à 20°C, lave à l'eau, extrait à l'acétate d'éthyle, concentre à sec par distillation sous pression réduite et utilise le produit brut pour l'exemple 46.

Exemple 65:

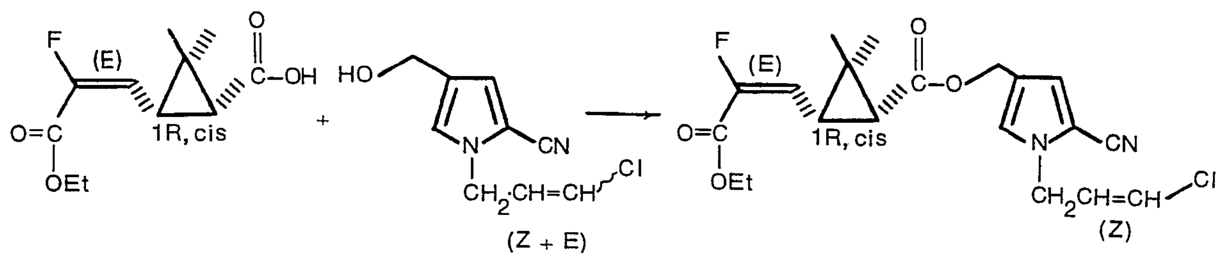

/α/$_D$ = +32,5° (c = 0,8%, toluène)

Exemple 66:

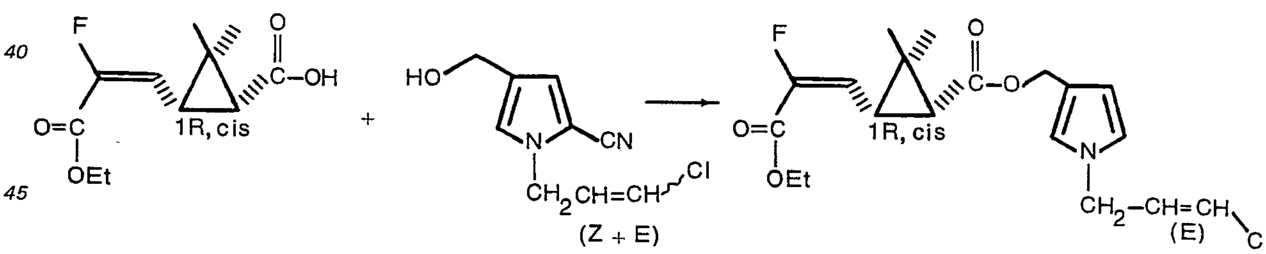

/α/$_D$ = +28° (c = 0,5%, toluène)

Exemple 65

1R,cis (E) 2,2-diméthyl 3-/2-fluoro 3-éthoxy 3-oxo 1-propényl/ cyclopropane carboxylate de /2-cyano 1-/3-chloro 2 (Z)-propényl/ 1H-pyrrol-4-yl/ méthyle.

On mélange 1,55 g de /2-cyano 1-/3-chloro 2 (Z + E) propényl/ 1H-pyrrol-4-yl/ méthanol, 1,8 g d'acide 1R,cis (E) 2,2-diméthyl 3-(2-fluoro 3-éthoxy 3-oxo 1-propényl) cyclopropane carboxylique, 20 $cm^3$ de dichlorométhane, introduit à +5°C, progressivement un mélange de 1,625 g de dicyclohexylcarbodiimide, 10 $cm^3$ de dichlorométhane et 29 mg de 4-diméthyl amino pyridine, agite pendant 20 heures à 20°C, filtre, concentre le filtrat à sec par distillation sous pression réduite, chromatographie sur silice en éluant avec le mélange hexane-acétate d'éthyle (7—3), puis par un mélange d'hexane et d'acétate d'éthyle (9—1) et obtient 0,83 g de produit recherché.

[α]$_D$ = +32,5° (c = 0,8%, toluène).

Exemple 66

1R,cis (E) 2,2-diméthyl 3-/2-fluoro 3-éthoxy 3-oxo 1-propényl/ cyclopropane carboxylate de /2-cyano 1-/3-chloro 2 (E) propényl/ 1H- pyrrol-4-yl/ méthyle.

En poursuivant le chromatographie de l'exemple 65, on obtient 0,32 g de produit recherché.

[α]$_D$ = +28° (c = 0,5%, toluène).

Préparation 20

Le /4-hydroxyméthyl 2-cyano 1-(3-chloro 2-propényl) 1H-pyrrole (cis, trans)/ utilisé au départ des exemples 65 et 66 peut être préparé de la façon suivante.

*STADE A:* 4-formyl 2-cyano 1-(3-chloro 2-propényl) 1H-pyrrole cis trans.

On introduit 4,15 g de 2-cyano 4-formyl 1H-pyrrole dans 20 $cm^3$ de diméthylformamide, refroidit à +10°C et ajoute par petites fractions 1,563 g d'hydrure de sodium à 55% en dispersion dans l'huile et agite ensuite 30 minutes à +15°C. On refroidit à +5°C et introduit en 20 minutes 32 $cm^3$ de 1,3-dichloropropène et agite pendant 2 heures à +15°C. On verse le mélange réactionnel dans l'eau et extrait à l'éther. On sèche, concentre les phases éthérées, chromatographie le résidu sur silice, élue avec un mélange hexane-acétate d'éthyle (1—1) et obtient 6,4 g de produit attendu.

*STADE B:* 4-hydroxyméthyl 2-cyano 1-(3-chloro 2-propényl) 1H-pyrrole (cis, trans).

On agite pendant 45 minutes 4 g de produit obtenu ci-dessus, 100 $cm^3$ de tétrahydrofuranne, 22 $cm^3$ d'eau distillée, 4,4 g de borohydrure de potassium, ajoute de l'éther éthylique et agite 2 heures. On décante, extrait à nouveau à l'éther, sèche les phases organiques et amène à sec.

Exemple 67:

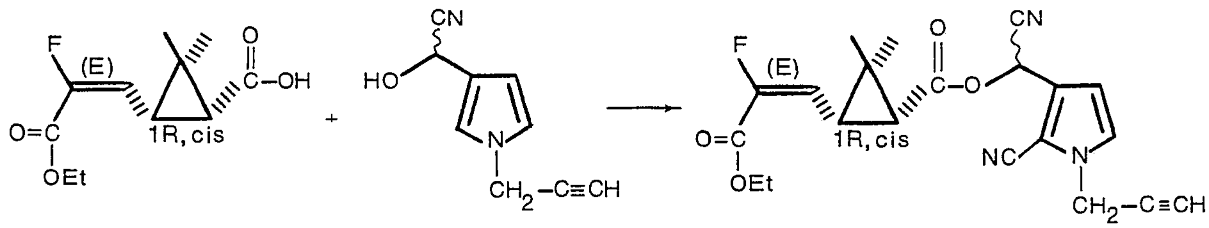

$/\alpha/_D$ = +21° (c = 0,5%, toluène).

Préparation 21

Le 2-hydroxy (R,S) 2-cyano 1-(2-propynyl) 1H-pyrrol 3-acétonitrile utilisé au début de l'exemple 67 peut être préparé de la manière suivante.

*STADE A:* 3-formyl 2-cyano 1-(2-propynyl) 1H-pyrrole.

On refroidit à −60°C 27 $cm^3$ d'une solution 1M de chlorure d'oxalyle dans du chlorure de méthylène, ajoute 13,5 $cm^3$ d'une solution 1M de diméthyl sulfoxyde dans le chlorure de méthylène, agite 5 minutes et ajoute 1,09 g de 3-hydroxy méthyl 1-(2-propynyl) 1H-pyrrol 2-carbonitrile dans 20 $cm^3$ de chlorure de méthylène en maintenant la température à −60°C pendant 2 heures. On ajoute 70 $cm^3$ d'une solution 1M de triéthylamine dans du chlorure de méthylène, agite 10 minutes à −60°C et laisse remonter la température à 20°C. On ajoute 20 $cm^3$ d'eau, agite, décante, concentre à sec. On chromatographie sur silice le résidu, élue par un mélange hexane-acétate d'éthyle (6—4) et obtient 456 mg de produit attendu.

Spectre de RMN ($CDCl_3$)

pics à 2,59—2,63—2,65 ppm proton du —C≡CH

pics à 6,75—6,8 et 7,15—7,18 ppm protons du pyrrole

pics à 4,9—4,95 ppm protons du $CH_2$ du propynyle

pics à 10 ppm proton du formyle.

*STADE B:* 2-hydroxy (R,S) 2-cyano 1-(2-propynyl) 1H-pyrrol 3-acétonitrile.

On opère comme au stade E de la préparation 19 à partir de 546 mg de 3-formyl 2-cyano 1-(2-propynyl) 1H-pyrrole et obtient le produit brut attendu.

En opérant comme à l'exemple 1, en suivant les schémas indiqués, on a obtenu les produits suivants:

Exemple 68

1R,cis (E) 2,2-diméthyl 3-/2-fluoro 3-éthoxy 3-oxo 1-propényl/ cyclopropane carboxylate de RS cyano /1-(2-propynyl) 3-cyano 1H-pyrrol-4-yl/ méthyle.

Exemple 69

1R,cis (Z) 2,2-diméthyl 3-/3-méthoxy 3-oxo 1-propényl/ cyclopropane carboxylate de RS cyano /1-(2-propynyl) 2,2,2-trifluorométhyl 1H-pyrrol-4-yl/ méthyle.

Exemple 70

1R,cis (E) 2,2-diméthyl 3-/2-fluoro 3-éthoxy 3-oxo 1-propényl/ cyclopropane carboxylate de /1-(2-propynyl) 2,2,2-trifluorométhyl 1H-pyrrol-4-yl/ méthyle.

Exemple 71

1R,cis (Z) 2,2-diméthyl 3-/3-méthoxy 3-oxo 1-propényl/ cyclopropane carboxylate de /1-(2-propynyl) 3,3,3-trifluorométhyl 1H-pyrrol-4-yl/ méthyle.

Exemple 72

1R,cis (E) 2,2-diméthyl 3-/2-fluoro 3-éthoxy 3-oxo 1-propényl/ cyclopropane carboxylate de /1-(2-propynyl) 3,3,3-trifluorométhyl 1H-pyrrol-4-yl/ méthyle.

Exemple 73

1R,cis 2,2-diméthyl 3-(2,2-dibromoéthényl) cyclopropane carboxylate de /2-cyano 1-/3-chloro 2-(Z)-propényl/ 1H-pyrrol/ méthyle.

Exemple 74

1R,cis 2,2-diméthyl 3-(2,2-dibromoéthényl) cyclopropane carboxylate de /2-cyano 1-/3-chloro 2-(E)-propényl/ 1H-pyrrol-4-yl/ méthyle.

Exemple 68:

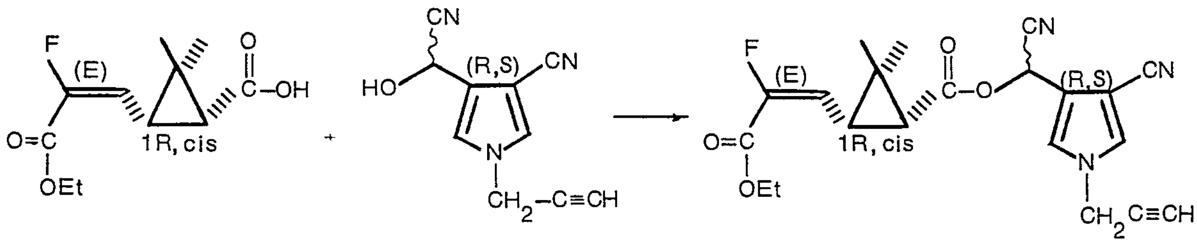

$/\alpha/_D = +12{,}5°$ (c = 0,6%, toluène)

Exemple 69:

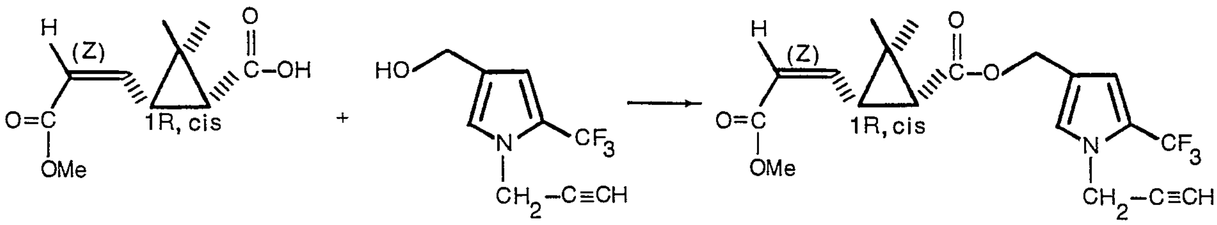

$/\alpha/_D = +59° \pm 4°$ (c = 0,4%, toluène)

Exemple 70:

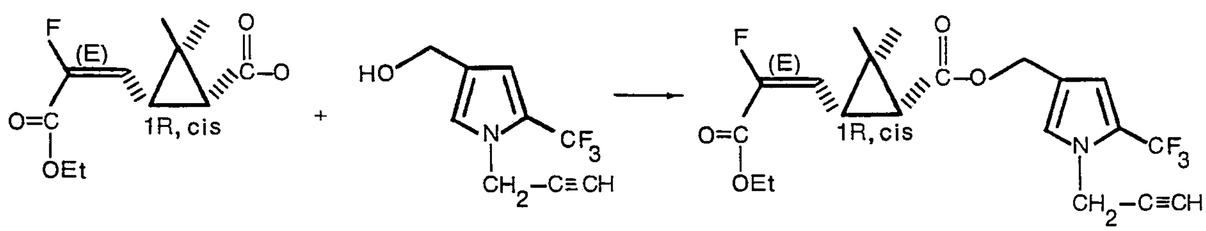

$/\alpha/_D = +40{,}5° \pm 2{,}5$ (c = 0,5%, toluène)

Préparation 22

Le 2-trifluorométhyl 1-(2-propynyl) 1H-pyrrol-4-méthanol utilisé au départ des exemples 69 et 70 peut être préparé comme suit:

*STADE A:* 4-/1-(2-propynyl) 2-trifluorométhyl 1H-pyrrol/ carboxylate d'éthyle.

On mélange 9 g de chlorhydrate de l'acide 3-aza 5-hexyne 1-oïque, 120 $cm^3$ de toluène, 9,15 $cm^3$ de propionate d'éthyle, introduit à +5°C, progressivement 18,6 $cm^3$ d'anhydride trifluoroacétique, porte le mélange réactionnel au reflux pendant 4 heures et 45 minutes, refroidit à +30°C, ajoute 9,3 $cm^3$ d'anhydride trifluoroacétique, porte le mélange réactionnel au reflux pendant 5 heures, agite pendant 12 heures à 20°C, ajoute 5,6 $cm^3$ d'anhydride acétique, porte le mélange réactionnel pendant 2 heures au reflux, refroidit, verse le mélange réactionnel dans l'eau, ajoute de l'éther, neutralise par addition d'une solution saturée de bicarbonate de sodium, extrait à l'éther, concentre à sec par distillation sous pression réduite, chromatographie le résidu sur silice en éluant par un mélange d'hexane et d'acétate d'éthyle (8—2) et obtient 3,964 g de produit recherché. F < 50°C.

Spectre IR (chloroforme)

absorption à 1712 $^{cm^{-1}}$ attribuée à ester C
||
O

absorption à 3140 $cm^{-1}$, 1572 $cm^{-1}$, 1519 $cm^{-1}$ attribuée à hétérocycle =CH
absorption à 1187 $cm^{-1}$, 1162 $cm^{-1}$, 1116 $cm^{-1}$ attribuée à $CF_3$
absorption à 3307 $cm^{-1}$ attribuée à C≡CH, ≡CH
absorption à 2125 $cm^{-1}$ attribuée à C≡C

*STADE B:* 2-trifluorométhyl 1-(2-propynyl) 1H-pyrrol-4-méthanol.

On dissout 4,785 g de 4-/1-(2-propynyl) 2-trifluorométhyl 1H-pyrrol/ carboxylate d'éthyle dans 40 $cm^3$ de tétrahydrofuranne, ajoute à 0°C, par petites fractions, 750 mg d'hydrure de lithium et d'aluminium, agite pendant 30 minutes à +7°C, puis pendant 3 heures à 20°C, ajoute quelques gouttes d'acétate d'éthyle et 10 $cm^3$ de solution aqueuse saturée de tartrate double de sodium, filtre le mélange réactionnel, concentre le filtrat à sec par distillation sous pression réduite, chromatographie le résidu sur silice en éluant avec un mélange d'hexane et d'acétate d'éthyle (6—4) et obtient 3,45 g de produit recherché.

Spectre IR (chloroforme)
absorption à 3610 $cm^{-1}$ attribuée à —OH
absorption à 3308 $cm^{-1}$ attribuée à —C≡CH
absorption à 1580 $cm^{-1}$, 1510 $cm^{-1}$, attribuée à 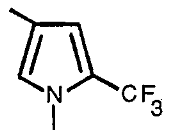

Exemple 71:

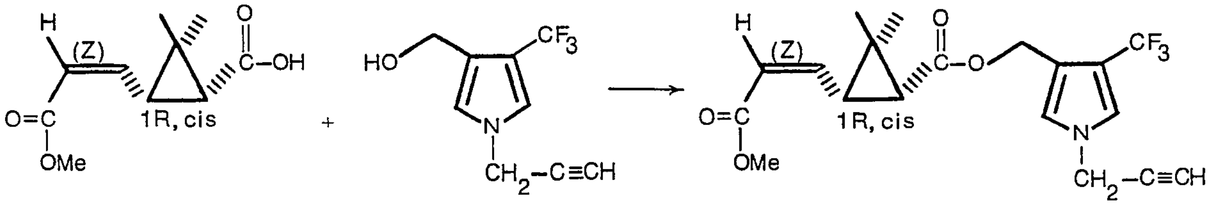

/α/$_D$ = +41,5° (c = 0,5%, chloroforme)

Exemple 72:

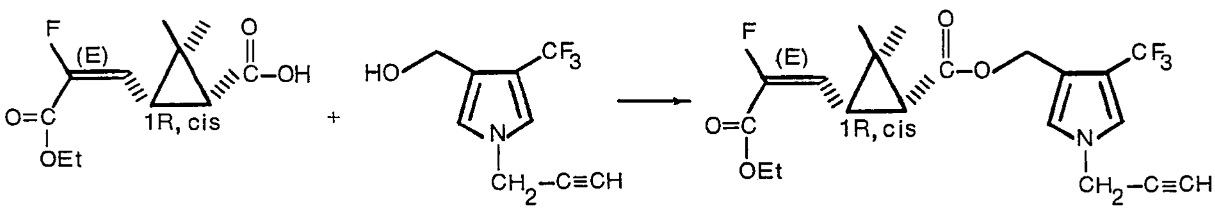

/α/$_D$ = +23,5° ± 3° (c = 0,2%, chloroforme)

De façon analogue aux exemples 65 et 66, on prépare les exemples 73 et 74.

Exemple 73:

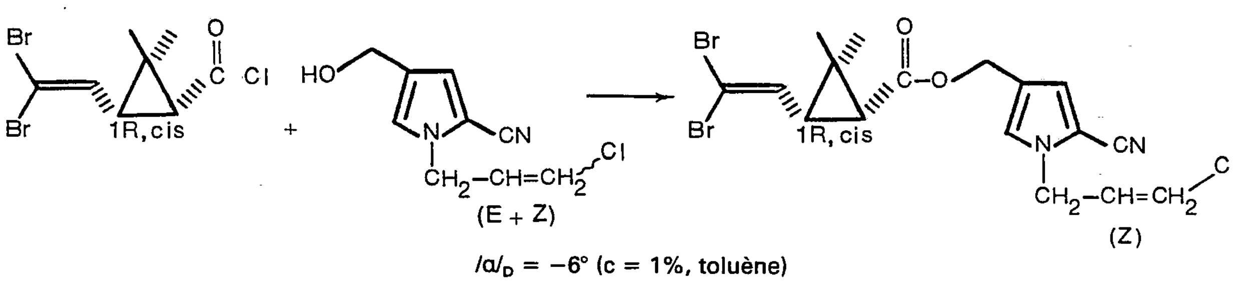

/α/$_D$ = −6° (c = 1%, toluène)

Exemple 74:

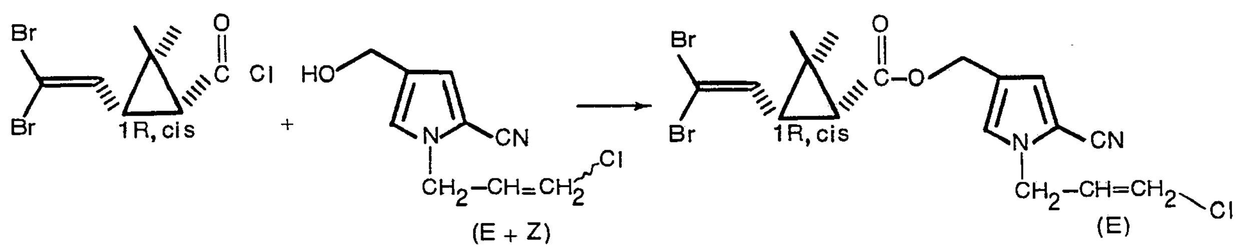

/α/$_D$ = −4° (c = 0,6%, toluène).

Exemple 75: préparation d'un concentré soluble

On effectue un mélange homegène de

| | |
|---|---|
| Produit de l'exemple 1 | 0,25 g |
| Butoxyde de pipéronyle | 1,00 g |
| Tween 80 | 0,25 g |
| Topanol A | 0,1 g |
| Eau | 98,4 g |

Exemple 76: préparation d'un concentré émulsifiable

On mélange intimement:

| | |
|---|---|
| Produit de l'exemple 2 | 0,015 g |
| Butoxyde de pipéronyle | 0,5 g |
| Topanol A | 0,1 g |
| Tween 80 | 3,5 g |
| Xylène | 95,885 g |

Exemple 77: préparation d'un concentré émulsifiable

On effectue un mélange homogéne de:

| | |
|---|---|
| Produit de l'exemple 40 | 1,5 g |
| Tween 80 | 20,00 g |
| Topanol A | 0,1 g |
| Xylène | 78,4 g |

Exemple 78: préparation d'une composition fumigène

On mélange d'une façon homogène:

| | |
|---|---|
| Produit de l'exemple 1 | 0,25 g |
| Poudre de tabu | 25,00 g |
| Poudre de feuille de cédre | 40,000 g |
| Poudre de bois de pin | 33,75 g |
| Vert brillant | 0,5 g |
| p-nitrophénol | 0,5 g |

Exemple 79: Exemple d'aliment composé pour animaux

On utilisé comme aliment équilibré de base un aliment comportant du maïs, de la luzerne déshydratée, de la paille de blé, du tourteau de palmiste mélassé, de l'urée, un condiment minéral vitaminé.

Cet aliment contient au minimum 11% de matières protéiques brutes (dont 2,8% apportés par l'urée), 2,5% de matières grasses et au maximum 15% de matières cellulosiques, 6% de matières minérales et 13% d'humidité.

L'aliment utilisé correspond à 82 unités fourragères pour 100 kilos et contient pour 100 kilos 910.000 U.I. de vitamine A, 91.000 U.I. de vitamines $D_3$, 150 mg de vitamine E, 150 mg de vitamine C.

On incorpore à cet aliment 0,3 kg de composé de l'exemple 1 pour 100 kg d'aliment au total.

Exemple 80: Exemple d'aliment composé pour animaux

On utilise le même aliment équilibré, de base qu'à l'exemple 79. On incorpore à cet aliment, 0,04 kg de composé de l'exemple 1 pour 100 kg d'aliment au total.

ETUDE BIOLOGIQUE

1) Etude de l'activité de choc sur mouche domestique

Les insectes tests sont des mouches domestiques femelles âgées de 4 jours. On opère par pulvérisation directe en chambre de Kearns et March en utilisant comme solvant un mélange d'acétone (5%) et d'Isopar L (solvant pétrolier) (quantité de solvant utilisée 2 ml en une seconde). On utilise 50 insectes par traitement. On effectue les contrôles toutes les minutes jusqu'à 10 minutes, puis à 15 minutes et l'on détermine le KT 50 par les méthodes habituelles.

Le résultat obtenu est le suivant:

| Composé de l'exemple | KT 50 (en mn) concentration à 0,1 g/l | Composé de l'exemple | KT 50 (en nm) concentration à 0,1 g/l |
|---|---|---|---|
| 1 | 1,3 | 50 | 3,5 |
| 2 | 1,6 | 56 | 4,0 |
| 3 | 5,8 | 43 | 2,9 |
| 4 | 2,4 | 53 | 2,4 |
| 5 | 3,2 | 54 | 4,7 |
| 6 | 4,7 | 55 | 8,7 |
| 15 | 1,8 | 60 | 1,8 |
| 26 | 3,5 | 57 | 4,0 |
| 40 | 5,6 | 65 | 3,1 |
| 44 | 2,2 | 66 | 7,9 |
| 48 | 3,7 | 67 | 3,8 |
| 51 | 1,9 | 45 | 6,1 |
| 52 | 1,5 | 72 | 3,8 |
| 49 | 11,0 | 69 | 4,8 |
| | | 70 | 3,9 |

*Conclusion:* Les produits de l'invention sont doués d'un très bon effet de choc sur mouches domestiques.

2) Etude de l'activité par contact tarsal sur blatte germanique.

Les insectes testés sont des mâles de blatte germanique (*Blatella germanica*). On opère par dépôt d'une solution acétonique de concentration déterminée sur le fond d'une boîte de Petri de 20 cm de diamètre. Après séchage, on laisse séjourner 20 mâles de blattes par concentration durant 1 heure puis on transfère les insectes sur milieu sain et on contrôle leur mortalité à 24 h, 48 h, 3 et 5 jours.

Le résultat est exprimé en concentration létale 50 (CL 50) en $mg/m^2$.

| Exemple | CL 50 |
|---|---|
| 43 | 2,8 |
| 45 | 6,4 |
| 72 | 6,7 |
| 70 | 4,5 |

*Conclusion:* Les produits de l'invention sont doués d'une activité insecticide sur blattes.

3) Etude de l'effet létal sur Aphis Cracivora

On utilise des adultes après 7 jours et l'on emploie 10 Aphis par concentration utilisée. On utilise une méthode de contact-ingestion. On effectue le traitement au pistolet de Fisher d'une feuille de fève que l'on dépose dans une boîte de Petri en matière plastique sur une rondelle de paper humidifiée. Le traitement est effectué à l'aide de 2 ml de solution acétonique de produit à tester (1 ml par face de feuille). L'infestation par insecte est effectuée après séchage de la feuille. On maintient les insectes en contact avec la feuille

pendant 1 heure. On place les insectes sur des feuilles non traitées et contrôle la mortalité au bout de 24 heures.

Les résultats expérimentaux obtenus sont résumés dans le tableau suivant:

| Exemple | DL 50 en mg/insecte |
|---|---|
| 49 | 6,5 |
| 43 | 7,5 |

*Conclusion:* Les produits de l'invention sont doués d'un effet létal sur Aphis cracivora.

4) Activité sur Tétranychus urticae. Essai adulticide.

On utilise des plants de haricot comportant deux feuilles cotylédonaires. Ces plants sont traités au pistolet Fisher avec une solution acétonique du produit. Après séchage, 25 femelles de l'acarien Tétranychus urticae sont disposés par feuilles soit 50 individus par dose expérimentée par plant. Le contrôle d'efficacité est effectué après 80 heures de contact. On mesure la DL 50 en mg/hl.

| Exemple | DL 50 |
|---|---|
| 40 | 32 |
| 43 | 342 |
| 45 | 264 |
| 70 | 2126 |

*Conclusion:* Les produits de l'invention et notamment celui de l'exemple 40 sont doués d'un remarquable effect acaricide sur Tétranychus urticae.

**Revendications**

1. Les composés de formule (I):

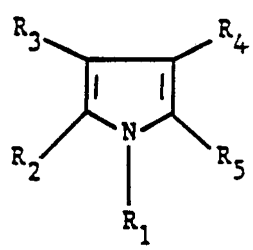

(I)

dans laquelle:

l'un des radicaux $R_2$ ou $R_3$ représente un radical:

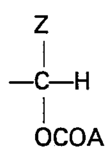

dans lequel

*ou bien A représente un radical*

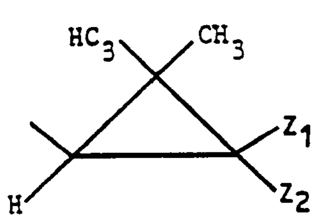

dans lequel

$Z_1$ et $Z_2$ représentent chacun un radical méthyle,

ou $Z_1$ représente un atome d'hydrogène et

*soit* $Z_2$ représente un radical

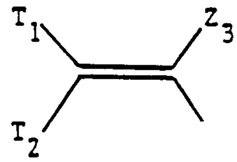

dans lequel $Z_3$ représente un atome d'hydrogène ou d'halogène et $T_1$ et $T_2$, identiques ou différents, représentent un atome d'hydrogène, un atome d'halogène, un radical alcoxyle ou alcoyle renfermant de 1 à 8 atomes de carbone, un radical $CF_3$ ou CN ou un noyau phényle éventuellement substitué par un halogène, ou $T_1$ et $T_2$ forment ensemble un radical cycloalcoyle renfermant de 3 à 6 atomes de carbone ou un radical:

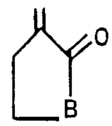

dans lequel B représente un atome d'oxygène ou de soufre;

*soit* $Z_2$ représente un radical:

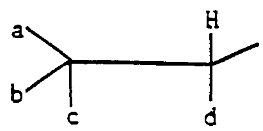

dans lequel a, b, c et d, identiques ou différents représentent chacun un atome d'halogène,

*soit* $Z_2$ représente un radical:

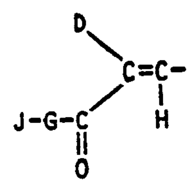

dans lequel D représente un atome d'hydrogène ou d'halogène, un radical alcoxyle renfermant de 1 à 8 atomes de carbone, G représente un atome d'oxygène ou de soufre et J représente un radical alcoyle linéaire [ou] ramifié saturé ou insaturé, renfermant de 1 à 8 atomes de carbone,

*ou bien A représente un radical:*

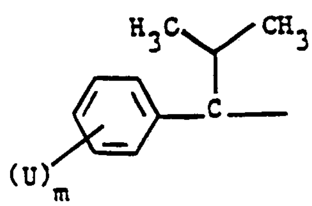

dans lequel U, en position quelconque sur le noyau benzénique, représente un atome d'halogène, un radical alcoyle renfermant de 1 à 8 atomes de carbone ou un radical alcoxy renfermant de 1 à 8 atomes de carbone, m représentant le nombre 0, 1 ou 2 et quand m est 2, les substituants U peuvent être identiques ou différents,

et Z représente un atome d'hydrogène, un groupement —C≡N, —C≡CH, —$CF_3$, ou un radical alkyle renfermant de 1 à 3 atomes de carbone et

celui ces radicaux $R_2$ et $R_3$ qui ne représente pas un radical:

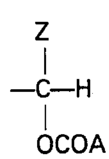

ainsi que les radicaux $R_4$ et $R_5$, identiques ou différents les uns des autres, représentent un atome d'hydrogène, un atome d'halogène, un radical alkyle renfermant jusqu'à 18 atomes de carbone, un radical

phenyl un radical phényalkyle renfermant jusqu'à 18 atomes de carbone, un radical cyano, un radical $CF_3$, un radical $CO_2$ alkyle renfermant jusqu'à 8 atomes de carbone, un radical $NO_2$, un radical alkoxyle renfermant jusqu'à 8 atomes de carbone, un radical:

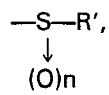 ou 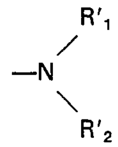 ,

n étant égal à 0, 1 ou 2 et les radicaux R', $R'_1$ et $R'_2$ représentant un radical alkyle renfermant de 1 à 8 atomes de carbone, les radicaux $R_4$ et $R_5$ pouvant former un homocycle carboné saturé ou insaturé renfermant jusqu'à 8 atomes de carbone et

$R_1$ représente:

soit un radical

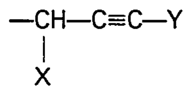

dans lequel X et Y, indentiques ou différents l'un de l'autre, représentent un atome d'hydrogène, un atome d'halogène, un radical alkyle renfermant de 1 à 8 atomes de carbone ou un radical phényle

soit un radical

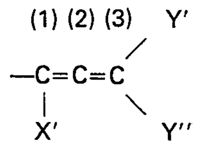 ,

dans lequel X', Y' et Y'', identiques ou différents les uns des autres, représentent l'une des valeurs indiquées ci-dessus pour X et Y, les traits pointillés représentent une double liaison éventuelle entre les carbones 1 et 2;

soit un radical

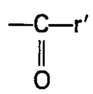

dans lequel r' peut avoir les valeurs indiquées précédemment pour $R_4$ et $R_5$, à l'exception d'halogène, cyano, $NO_2$,

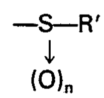

dans lequel n est égal à 1 ou 2 et

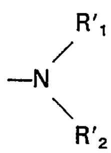 ; soit un radical 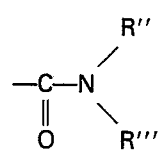 ,

dans lequel R'' et R''', identiques ou différents l'un de l'autre, représentent un atome d'hydrogène, un radical alkyle renfermant de 1 à 8 atomes de carbone, un radical phényle un radical phényle renfermant jusqu'à 18 atomes de carbone, un radical $CF_3$, un radical $CO_2$-alkyle renfermant jusqu'à 8 atomes de carbone ou un radical alkoxy renfermant jusqu'à 8 atomes de carbone.

2. Les composés de formule I tels que définis à la revendication 1 dans lesquels $R_1$ représente un radical $-CH_2-C\equiv CH$.

3. Les composés de formule I tels que définis à l'une quelconque des revendications 1 et 2, dans lesquels le substituant Z du radical:

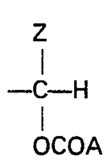

représente un atome d'hydrogène.

4. Les composés de formule I tels que définis à l'une quelconque des revendications 1 et 2 dans lesquels le substituant Z du radical:

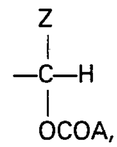

représente un radical cyano.

5. Les composés de formule I tels que définis à l'une quelconque des revendications 1 à 4 dans lesquels c'est le substituant $R_3$ qui représente le radical:

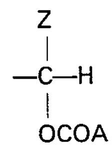

6. Les composés de formule I tels que définis à l'une quelconque des revendications 1 à 5 dans lesquels les substituants $R_2$, $R_4$ et $R_5$ représentent chacun un atome d'hydrogène.

7. Les composés de formule I tels que définis à l'une quelconque des revendications 1 à 5 dans lesquels l'un des radicaux $R_2$, $R_4$ et $R_5$ représente un radical nitro.

8. Les composés de formule I tels que définis à l'une quelconque des revendications 1 à 5 dans lesquels l'un des radicaux $R_2$, $R_4$ et $R_5$ représente un radical cyano.

9. Les composés de formule I tels que définis à l'une quelconque des revendications 1 à 5 dans lesquels des radicaux $R_2$, $R_4$ et $R_5$ représente un radical trifluorométhyle.

10. Les composés de formule I tels que définis à l'une quelconque des revendications 1 à 9, dans lesquels A représente un radical:

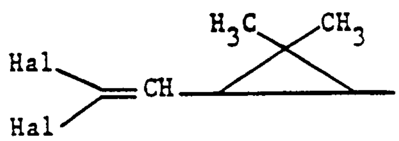

dans lequel Hal représente un atome d'halogène.

11. Les composés de formule I, tels que définis à l'une quelconque des revendications 1 à 10 dans lesquels A représente un radical:

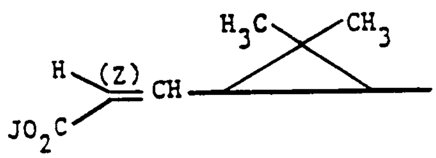

dans lequel J représente un radical alcoyle renfermant de 1 à 8 atomes de carbone, linéaire [ou] ramifié et saturé, la double liaison ayant la géométrie Z.

12. Les composés de formule I tels que définis à l'une quelconque des revendications 1 à 9 dans lesquels A représente un radical:

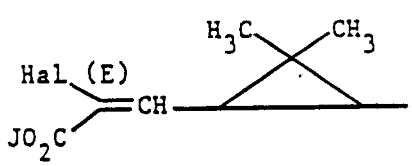

dans lequel Hal représente un atome d'halogène et J représente un radical alcoyle renfermant de 1 à 8 atomes de carbone, linéaire [ou] ramifié et saturé, la double liaison ayant la géométrie (E).

13. Les composés de formule I tels que définis à la revendication 12 dans lesquels Hal représente un atome de fluor.

14. Les composés de formule I tels que définis à la revendication 1 dont les noms suivent:

le 1R,cis (ΔE) 2,2-diméthyl -3-(3-éthoxy-3-oxo 2-fluoro-1-propényl) cyclopropane carboxylate de [1-(2-propynyl)-1H-pyrrol-3-yl] méthyle,

le 1R,cis (ΔZ) 2,2-diméthyl [[-3-(3-méthoxy-3-oxo 1-propényl) cyclopropane carboxylate de [1-(2-propynyl)-1H-pyrrol-3-yl] méthyle,

le 1R,cis (ΔE) 2,2-diméthyl -3-(3-éthoxy 3-oxo 2-fluoro-1-propényl) cyclopropane carboxylate de [1-(2-propynyl) 2-trifluorométhyl 1H-pyrrol-3-yl] méthyle,

15. Le 1R,cis (ΔE) 2,2-diméthyl 3-(3-terbutoxy 3-oxo 2-fluoro 1-propényl) cyclopropane carboxylate de [1-(2-propynyl) 2-trifluorométhyl 1H-pyrrol-3-yl] méthyle.

16. Procédé de préparation des composés de formule I tels que définis à l'une quelconque des revendications 1 à 15 caractérisé en ce que l'on soumet un alcool de formule:

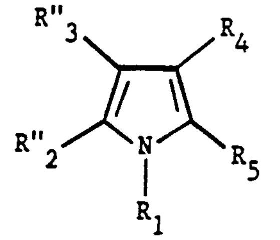

(II)

dans laquelle l'un des radicaux $R''_2$ et $R''_3$ représente un groupement

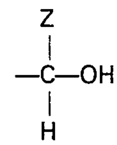

Z conservant la même signification que dans la revendication 1, l'autre radical $R''_2$ ou $R''_3$ ainsi que les radicaux $R_4$ et $R_5$ conservent la même signification que $R_4$ et $R_5$ dans la revendication 1 et $R_1$ conserve la même signification que dans la revendication 1 à l'action d'un acide de formule (III)

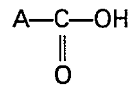

ou d'un dérivé fonctionnel de cet acide, A conservant la même signification que dans la revendication 1.

17. A titre de produits industriels nouveaux, les composés de formule (II):

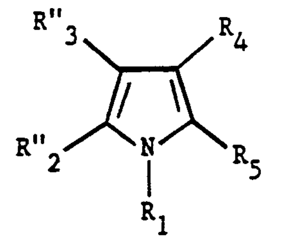

(II)

dans laquelle $R_1$, $R''_2$, $R''_3$, $R_4$ et $R_5$ conservent la même signification que dans la revendication 16.

18. A titre de produit industriel nouveau le 1-(2-propynyl) 2-trifluorométhyl-3-pyrrol-méthanol.

19. Application des composés de formule I tels que définis à l'une quelconque des revendications 1 à 15 à la lutte contre les parasites des végétaux, les parasites des locaux et les parasites des animaux à sang chaud.

20. Les compositions destinées à la lutte contre les parasites des végétaux, les parasites des locaux et les parasites des animaux à sang chaud, caractérisées en ce qu'elles renferment comme principe actif au moins un des produits définis à l'une quelconque des revendications 1 à 13.

21. Les compositions destinées à la lutte contre les parasites des végétaux, les parasites des locaux et les parasites des animaux à sang chaud, caractérisées en ce qu'elles renferment comme principe actif au moins un des produits définis à la revendication 14 ou 15.

22. Les compositions insecticides renfermant comme principe actif au moins l'un des produits définis à l'une quelconque des revendications 1 à 15.

23. Les compositions acaracides renfermant comme principe actif au moins l'un des produits définis à l'une quelconque des revendications 1 à 15.

24. Les compositions nématicides renfermant comme principe actif au moins l'un des produits définis à l'une quelconque ds revendications 1 à 15.

25. Les compositions acaracides destinées à la lutte contre les parasites des animaux à sang chaud, notamment contre les tiques et les gales, caractérisées en ce qu'elles renferment comme principe actif, au moins l'un des produits définis à l'une quelconque des revendications 1 à 15.

26. Les compositions destinées à l'alimentation animale renfermant comme principe actif au moins l'un des produits définis à l'une quelconque des revendications 1 à 15.

27. Associations douées d'activité insecticide, acaricide ou nématicide, caractérisées en ce qu'elles contiennent comme matière active, d'une part un au moins des composés de formule générale (I) telle que définie à la revendication 1, et d'autre part, un au moins des esters pyréthrinoïdes choisis dans le groupe constitué par les esters d'alléthrolone, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcool α-cyano 3-phénoxy benzyliques des acides 2,2-diméthyl 3-(2,2-dichlorovinyl) cyclopropane-1-carboxyliques, par les esters d'alcools -cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolone, d'alcool 3,4,5,6-tétrahydrophtalimido méthylique, d'alcool 5-benzyl 3-furyl méthylique, d'alcool 3-phénoxy benzylique et d'alcools α-cyano 3-phénoxybenzyliques des acides 2,2-diméthyl 3-(1,2-2,2-tétrahaloéthyl) cyclopropane-1-carboxyliques, dans lesquels "halo" représente un atome de fluor, de chlore ou de brome, étant entendu que les copules acides et alcools des esters pyréthrinoides ci-dessus peuvent exister sous toutes leurs formes stéréoisomères possibles.

28. Les compositions telles que définies à l'une des revendications 20 à 27, caractérisées en ce qu'elles renferment en outre un synergiste des pyréthrinoïdes.

**Patentansprüche**

1. Verbindungen der Formel (I)

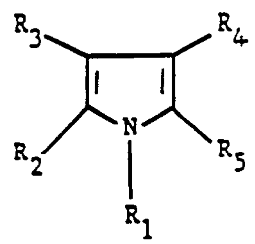

(I)

worin

einer der Reste $R_2$ oder $R_3$ einen Rest

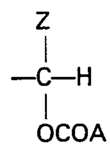

wiedergibt, worin

entweder A einen Rest der Formel

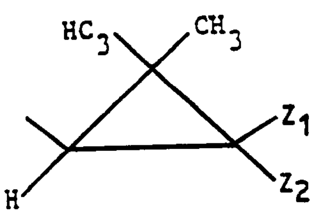

bedeutet, worin

$Z_1$ und $Z_2$ jeweils einen Methylrest wiedergeben, oder $Z_1$ ein Wasserstoffatom bedeutet und *entweder* $Z_2$ einen Rest

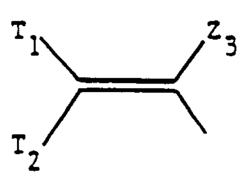

bedeutet, in dem $Z_3$ für ein Wasserstoffatom oder ein Halogenatom steht und $T_1$ und $T_2$, die gleich oder voneinander verschieden sind, ein Wasserstoffatom, ein Halogenatom, einen Alkoxy- oder Alkylrest mit 1 bis 8 Kohlenstoffatomen, einen Rest $CF_3$ oder CN oder einen Phenylring, der gegebenenfalls durch ein Halogenatom substituiert ist, wiedergeben oder $T_1$ und $T_2$ gemeinsam einen Cycloalkylrest mit 3 bis 6 Kohlenstoffatomen oder einen Rest

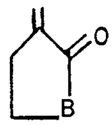

bilden, worin B für ein Sauerstoff- oder Schwefelatom steht;

*oder* $Z_2$ einen Rest

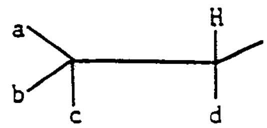

bedeutet, worin a, b, c und d, die gleich oder voneinander verschieden sind, jeweils eines Halogenatom wiedergeben,

*oder* $Z_2$ einen Rest

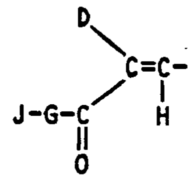

bedeutet, worin D für ein Wasserstoff- oder Halogenatom oder einen Alkoxyrest mit 1 bis 8 Kohlenstoffatomen steht, G ein Sauerstoff- oder Schwefelatom wiedergibt und J für einen linearen oder verzweigten, gesättigten oder ungesättigten Alkylrest mit 1 bis 8 Kohlenstoffatomen steht;

oder A einen Rest

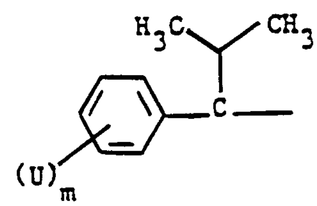

bedeutet, worin U in beliebiger Stellung an dem Benzolring für ein Halogenatom, einen Alkylrest mit 1 bis 8 Kohlenstoffatomen oder einen Alkoxyrest mit 1 bis 8 Kohlenstoffatomen steht, m die Zahl 0, 1 oder 2 wiedergibt, und wenn m für 2 steht, die substituenten U gleich oder voneinander verschieden sein können,

und Z ein Wasserstoffatom, eine Gruppe C≡N, C≡CH, $CF_3$ oder einen Alkylrest mit 1 bis 3 Kohlenstoffatomen bedeutet, und

derjenige der Reste $R_2$ oder $R_3$, der nicht einen Rest

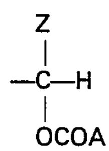

wiedergibt, sowie die Reste $R_4$ und $R_5$, die gleich oder voneinander verschieden sein können, für ein Wasserstoffatom, ein Halogenatom, einen Alkylrest mit bis zu 18 Kohlenstoffatomen, einen Phenylrest, einen Phenylalkylrest mit bis zu 18 Kohlenstoffatomen, eine Cyanogruppe, einen Rest $CF_3$, einen Rest $CO_2$-alkyl mit bis zu 8 Kohlenstoffatomen, eine $NO_2$-Gruppe, einen Alkoxyrest mit bis zu 8 Kohlenstoffatomen, einen Rest

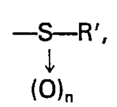 , oder 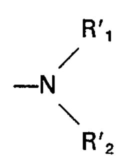 ,

bedeuten, worin n für 0, 1 oder 2 steht und die Reste R', $R'_1$ und $R'_2$ für einen Alkylrest mit 1 bis 8 Kohlenstoffatomen stehen, wobei die Reste $R_4$ und $R_5$ einen gesättigten oder ungesättigten Kohlenstoffhomocyclus mit bis zu 8 Kohlenstoffatomen bilden können, und

$R_1$ bedeutet:
entweder einen Rest

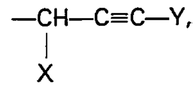

worin X und Y, die gleich oder voneinander verschieden sind, für ein Wasserstoffatom, ein Halogenatom, einen Alkylrest mit 1 bis 8 Kohlenstoffatomen oder einen Phenylrest stehen,

oder einen Rest

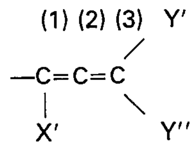

,

worin X', Y' und Y'', die gleich oder voneinander verschieden sein können, eine der vorstehend für X und Y angegebenen Bedeutungen wiedergeben können, wobei die gepunkteten Linien eine etwaige Doppelbindung zwischen den 1 und 2 Kohlenstoffatomen wiedergeben;

oder einen Rest

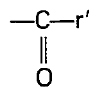

worin r' die vorstehend für $R_4$ und $R_5$ angegebenen Bedeutungen mit Ausnahme von Halogen, Cyano, $NO_2$,

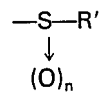

worin n gleich 1 oder 2 ist, und

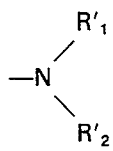

annehmen kann;

oder einen Rest

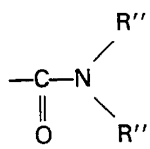

,

worin R'' und R''', die gleich oder voneinander verschieden sein können, für ein Wasserstoffatom, einen Alkylrest mit 1 bis 8 Kohlenstoffatomen, einen Phenylrest, einen Phenylalkylrest mit bis zu 18 Kohlenstoffatomen, eine $CF_3$-Gruppe, einen Rest $CO_2$-alkyl mit bis zu 8 Kohlenstoffatomen oder einen Alkoxyrest mit bis zu 8 Kohlenstoffatomen stehen.

2. Verbindungen der Formel I gemäß Anspruch 1, worin $R_1$ für einen Rest $—CH_2—C{\equiv}CH$ steht.

3. Verbindungen der Formel I gemäß einem der Ansprüche 1 und 2, worin der Substituent Z des Restes

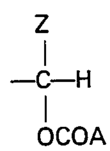

für ein Wasserstoffatom steht.

4. Verbindungen der Formel I gemäß einem der Ansprüche 1 und 2, worin der Substituent Z des Restes

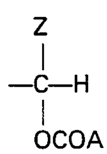

für eine Cyanogruppe steht.

5. Verbindungen der Formel I gemäß einem der Ansprüche 1 bis 4, worin es der Rest $R_3$ ist, der für den Rest

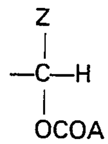

steht.

6. Verbindungen der Formel I gemäß einem der Ansprüche 1 bis 5, worin die Substituenten $R_2$, $R_4$ und $R_5$ jeweils ein Wasserstoffatom bedeuten.

7. Verbindungen der Formel I gemäß einem der Ansprüche 1 bis 5, worin einer der Reste $R_2$, $R_4$ und $R_5$ eine Nitrogruppe bedeutet.

8. Verbindungen der Formel I gemäß einem der Ansprüche 1 bis 5, worin einer der Reste $R_2$, $R_4$ und $R_5$ für eine Cyanogruppe steht.

9. Verbindungen der Formel I gemäß einem der Ansprüche 1 bis 5, worin einer der Reste $R_2$, $R_4$ und $R_5$ für eine Trifluormethylgruppe steht.

10. Verbindungen der Formel I gemäß einem der Ansprüche 1 bis 9, worin A für einen Rest

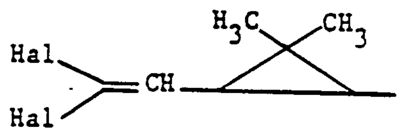

steht, worin Hal ein Halogenatom wiedergibt.

11. Verbindungen der Formel I gemäß einem der Ansprüche 1 bis 10, worin A für einen Rest

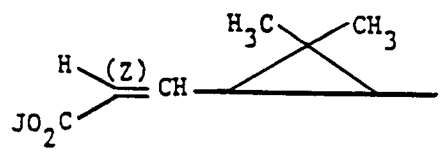

steht, worin J eine lineare oder verzweigte, gesättigte Alkylgruppe mit 1 bis 8 Kohlenstoffatomen wiedergibt und die Doppelbindung die Z-Geometrie bezitzt.

12. Verbindungen der Formel I gemäß einem der Ansprüche 1 bis 9, worin A für einen Rest

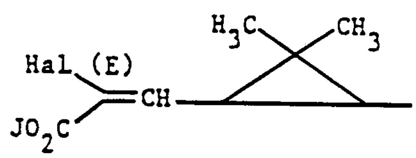

steht, worin Hal ein Halogenatom wiedergibt und J einen linearen oder verzweigten, gesättigten Alkylrest mit 1 bis 8 Kohlenstoffatomen bedeutet, wobei die Doppelbindung die Doppelbindung die (E)-Geometrie aufweist.

13. Verbindung der Formel I gemäß Anspruch 12, worin Hal für ein Fluoratom steht.

14. Verbindung der Formel I gemäß Anspruch 1 mit den folgenden Bezeichnungen:

[1-(2-Propinyl)-1H-pyrrol-3-yl]-methyl-1R,cis-(ΔE)-2,2-dimethyl-3-(3-ethoxy-3-oxo-2-fluor-1-propenyl)-cyclopropan-carboxylat,

[1-(2-Propinyl)-1H-pyrrol-3-yl]-methyl-1R,cis-(ΔZ)-2,2-dimethyl-[[3-(3-methoxy-3-oxo-1-propenyl)-cyclopropan-carboxylat,

[1-(2-Propinyl)-2-trifluormethyl-1H-pyrrol-3-yl]-methyl-1R,cis-(ΔE)-2,2-dimethyl-3-(3-ethoxy-3-oxo-2-fluor-1-propenyl)-cyclopropan-carboxylat.

15. [1-(2-Propinyl)-2-trifluormethyl-1H-pyrrol-3-yl]-methyl-1R,cis-(ΔE)-2,2-dimethyl-3-(3-tert.-butoxy-3-oxo-2-fluor-1-propenyl)-cyclopropan-carboxylat.

16. Verfahren zur Herstellung der Verbindungen der Formel I gemäß einem der Ansprüche 1 bis 15, dadurch gekennzeichnet, daß man einen Alkohol der Formel

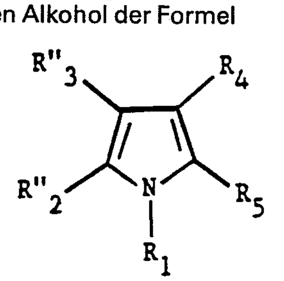

(II)

worin einer der Reste $R''_2$ und $R''_3$ für eine Gruppe

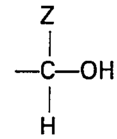

steht, wobei Z die in Anspruch 1 angegebene Bedeutung besitzt, der andere Rest $R''_2$ oder $R''_3$ sowie die Reste $R_4$ und $R_5$ die Bedeutung von $R_4$ und $R_5$, wie in Anspruch 1 angegeben, besitzen und $R_1$ die in Anspruch 1 angegebene Bedeutung besitzt, der Einwirking einer Säure der Formel (III)

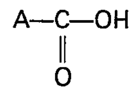

oder eines funktionellen Derivats dieser Säure unterzieht, wobei A die in Anspruch 1 angegebene Bedeutung besitzt.

17. Als neue, industrielle Produkte die Verbindungen der Formel (II)

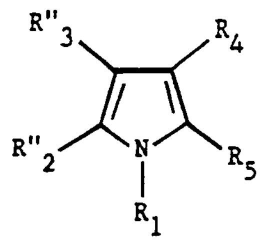

(II)

worin $R_1$, $R''_2$, $R''_3$, $R_4$ und $R_5$ die in Anspruch 16 angegebene Bedeutung besitzen.

18. Als neues, industrielles Produkt das 1-(2-Propinyl)-2-trifluormethyl-3-pyrrol-methanol.

19. Verwendung der Verbindungen der Formel I gemäß einem der Ansprüche 1 bis 15 zur Bekämpfung von Parasiten der Pflanzen, Parasiten von Räumlichkeiten und Parasitic warmblütiger Tiere.

20. Zusammensetzungen für die Bekämpfung von Parasiten der Pflanzen, Parasiten von Räumlichkeiten und Parasiten warmblütiger Tiere, dadurch gekennzeichnet, daß sie als Wirkstoff zumindest eines der Produkte gemäß einem der Ansprüche 1 bis 13 enthalten.

21. Zusammensetzungen für die Bekämpfung von Parasiten der Pflanzen, Parasiten von Räumlichkeiten und Parasiten warmblütiger Tiere, dadurch gekennzeichnet, daß sie als Wirkstoff zumindest eines der Produkte gemäß Anspruch 14 oder 15 enthalten.

22. Insektizide Zusammensetzungen, enthaltend als Wirkstoff zumindest eines der Produkte gemäß einem der Ansprüche 1 bis 15.

23. Akarizide Zusammensetzungen, enthaltend als Wirkstoff zumindest eines der Produkte gemäß einem der Ansprüche 1 bis 15.

24. Nematizide Zusammensetzungen, enthaltend als Wirkstoff zumindest eines der Produkte gemäß einem der Ansprüche 1 bis 15.

25. Akarazide Zusammensetzung für die Bekämpfung von Parasiten wärmblütiger Tiere, insbesondere von Zecken und Krätzmilben, dadurch gekennzeichnet, daß sie als Wirkstoff zumindest eines der Produkte gemäß einem der Ansprüche 1 bis 15 enthalten.

26. Zusammensetzung für die tierische Ernährung, dadurch gekennzeichnet, daß sie als Wirkstoff zumindest eines der Produkte gemäß einem der Ansprüche 1 bis 15 enthalten.

27. Mit insektizider, akarizider oder nematizider Aktivität versehene Assoziationen, dadurch gekennzeichnet, daß sie als Wirkstoff einesteils zumindest eine der Verbindungen der allgemeinen Formel (I), wie in Anspruch 1 definiert, und anderenteils zumindest einen Pyrethrinoidester ausgewählt unter den Estern des Allethrolons, des 3,4,5,6-Tetrahydrophthalimidomethylalkohols, des 5-Benzyl-3-furylmethylalkohols, des 3-Phenoxybenzylalkohols und der α-Cyano-3-phenoxybenzylalkohole, der Chrysanthemumsäuren, den Estern des 5-Benzyl-3-furylmethylalkohols der 2,2-Dimethyl-3-(2-oxo-3-tetrahydrothiophenylidenmethyl)-cyclopropan-1-carbonsäuren, den Estern des 3-Phenoxybenzylalkohols und der α-Cyano-3-phenoxybenzylalkohole der 2,2-Dimethyl-3-(2,2-dichlorvinyl)-cyclopropan-1-carbonsäuren, den Estern der α-Cyano-3-phenoxybenzylalkohole der 2,2-Dimethyl-3-(2,2-dibromvinyl)-cyclopropan-1-carbonsäuren, den Estern des 3-Phenoxybenzylalkohols der 2-p-Chlorphenyl-2-isopropylessigsäuren, den Estern des Allethrolons, des 3,4,5,6-Tetrahydrophthalimidomethylalkohols, des 5-Benzyl-3-furylmethylalkohols, des 3-Phenoxybenzylalkohols und der α-Cyano-3-phenoxybenzylalkohole der 2,2-Dimethyl-3-(1,2,2,2-tretrahaloethyl)-cyclopropan-1-carbonsäuren, enthalten, worin "halo" ein Fluor-, Chlor- oder Bromatom wiedergibt, mit der Maßgabe, daß die sauren Verknüpfungskomponenten und die Alkohole der vorstehenden Pyrethrinoidester in sämtlichen ihrer möglichen stereoisomeren Formen vorliegen können.

28. Zusammensetzungen gemäß einem der Ansprüche 20 bis 27, dadurch gekennzeichnet, daß sie außerdem einen Synergisten der Pyrethrinoide enthalten.

**Claims**

1. The compounds of formula (I):

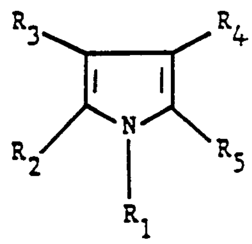

(I)

in which:

one of the radicals $R_2$ or $R_3$ represents a radical:

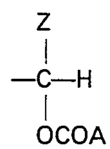

in which

*either A represents a radical*

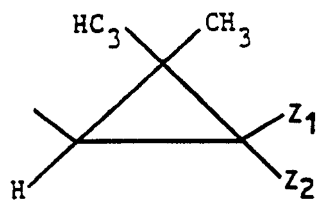

in which

$Z_1$ and $Z_2$ each represents a methyl radical,
or $Z_1$ represents a hydrogen atom and
*either* $Z_2$ represents a radical

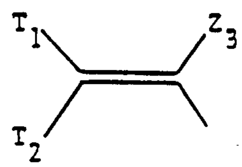

in which $Z_3$ reprsents a hydrogen or halogen atom and $T_1$ and $T_2$, identical or different, represent a hydrogen atom, a halogen atom, an alkoxyl or alkyl radical containing 1 to 8 carbon atoms, a $CF_3$ or CN radical or a phenyl nucleus optionally substituted by a halogen, or $T_1$ and $T_2$ together form a cycloalkyl radical containing 3 to 6 carbon atoms or a radical:

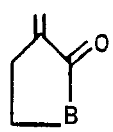

in which B represents an oxygen or sulphur atom;

*or* $Z_2$ represents a radical:

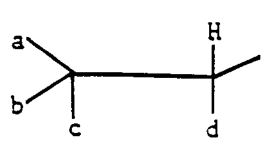

in which a, b, c and d, identical or different, each represents a halogen atom,

*or* $Z_2$ represents a radical:

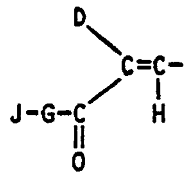

in which D represents a hydrogen or halogen atom, an alkoxyl radical containing 1 to 8 carbon atoms, G represents an oxygen or sulphur atom and J represents a saturated or unsaturated, linear or branched alkyl radical containing 1 to 8 carbon atoms,

*or A represents a radical:*

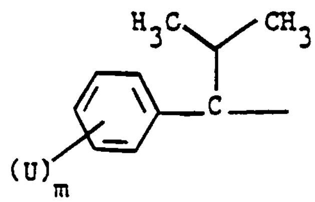

in which U, in any position on the benzene nucleus, represents a halogen atom, an alkyl radical containing 1 to 8 carbon atoms or an alkoxy radical containing 1 to 8 carbon atoms, m representing the number 0, 1 or 2 and when m is 2, the U substituents can be identical or different, and Z represents a hydrogen atom, a C≡N, C≡CH, $CF_3$ group, or an alkyl radical containing 1 to 3 carbon atoms and

that of radical $R_2$ or $R_3$ which does not represent a radical:

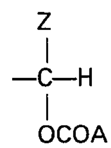

as well as the radicals $R_4$ and $R_5$, identical or different from each other, represent a hydrogen atom, a halogen atom, an alkyl radical containing up to 18 carbon atoms, a phenyl radical, a phenylalkyl radical containing up to 18 carbon atoms, a cyano radical, a $CF_3$ radical, a $CO_2$ alkyl radical containing up to 8 carbon atoms, an $NO_2$ radical, an alkoxyl radical containing up to 8 carbon atoms, a radical:

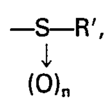 or 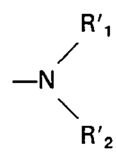 ,

n being equal to 0, 1 or 2 and the radicals R', $R'_1$ and $R'_2$ representing an alkyl radical containing 1 to 8 carbon atoms, the radicals $R_4$ and $R_5$ able to form a saturated or unsaturated carbonated homocycle containing up to 8 carbon atoms and

$R_1$ represents:

either a

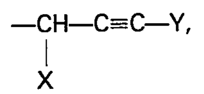

radical in which X and Y, identical or different from each other, represent a hydrogen atom, a halogen atom, an alkyl radical containing 1 to 8 carbon atoms or a phenyl radical,

or a

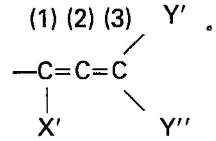

radical, in which X', Y' and Y'', identical or different from each other, represent one of the values indicated above for X and Y, the dotted lines represent an optional double bond between carbons 1 and 2;

or a

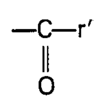

radical in which r' can have the values indicated previously for $R_4$ and $R_5$, with the exception of halogen, cyano, $NO_2$,

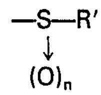

in which n is equal to 1 or 2 and

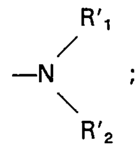 ; or a 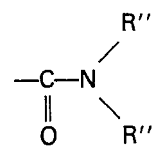

radical, in which R'' and R''', identical or different from each other, represent a hydrogen atom, an alkyl radical containing 1 to 8 carbon atoms, a phenyl radical, a phenylalkyl radical containing up to 18 carbon atoms, a $CF_3$ radical, a $CO_2$-alkyl radical containing up to 8 carbon atoms or an alkoxy radical containing up to 8 carbon atoms.

2. The compounds of formula (I) as defined in claim 1 in which $R_1$ represents a $—CH_2—C{\equiv}CH$ radical.

3. The compounds of formula (I) as defined in any one of claims 1 and 2, in which the substituent Z of the radical:

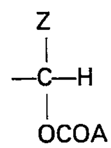

represents a hydrogen atom.

4. The compounds of formula (I) as defined in one of claims 1 and 2 in which the substituent Z of the radical:

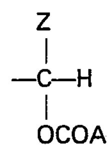

represents a cyano radical.

5. The compounds of formula (I) as defined in any one of claims 1 to 4 in which it is the substituent $R_3$ which represents the radical:

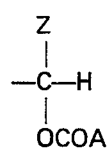

6. The compounds of formula (I) as defined in any one of claims 1 to 5 in which the substituents $R_2$, $R_4$ and $R_5$ each represents a hydrogen atom.

7. The compounds of formula (I) as defined in any one of claims 1 to 5 in which one of the radicals $R_2$, $R_4$ and $R_5$ represents a nitro radical.

8. The compounds of formula (I) as defined in any one of claims 1 to 5 in which one of the radicals $R_2$, $R_4$ and $R_5$ represents a cyano radical.

9. The compounds of formula (I) as defined in any one of claims 1 to 5 in which one of the radicals $R_2$, $R_4$ and $R_5$ represents a trifluoromethyl radical.

10. The compounds of formula (I) as defined in any one of claims 1 to 9, in which A represents a radical:

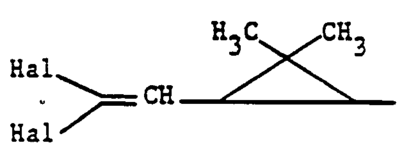

in which Hal represents a halogen atom.

11. The compounds of formula (I) as defined in any one of claims 1 to 10, in which A represents a radical:

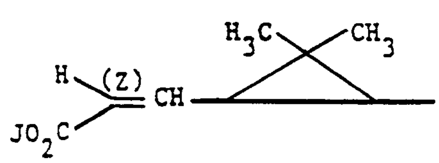

in which J represents an alkyl radical containing 1 to 8 carbon atoms, linear or branched and saturated, the double bond having Z geometry.

12. The compounds of formula (I) as defined in any one of claims 1 to 9 in which A represents a radical:

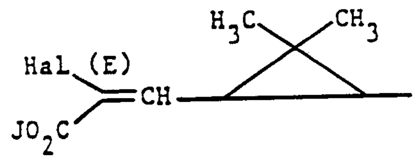

in which Hal represents a halogen atom and J represents an alkyl radical containing 1 to 8 carbon atoms, linear or branched and saturated, the double bond having (E) geometry.

13. The compounds of formula (I) as defined in claim 12 in which Hal represents a fluorine atom.

14. The compounds of formula (I) as defined in claim 1 of which the names follow:

1R,cis (ΔE) 2,2-dimethyl-3-(3-ethoxy-3-oxo-2-fluoro-1-propenyl)-cyclopropane carboxylate of [1-(2-propynyl)-1H-pyrrol-3-yl]-methyl,

1R,cis (ΔZ) 2,2-dimethyl-3-(3-methoxy-3-oxo-1-propenyl)-cyclopropane carboxylate of [1-(2-propynyl)-1H-pyrrol-3-yl]-methyl,

1R,cis (ΔE) 2,2-dimethyl-3-(3-ethoxy-3-oxo-2-fluoro-1-propenyl)-cyclopropane carboxylate of [1-(2-propynyl)-2-trifluoromethyl-1H-pyrrol-3-yl]-methyl.

15. 1R,cis (ΔE) 2,2-dimethyl-3-(3-tertbutoxy-3-oxo-2-fluoro-1-propenyl)-cyclopropane carboxylate of [1-(2-propynyl)-2-trifluoromethyl-1H-pyrrol-3-yl]-methyl.

16. Process for the preparation of compounds of formula (I) as defined in any one of claims 1 to 15 , characterized in that an alcohol of formula:

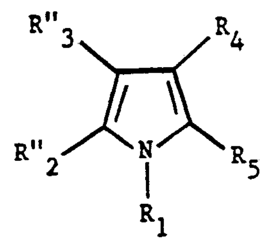

(II)

in which one of the radicals $R''_2$ and $R''_3$ represents a group:

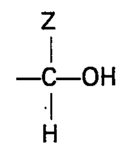

Z keeping the same meaning as in claim 1, the other radical $R''_2$ or $R''_3$ as well as radicals $R_4$ and $R_5$, keep the same meaning as $R_4$ and $R_5$ in claim 1 and $R_1$ keeps the same meaning as in claim 1, is subjected to the action of an acid of formula (III):

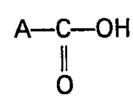

or a functional derivative of this acid, A keeping the same meaning as in claim 1.

17. As new industrial products, the compounds of formula (II):

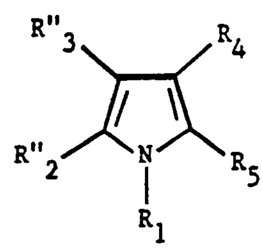

(II)

in which $R_1$, $R''_2$, $R''_3$, $R_4$ and $R_5$ keep the same meaning as in claim 16.

18. As a new industrial product, 1-(2-propynyl)-2-trifluoromethyl-3-pyrrol-methanol.

19. Use of the compounds of formula (I) as defined in any one of claims 1 to 15 for combating parasites of vegetation, parasites of premises and parasites of warm-blooded animals.

20. The comositions intended for combating parasites of vegetation, parasites of premises and parasites of warm-blooded animals, characterized in that they contain as active ingredient at least one of the products defined in any one of claims 1 to 13.

21. The compositions intended for combating parasites of vegetation, parasites of premises and parasites of warm-blooded animals, characterized in that they contain as active principle at least one of the products defined in claim 14 or 15.

22. The insecticide compositions containing as active ingredient at least one of the products defined in any one of claims 1 to 5.

23. The acaricide compositions containing as active ingredient at least one of the products defined in any one of claims 1 to 5.

24. The nematicide compositions containing as active ingredient at least one of the products defined in any one of claims 1 to 5.

25. The acaricide compositions intended for combating parasites of warm-blooded animals, notably ticks and mites, characterized in that they contain as active ingredient, at least one of the products defined in any one of claims 1 to 15.

26. The compositions intended for animal fodder containing as active ingredient at least one of the products defined in any one of claims 1 to 15.

27. Combinations endowed with insecticide, acaricide or nematicide activity, characterized in that they contain as active material, on the one hand at least one of the compounds of general formula (I) as defined in claim 1, and on the other hand at least one of the pyrethinoid esters chosen from the group constituted by the esters of allethrolone, of 3,4,5,6-tetrahydrophthalimidomethyl alcohol, of 5-benzyl-3-furyl methyl alcohol, of 3-phenoxybenzyl alcohol and of alpha-cyano-3-phenoxybenzyl alcohols with chrysanthemic acids, by the esters of 5-benzyl-3-furyl methyl alcohol with 2,2-dimethyl-3-(2-oxo-3-tetrahydrothiophenyl-idenemethyl)-cyclopropane-1-carboxylic acids, by the esters of 3-phenoxybenzyl alcohol and of alpha-cyano-3-phenoxybenzyl alcohol with 2,2-dimethyl-3-(2,2-dichlorovinyl)-cyclopropane-1-carboxylic acids, by the esters of alpha-cyano-3-phenoxy-benzyl alcohols with 2,2-dimethyl-3-(2,2-dibromovinyl)-cyclopropane-1-carboxylic acids, by the esters of 3-phenoxybenzyl alcohol with 2-parachlorophenyl-2-isopropyl acetic acids, by the esters of allethrolone, of 3,4,5,6-tetrahydrophthalimidomethyl alcohol, of 5-benzyl-3-furyl methyl alcohol, of 3-phenoxybenzyl alcohol, and of alpha-cyano-3-phenoxybenzyl alcohols with 2,2-dimethyl-3-(1,2,2,2-tetrahaloethyl)cyclopropane-1-carboxylic acids, in which "halo" represents a fluorine, chlorine or bromine atom, it being understood that the acid and the alcohol copulas of the above pyrethrinoid esters can exist in all their possible stereoisomer forms.

28. The compositions as defined in one of claims 20 to 27, characterized in that they contain in addition a pyrethrinoid synergist.